(19) Europäisches Patentamt / European Patent Office / Office européen des brevets

(11) **EP 4 509 607 A2**

(12) **EUROPEAN PATENT APPLICATION**

(43) Date of publication:
**19.02.2025 Bulletin 2025/08**

(21) Application number: **24208212.1**

(22) Date of filing: **20.11.2019**

(51) International Patent Classification (IPC):
***C12N 15/63*** *(2006.01)*

(52) Cooperative Patent Classification (CPC):
(C-Sets available)
**C12N 15/67; A61K 31/7105; A61K 31/713; A61K 38/465; A61P 7/00; A61P 7/06; C07K 14/47; C07K 14/805; C12N 9/22; C12N 15/113; C12N 15/63;** C12N 2310/20 (Cont.)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **20.11.2018 US 201862769796 P**

(62) Document number(s) of the earlier application(s) in accordance with Art. 76 EPC:
**19818447.5 / 3 883 597**

(71) Applicant: **Fulcrum Therapeutics, Inc.**
**Cambridge, Massachusetts 02139 (US)**

(72) Inventors:
- **RAHL, Peter**
  **Cambridge, 02139 (US)**
- **CACACE, Angela**
  **Cambridge, 02139 (US)**
- **CAMERON, Michael**
  **Cambridge, 02139 (US)**
- **KAKUMANU, Akshay**
  **Cambridge, 02139 (US)**

(74) Representative: **HGF**
**HGF Limited**
**1 City Walk**
**Leeds LS11 9DX (GB)**

Remarks:
- The complete document including Reference Table(s) and the Sequence Listing(s) can be downloaded from the EPO website
- This application was filed on 22-10-2024 as a divisional application to the application mentioned under INID code 62.
- Claims filed after the date of receipt of the application (Rule 68(4) EPC).

(54) **COMPOSITIONS AND METHODS FOR INCREASING FETAL HEMOGLOBIN AND TREATING SICKLE CELL DISEASE**

(57) The present invention relates to compositions and methods of increasing levels of fetal hemoglobin (HbF) in cells. The present invention further relates to methods for treating patients suffering from blood cell diseases, including those associates with reduced amounts of functional adult hemoglobin (HbA), such as sickle cell disease and β-thalassemias.

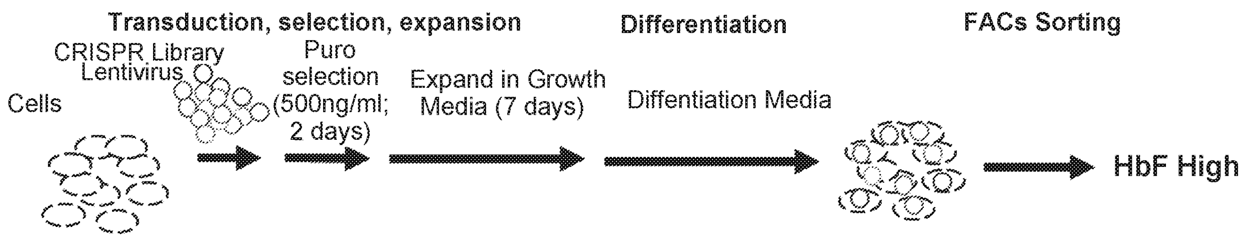


FIG. 1

EP 4 509 607 A2

(52) Cooperative Patent Classification (CPC): (Cont.)

C-Sets
C12N 2310/14, C12N 2310/531

## Description

## CROSS REFERENCE TO RELATED APPLICATIONS

This application claims the benefit of, and priority to, U.S. Provisional Application No. 62/769,796, filed on November 20, 2018, the contents of which is incorporated herein by reference in their entireties.

STATEMENT REGARDING SEQUENCE LISTING

The Sequence Listing associated with this application is provided in text format in lieu of a paper copy, and is hereby incorporated by reference into the specification. The name of the text file containing the Sequence Listing is FULC_033_01WO_ST25.txt. The text file is 33 KB, was created on November 20, 2019, and is being submitted electronically via EFS-Web.

## FIELD OF THE DISCLOSURE

The present disclosure relates to targets, compositions and methods of inducing fetal hemoglobin (hemoglobin γ (HBγ) or HbF) expression in erythroid cells. The present disclosure further relates to methods for treating patients suffering from diseases associated with blood cell disorders, such as Sickle Cell Disease (SCD) or β-thalassemias, including those where elevated expression of HbF protein can compensate for a mutant or defective hemoglobin β (*HBB*) gene, a mutant or defective HBB protein, or changes in HBB protein levels.

## BACKGROUND

Hemoglobin is the critical protein involved in oxygen transport throughout the body of vertebrates. It is found in red blood cells and consists of two α subunits and two β-like subunits. The composition of hemoglobin is developmentally regulated, and the human genome encodes multiple versions of these proteins, which are expressed during distinct stages of development (Blobel et al, Exp Hematol 2015; Stamatoyannopoulos G. Exp Hematol 2005). In general, fetal hemoglobin (HbF) is composed of two subunits of hemoglobin γ (HBγ) and two subunits of hemoglobin α (HBα) and adult hemoglobin (HbA) is composed of two subunits of hemoglobin β (HBβ) and two subunits of HBα. Thus, the β-like subunit utilized during the fetal stage of development (HBγ) switches to hemoglobin β (HBβ) after birth.

The developmental regulation of the expression of β-like subunits has been the focus of intense studies for decades (Li et al. Blood 2002). All five β-like subunits in humans reside on chromosome 11, where their genomic location corresponds to their temporal expression pattern. A distal cluster of enhancer elements, called the locus control region (LCR), coordinates the expression pattern at the β globin locus, where multiple transcription factors, including GATA1, GATA2, KLF1, KLF2, and MYB and TAL1, bind at specific locations within the LCR at specific times in development. The five human β-like subunits are epsilon (*HBE1*; ε). gammaG (*NBG2:* γ), gammaA (*HBC1*; γ), delta (*HBD; δ*) and beta (*HBB;* β). The *HBE1* gene is expressed during embryonic development, the *HBG1* and *HBG2* genes are expression during fetal development, and *HBD* and *HBB* genes are expressed in adults. The *HBG1* and *HBG2* genes encode identical proteins except for a single amino acid change at residue 136 (*HBG1* = gly; *HBG2* = ala). Red blood cell disorders like Sickle Cell Disease (SCD) and β-thalassemias are caused by alterations within the gene for the hemoglobin β (HBβ) subunit.

SCD affects millions of people worldwide and is the most common inherited blood disorder in the United States (70,000-80,000 Americans). SCD has a high incidence in African Americans, where it is estimated to occur in 1 in 500 individuals. SCD is an autosomal recessive disease caused by single homozygous mutations in both copies of the *HBB* gene (E6V) that result in a mutant hemoglobin protein called HbS (https://ghr.nlm.nih.gov/condition/sickle-cell-disease). Under deoxygenated conditions, the HbS protein polymerizes, which leads to abnormal red blood cell morphology. This abnormal morphology can lead to multiple pathologic symptoms including vaso-occlusion, pain crises, pulmonary hypertension, organ damage and stroke.

β-thalassemia is caused by mutations in the *HBB* gene and results in reduced hemoglobin production (https://ghr.nlm.nih.gov/condition/beta-thalassemia). The mutations in the *HBB* gene typically reduce the production of adult β-globin protein, which leads to low levels of adult hemoglobin, HbA. This leads to a shortage of red blood cells and a lack of oxygen distribution throughout the body. Patients with β-thalassemias can have weakness, fatigue and are at risk of developing abnormal blood clots. Thousands of infants are born with β-thalassemia each year. and symptoms are typically detected within the first two years of life.

The identification of factors that regulate the expression of fetal hemoglobin could be useful targets for the treatment of SCD and β-thalassemias, since upregulation of fetal hemoglobin could compensate for mutant HbS protein in SCD or a lack of HbA in β-thalassemias. Because β-like globin expression is developmentally regulated, with a reduction in the fetal ortholog (γ) occurring shortly after birth concomitantly with an increase in the adult ortholog (β), it has been postulated that maintaining expression of the anti-sickling γ ortholog may be of therapeutic benefit in both children and adults. A fetal ortholog of HBβ, hemoglobin γ (HBγ) can reverse disease-related pathophysiology in these disorders by also forming complexes with the required hemoglobin α subunit (Paikari and Sheehan, Br J Haematol 2018; Lettne and Bauer, Lancet 2016). Expression of the fetal hemoglobin protein can reverse the SCD pathophysiology through inhibiting HbS polymerization and morphologically defective red blood cells. Functionally, upregulation of either the *HBG1* or *HBG2* gene can compensate for mutant or defective adult HBβ. Based on clinical and preclinical studies, upregulation of hemoglobin γ (HBγ) is the proposed mechanism for compounds including Palmolidomide and Hydroxyurea and targets including EHMTI/EHMT2 and LSD1 (Moutouh-de Parseval et al. J Clin Invest 2008; Letvin et al. NEJM 1984; Renneville et al. Blood 2015; Shi et al. Nature Med 2015).

Given the severity and lack of effective treatments for blood cell disorders, such as Sickle Cell Disease (SCD) and β-thalassemias, including those where elevated expression of HbF protein could compensate for a mutant or defective hemoglobin β (HBβ) gene, there is clearly a need for new methods of treatment for these disorders. The present disclosure meets this need by providing new therapeutic agents and methods for increasing HbF for the treatment of these disorders.

## SUMMARY OF THE INVENTION

The present disclosure is based, in part, on the identification of novel targets for inducing fetal hemoglobin (hemoglobin γ (HBγ) or HbF) expression in erythroid cells. The present disclosure further relates to methods for treating patients suffering from diseases associated with blood cell disorders, such as Sickle Cell Disease (SCD) or β-thalassemias.

In one embodiment, the present disclosure provides a method for increasing expression of a fetal hemoglobin (HbF) in a cell, comprising contacting a cell with an inhibitor of a target protein or protein complex that functions to regulate HbF expression. In some embodiments, the HbF comprises hemoglobin gamma and hemoglobin alpha. In some embodiments, the hemoglobin gamma comprises hemoglobin gamma G1 (HBG1) and/or or hemoglobin gamma G2 (HBG2). In particular embodiments, the target protein or protein complex regulates HbF expression via a molecular signaling pathway listed in Table 5. In particular embodiments, the molecular signaling pathway is selected from the group consisting of glucagon signaling pathway, carbon metabolism, oxytocin signaling, glycolysis, gluconeogenesis, endocrine resistance, Gonadotropin-releasing hormone (GnRH) signaling, oocyte meiosis. fatty acid degradation, and inflammatory mediator regulation of Transient Receptor Potential (TRP) channels. In certain embodiments, the target protein is CUL3. In certain embodiments, the target protein is SPOP. In certain embodiments, the target protein is selected from those listed in Table 1, Table 2, Table 3, Table 4, Table 5, Table 6 or Table 7. In certain embodiments, the hit shows enriched expression in whole blood versus other tissues and cell types. In certain embodiments, the target protein (or hit) is expressed in late stage erythroid cells or listed in Table 7. In some embodiments, the target protein is permanently or transiently associated with a multi-protein complex that regulates HbF expression. In some embodiments, the multi-protein complex is selected from those listed in Table 3 or Table 4, and the target is selected from those listed in Table 3 or Table 4. In certain embodiments, CUL3 is permanently or transiently associated with the multi-protein complex. In certain embodiments, the multi-protein complex is selected from D(4) dopamine receptor (DRD4)-Kelch like protein 12 (KLH12)-CUL3, ubiquitin E3 ligase, coiled coil domain containing protein 22 (CCDC22)-COMM domain containing protein 8 (COMMD8)-CUL3, or Cullin associated NEDD8 dissociated protein (CAND1)-CUL3- E3 ubiquitin protein ligase RBX1 (RBX). In certain embodiments, SPOP is permanently or transiently associated with the multi-protein complex. In certain embodiments, the multi-protein complex is a ubiquitin E3 ligase complex. In particular embodiments, the inhibitor targets a nucleotide sequence encoding the target protein or protein complex thereby inhibiting or preventing the expression of the target protein or protein complex. In some embodiments, the nucleotide sequence encoding the target protein or protein complex is DNA or RNA. In certain embodiments, the nucleotide sequence encodes CUL3, and optionally comprises or consists of a nucleic acid encoding the amino acid sequence of SEQ ID NO: 108. In certain embodiments, the nucleotide sequence encodes SPOP, and optionally comprises or consists of a nucleic acid encoding the amino acid sequence of SEQ ID NO: 109. In some embodiment, the inhibitor is selected from a group consisting of a small molecule, a nucleic acid, a polypeptide, and a nucleoprotein complex, e.g., which bind to a target protein or a polynucleotide sequence encoding the target protein, such as a gene or mRNA encoding the target protein. It should be understood that an inhibitor or a target protein may inhibit the target protein by inhibiting the target protein directly, e.g., by binding to the target protein, or by inhibiting expression of the target protein, e.g., by binding to a polynucleotide encoding the target protein. In some embodiments, the nucleic acid is selected from DNA, RNA, shRNA, siRNA, microRNA, gRNA, and antisense oligonucleotide. In certain embodiments, the polypeptide is selected from a protein, a peptide, a protein mimetic, a peptidomimetic, an antibody or functional fragment thereof, and an antibody-drug conjugate or a functional fragment thereof. In particular embodiments, the nucleoprotein complex is a ribonucleoprotein complex (RNP) comprising: a) a first sequence comprising a guide RNA (gRNA) that specifically binds a target sequence, wherein the target sequence comprises a regulator of HbF expression and b) a second sequence encoding a CRISPR-Cas protein wherein the CRISPR-Cas protein comprises a DNA-nuclease activity. In particular embodiments, the cell is a blood cell, e.g., an erythrocyte. In certain embodiments, the contacting a cell occurs in vitro, in vivo, ex vivo, or in situ.

In a related embodiment, the disclosure provides a pharmaceutical composition for increasing expression of fetal hemoglobin (HbF) comprising: an inhibitor of a target protein or protein complex that functions to regulate HbF expression, and a diluent, excipient. and carrier formulated for delivery to a patient in need thereof. In particular embodiments, the inhibitor is a small molecule, a nucleic acid, e.g., DNA, RNA, shRNA, siRNA, microRNA, gRNA, or antisense oligonucleotide., or a polypeptide, e.g., a protein, a peptide, a protein mimetic, a peptidomimetic, an antibody or functional fragment thereof, or antibody-drug conjugate or a functional fragment thereof. In some embodiments, the small molecule inhibitor targets CUL3. In some embodiments, the CUL3 small molecule inhibitor is selected from MLN4924, suramin, or DI-591. In some embodiments, the polypeptide specifically binds a regulator of HbF expression. In certain embodiments, the inhibitor is a ribonucleoprotein (RNP) complex comprising: a) a first sequence comprising a guide RNA (gRNA) that specifically binds a target sequence, wherein the target sequence comprises a regulator of HbF expression and b) a second sequence encoding a CRISPR-Cas protein wherein the CRISPR-Cas protein comprises a DNA-nuclease activity. In certain embodiments, the gRNA binds a gene encoding the regulator of HbF expression. In certain embodiments, the target sequence is listed in any of Tables 1, 3-4, or 6-7. In some embodiments, the gRNA comprises any one of the targets or sequences in Table 2. or a fragment thereof, or an antisense sequence of the target sequence or fragment thereof. In some embodiments, the target sequence is CUL3. In some embodiments, wherein the target sequence is SPOP. In some embodiments, the gRNA comprises any one of the sequences disclosed in Table 2. In some embodiments, the gRNA binds a gene encoding CUL3, and optionally comprises or consists of GAGCATCTCAAACACAACGA (SEQ ID NO: 94), CGAGATCAAGTTGTACGTTA (SEQ ID NO: 95), or TCATCTACGGCAAACTCTAT (SEQ ID NO: 96). In some embodiments, the gRNA binds a gene encoding SPOP. and optionally comprises or consists of TAACTTTAGCTTTTGCCGGG (SEQ ID NO: 91), CGGGCATATAGGTTTGTGCA (SEQ ID NO: 92), or GTTTGCGAGTAAACCCCAAA (SEQ ID NO: 93). In certain embodiments, the first sequence comprising the gRNA comprises a sequence encoding a promoter capable of expressing the gRNA in a eukaryotic cell. In some embodiments, the second sequence comprising the CRISPR-Cas protein comprises a sequence capable of expressing the CRISPR-Cas protein in a eukaryotic cell. e.g., a mammalian cell, such as a blood cell, e.g., an erythrocyte. In some embodiments, the composition is delivered via a vector, e.g., a viral vector, such as an AAV.

In another related embodiment, the disclosure provides a method of treating a disease or disorder associated with a defect in a hemoglobin protein activity or expression, comprising providing to a subject in need thereof the composition disclosed herein. In some embodiments, the disease or disorder is a blood disorder, e.g., Sickle cell disease, β-thalassemia, β-thalessemia intermedia, β-thalessemia major, β-thalessemia minor, and Cooley's anemia. In some embodiments, the hemoglobin protein is selected from hemoglobin-alpha and hemoglobin-beta. In certain embodiments, the defect in the hemoglobin protein activity or expression results from a mutation, substitution, deletion, insertion, frameshift, inversion, or transposition to a nucleotide sequence which encodes the hemoglobin protein.

## BRIEF DESCRIPTION OF THE DRAWINGS

FIG. 1 is a schematic detailing the CRISPR pooled screen sample collection process. Samples were collected following puromycin selection (1), prior to FACs sorting (2) and after sorting for HbF high cells (3).

FIG. 2 provides FACS sorting plots from the CRISPR screen with Library #1. FACs plots are shown for HUDEP2 cells with control sgGFP (dark gray) and CRISPR Library #1 (light gray). The left panel plots the level of HbF (X-axis) and β-Actin (Y-axis) for each event and the line "L" indicates the HbF threshold for HbF high cells. The right panel represents the same data in a one-dimensional plot showing the HbF levels (X-axis) and Events (Y-axis) and the line "C'' indicates the HbF threshold for HbF high cells. Any cell above the HbF threshold was collected in the HbF high population.

FIG. 3 provides FACS sorting plots from the CRISPR screen with Library #2. FACs plots are shown for HUDEP2 cells with control sgGFP (dark gray) and CRISPR Library #2 (light gray). The left panel plots the level of HbF (X-axis) and β-Actin (Y-axis) for each event and the line "L" indicates the HbF threshold for HbF high cells. The right panel represents the same data in a one-dimensional plot showing the HbF levels (X-axis) and Events (Y-axis) line "C" indicates the HbF threshold for HbF high cells. Any cell above the HbF threshold was collected in the HbF high population.

FIG. 4A details a list of all bioinformatics analysis performed on the CRISPR screen data: Genome alignment (left panel), hit quantification (middle panel) and hit prioritization (right panel).

FIG. 4B is a series of plots showing the distribution of guide abundance in different samples across two different screening libraries (Library #1, left; Library #2, right). Arrow indicate the peaks for the number of guides with a given abundance level at input, post-selection and following HBF+ve (HbF high positive sorted population)..

FIG. 4C is a plot showing the distribution of z-score differences across samples for the Library #1. Squares indicate hits that help differentiation, and triangles indicate hits that impede differentiation.

FIG. 5A is a heatmap showing all genes that have more than one enriched gRNA in initial Library #1 screening data.

FIG. 5B is a plot detailing the overlap between Library #1 and Library #2. The triangles correspond to genes that were called hits in both the screening libraries.

FIG. 5C is an exemplary graph displaying Z-score (y-axis) vs. UBE2H gene locus (x-axis), indicating that 4 out of the 10 designed guides RNAs have a Z-score greater than 2.5.

FIG. 6 is chart detailing the number of hits for each of the indicated distinct biological complexes. Complex membership information was taken from the CORUM database.

FIG. 7A is a heatmap showing the expression z-score of CRISPR hits enriched in whole blood (32 out of 307 hits show highly enriched expression in whole blood versus other tissues and cell types, data source: GTEx). The 32 hits showing highly enriched expression in whole blood are listed in Table 7.

FIG. 7B is a heatmap showing hits with "Late Erythroid" expression pattern (data source: DMAP). Hits with "Late Erythroid" expression include: CUL3, SAP130. PRPS1, NAP1L4, GCLC. CUL4A, GCDH. NEK1, HIRA. MST1, SPOP, GOLGA5, AUH. MAST3, CDKNIB, UBR2, MAP4K4, TAF10, HDGF, YWHAE, AMD1, EID1, HIF1AN, CDK8, DCK, FXR2, UQCRC1, TESK2, ADCK2, USP21, CAMK2D, FGFR1, PHC2, UBE2H. BPGM, SIRT2, SIRT3, NFYC, and CPT2.

FIG. 7C is a hierarchical differentiation tree of UBE2H with exemplary "Late Erythroid" expression pattern.

FIG. 8A is a series of images depicting HbF levels determined by HbF immunocytochemistry (ICC) using CRISPR Cas9-RNP-based loss of function. Cas9-RNP complexes were electroporated into proliferating CD34+ cells. Cells were then differentiated for 7 days down the erythroid lineage and HbF levels were quantified using HbF ICC. The percent F cells (top row) and mean HbF intensity (bottom row) were quantified for negative control, sgBCL11A, sgSPOP and sgCUL3.

FIGS. 8B - 8E is a series of graph depicting HbF levels determined by HbF ICC using shRNA-based loss of function. Percent F cells (FIG. 8B and FIG. 8D) and mean HbF intensity (FIG. 8C and FIG. 8E) were quantified for individual shRNA constructs for negative control, shBCL11A, shSPOP and shCUL3.

## DETAILED DESCRIPTION OF THE INVENTION

The present invention relates to targets, compositions and methods for increasing fetal hemoglobin (HbF) in erythroid cells, e.g., by increasing expression of hemoglobin γ (HBγ). This can occur through upregulation of hemoglobin γ mRNA levels (e.g., *HBG1* or *HBG2*) and/or upregulation of fetal hemoglobin protein (HBγ) levels, which results in an elevation in HbF. The targets, compositions or methods can be used alone or in combination with another agent that upregulates HbF or targets symptoms of SCD or β-thalassemia, including but not limited to, vaso-occlusion and anemia.

### Abbreviations

As used in this specification and the appended claims, the singular forms "a," "an" and "the'' include plural references unless the content clearly dictates otherwise.

As used in this specification, the term "and/or" is used in this disclosure to either "and" or "or" unless indicated otherwise.

Throughout this specification, unless the context requires otherwise, the word "comprise", or variations such as "comprises" or "comprising", will be understood to imply the inclusion of a stated element or integer or group of elements or integers but not the exclusion of any other element or integer or group of elements or integers.

As used in this application, the terms "about" and "approximately" are used as equivalents. Any numerals used in this application with or without about/approximately are meant to cover any normal fluctuations appreciated by one of ordinary skill in the relevant art. In certain embodiments, the term "approximately" or "about" refers to a range of values that fall within 25%, 20%, 19%, 18%, 17%, 16%, 15%, 14%, 13%, 12%, 11%, 10%, 9%, 8%, 7%, 6%, 5%, 4%, 3%, 2%, 1%, or less in either direction (greater than or less than) of the stated reference value unless otherwise stated or otherwise evident from the context (except where such number would exceed 100% of a possible value).

"Administration" refers herein to introducing an agent or composition into a subject or contacting an agent or composition with a cell and/or tissue.

### Methods and Compositions

In one aspect, the present disclosure provides methods for increasing the amount of fetal hemoglobin (HbF) in a cell. In particular embodiments, the method comprises increasing expression of one or more components of HbF in a cell. In particular embodiments, the component of HbF is a hemoglobin γ (HBγ), e.g., human hemoglobin subunit gamma-1 (HBG1) or human hemoglobin subunit gamma-2 (HBG2). In particular embodiments, the component of fetal hemoglobin is a hemoglobin α (HBα), e.g., human hemoglobin subunit alpha-1 (HBA1) or human hemoglobin subunit alpha-2 (HBA2). In certain embodiments, expression of both HBγ and HBα is increased.

In certain embodiments, the fetal hemoglobin comprises a human hemoglobin subunit gamma-1 (HBG1) having the protein sequence set forth in NCBI Reference Sequence: NP_000550.2 and shown below:

MGHFTEEDKATITSLWGKVNVEDAGGETLGRLLVVYPWTQRFFDSFGNLSSASAIM
GNPKVKAHGKKVLTSLGDAIKHLDDLKGTFAQLSELHCDKLHVDPENFKLLGNVLV
TVLAIHFGKEFTPEVQASWQKMVTAVASALSSRYH (SEQ ID NO: 1).

In certain embodiments, the HBG1 protein is encoded by the polynucleotide sequence set forth in NCBI Reference Sequence: NM_000559.2 and shown below:

```
  1 acactcgctt ctggaacgtc tgaggttatc aataagctcc tagtccagac gccatgggtc
 61 atttcacaga ggaggacaag gctactatca caagcctgtg gggcaaggtg aatgtggaag
121 atgctggagg agaaaccctg ggaaggctcc tggttgtcta cccatggacc cagaggttct
181 ttgacagctt tggcaacctg tcctctgcct ctgccatcat gggcaacccc aaagtcaagg
241 cacatggcaa gaaggtgctg acttccttgg gagatgccac aaagcacctg gatgatctca
301 agggcacctt tgcccagctg agtgaactgc actgtgacaa gctgcatgtg gatcctgaga
361 acttcaagct cctgggaaat gtgctggtga ccgttttggc aatccatttc ggcaaagaat
421 tcacccctga ggtgcaggct tcctggcaga agatggtgac tgcagtggcc agtgccctgt

481 cctccagata ccactgagct cactgcccat gattcagagc tttcaaggat aggctttatt
541 ctgcaagcaa tacaaataat aaatctattc tgctgagaga tcac
```
(SEQ ID NO: 104).

In certain embodiments, the fetal hemoglobin comprises a human hemoglobin subunit gamma-2 (HBG2) having the protein sequence set forth in NCBI Reference Sequence: NP_000175.1 and shown below:

MGHFTEEDKATITSLWGKVNVEDAGGETLGRLLVVYPWTQRFFDSFGNLSSASAIM
GNPKVKAHGKKVLTSLGDAIKHLDDLKGTFAQLSELHCDKLHVDPENFKLLGNVLV
TVLAIHFGKEFTPEVQASWQKMVTGVASALSSRYH (SEQ ID NO: 2).

In certain embodiments, the HBG2 protein is encoded by the polynucleotide sequence set forth in NCBI Reference Sequence: NM_000184.2, NCBI Reference Sequence: NM_000184.3, or shown below:

```
  1 acactcgctt ctggaacgtc tgaggttatc aataagctcc tagtccagac gccatgggtc
 61 atttcacaga ggaggacaag gctactatca caagcctgtg gggcaaggtg aatgtggaag
121 atgctggagg agaaaccctg ggaaggctcc tggttgtcta cccatggacc cagaggttct
181 ttgacagctt tggcaacctg tcctctgcct ctgccatcat gggcaacccc aaagtcaagg
241 cacatggcaa gaaggtgctg acttccttgg gagatgccat aaagcacctg gatgatctca
301 agggcacctt tgcccagctg agtgaactgc actgtgacaa gctgcatgtg gatcctgaga
361 acttcaagct cctgggaaat gtgctggtga ccgttttggc aatccatttc ggcaaagaat
421 tcacccctga ggtgcaggct tcctggcaga agatggtgac tggagtggcc agtgccctgt
481 cctccagata ccactgagct cactgcccat gatgcagagc tttcaaggat aggctttatt
541 ctgcaagcaa tcaaataata aatctattct gctaagagat cacaca
```
(SEQ ID NO: 105).

In certain embodiments, the fetal hemoglobin comprises a human hemoglobin subunit alpha-1 (HBA1) having the protein sequence set forth in NCBI Reference Sequence: NP_000549.1 and shown below:

MVLSPADKTNVKAAWGKVGAHAGEYGAEALERMFLSFPTTKTYFPHFDLSHGSAQ
VKGHGKKVADALTNAVAHVDDMPNALSALSDLHAHKLRVDPVNFKLLSHCLLVTL
AAHLPAEFTPAVHASLDKFLASVSTVLTSKYR (SEQ ID NO: 3).

In certain embodiments, the HBA1 protein is encoded by the polynucleotide sequence set forth in NCBI Reference Sequence: NM_000558.4, NCBI Reference Sequence: NM_000558.5, or shown below:

```
  1 actcttctgg tccccacaga ctcagagaga acccaccatg gtgctgtctc ctgccgacaa
 61 gaccaacgtc aaggccgcct ggggtaaggt cggcgcgcac gctggcgagt atggtgcgga
121 ggccctggag aggatgttcc tgtccttccc caccaccaag acctacttcc cgcacttcga
181 cctgagccac ggctctgccc aggttaaggg ccacggcaag aaggtggccg acgcgctgac
241 caacgccgtg gcgcacgtgg acgacatgcc caacgcgctg tccgccctga gcgacctgca

301 cgcgcacaag cttcgggtgg acccggtcaa cttcaagctc ctaagccact gcctgctggt
361 gaccctggcc gcccacctcc ccgccgagtt cacccctgcg gtgcacgcct ccctggacaa
421 gttcctggct tctgtgagca ccgtgctgac ctccaaatac cgttaagctg gagcctcggt
481 ggccatgctt cttgcccctt gggcctcccc ccagcccctc ctccccttcc tgcacccgta
541 cccccgtggt ctttgaataa agtctgagtg ggcggca (SEQ ID NO: 106).
```

In certain embodiments, the fetal hemoglobin comprises a human hemoglobin subunit alpha-2 (HBA2) having the protein sequence set forth in NCBI Reference Sequence: NP_000508.1 and shown below:

MVLSPADKTNVKAAWGKVGAHAGEYGAEALERMFLSFPTTKTYFPHFDLSHGSAQ
VKGHGKKVADALTNAVAHVDDMPNALSALSDLHAHKLRVDPVNFKLLSHCLLVTL
AAHLPAEFTPAVHASLDKFLASVSTVLTSKYR (SEQ ID NO: 4).

In certain embodiments, the HBA2 protein is encoded by the polynucleotide sequences set forth in NCBI Reference Sequence: NM_000517.4, NCBI Reference Sequence: NM_000517.6, or shown below:

```
  1 actcttctgg tccccacaga ctcagagaga acccaccatg gtgctgtctc ctgccgacaa
 61 gaccaacgtc aaggccgcct ggggtaaggt cggcgcgcac gctggcgagt atggtgcgga
121 ggccctggag aggatgttcc tgtccttccc caccaccaag acctacttcc cgcacttcga
181 cctgagccac ggctctgccc aggttaaggg ccacggcaag aaggtggccg acgcgctgac
241 caacgccgtg gcgcacgtgg acgacatgcc caacgcgctg tccgccctga gcgacctgca
301 cgcgcacaag cttcgggtgg acccggtcaa cttcaagctc ctaagccact gcctgctggt
361 gaccctggcc gcccacctcc ccgccgagtt cacccctgcg gtgcacgcct ccctggacaa
421 gttcctggct tctgtgagca ccgtgctgac ctccaaatac cgttaagctg gagcctcggt
481 agccgttcct cctgcccgct gggcctccca acgggccctc ctcccctcct tgcaccggcc
541 cttcctggtc tttgaataaa gtctgagtgg gcagca (SEQ ID NO: 107).
```

In certain embodiments, the fetal hemoglobin comprises two HBG1 and/or HBG2 proteins and two HBA1 and/or HBA2 proteins.

The methods disclosed herein may be practiced in vitro or in vivo.

The methods disclosed herein comprise contacting a cell with an inhibitor of a target gene, mRNA or protein (which may collectively be referred to as "target") disclosed herein, wherein inhibition of the target results in an increased amount of fetal hemoglobin in the cell, e.g., an erythroid or red blood cell. In particular embodiments, inhibition of the target results in an increased amount of HBG1 or HBG2 in the cell. In particular embodiments, an amount of the inhibitor effective to result in increased levels of Hbγ and/or HbF is used. In particular embodiments, the methods comprise contacting a tissue, organ or organism, e.g., a mammal, with the inhibitor. In certain embodiments, one or more inhibitors, each targeting the same or different targets, may be used.

In certain embodiments, the target gene, mRNA, or protein is Cullin 3 (CUL3). CUL3 is a core component of multiple E3 ubiquitin ligase protein complexes that regulate the ubiquitination of target proteins leading to proteasomal degradation. In some embodiments. CUL3-E3 ubiquitin ligase complexes regulate multiple cellular processes responsible for protein trafficking, stress response, cell cycle regulation, signal transduction, protein quality control, transcription, and DNA replication.

In one aspect, the present disclosure provides methods for increasing the amount of fetal hemoglobin (HbF) in a cell by inhibiting or modulating the expression of CUL3.

In certain embodiments, CUL3 comprises the protein sequence:

MSNLSKGTGSRKDTKMRIRAFPMTMDEKYVNSIWDLLKNAIQEIQRKNNSGLSFEEL
YRNAYTMVLHKHGEKLYTGLREVVTEHLINKVREDVLNSLNNNFLQTLNQAWNDH
QTAMVMIRDILMYMDRVYVQQNNVENVYNLGLIIFRDQVVRYGCIRDHLRQTLLD
MIARERKGEVVDRGAIRNACQMLMILGLEGRSVYEEDFEAPFLEMSAEFFQMESQKF
LAENSASVYIKKVEARINEEIERVMHCLDKSTEEPIVKVVERELISKHMKTIVEMENS
GLVHMLKNGKTEDLGCMYKLFSRVPNGLKTMCECMSSYLREQGKALVSEEGEGKN
PVDYIQGLLDLKSRFDRFLLESFNNDRLFKQTIAGDFEYFLNLNSRSPEYLSLFIDDKL
KKGVKGLTEQEVETILDKAMVLFRFMQEKDVFERYYKQHLARRLLTNKSVSDDSEK
NMISKLKTECGCQFTSKLEGMFRDMSISNTTMDEFRQHLQATGVSLGGVDLTVRVL
TTGYWPTQSATPKCNIPPAPRHAFEIFRRFYLAKHSGRQLTLQHHMGSADLNATFYG
PVKKEDGSEVGVGGAQVTGSNTRKHILQVSTFQMTILMLFNNREKYTFEEIQQETDIP
ERELVRALQSLACGKPTQRVLTKEPKSKEIENGHIFTVNDQFTSKLHRVKIQTVAAK
QGESDPERKETRQKVDDDRKHEIEAAIVRIMKSRKKMQHNVLVAEVTQQLKARFLP
SPVVIKKRIEGLIEREYLARTPEDRKVYTYVA (SEQ ID NO: 108).

In certain embodiments, the target gene, mRNA, or protein is Speckle-type POZ protein (SPOP). In certain embodiments, SPOP is associated with multiple E3 ubiquitin ligase complexes.

In one aspect, the present disclosure provides methods for increasing the amount of fetal hemoglobin (HbF) in a cell by inhibiting or modulating the expression of SPOP.

In certain embodiments, SPOP comprises the protein sequence:

MSRVPSPPPPAEMSSGPVAESWCYTQIKVVKFSYMWTINNFSFCREEMGEVIKSSTFS
SGANDKLKWCLRVNPKGLDEESKDYLSLYLLLVSCPKSEVRAKFKFSILNAKGEETK
AMESQRAYRFVQGKDWGFKKFIRRDFLLDEANGLLPDDKLTLFCEVSVVQDSVNIS
GQNTMNMVKVPECRLADELGGLWENSRFTDCCLCVAGQEFQAHKAILAARSPVFS
AMFEHEMEESKKNRVEINDVEPEVFKEMMCFIYTGKAPNLDKMADDLLAAADKYA

LERLKVMCEDALCSNLSVENAAEILILADLHSADQLKTQAVDFINYHASDVLETSGW
KSMVVSHPHLVAEAYRSLASAQCPFLGPPRKRLKQS (SEQ ID NO: 109).

The term "inhibitor" may refer to any agent that inhibits the expression or activity of a target gene, mRNA and/or protein in a cell, tissue, organ, or subject. The expression level or activity of target mRNA and/or protein in a cell may be reduced via a variety of means, including but not limited to reducing the total amount of target protein or inhibiting one or more activity of the target protein. In various embodiments, an inhibitor may inhibit the expression of a target gene, target mRNA, or a target protein, and/or an inhibitor may inhibit a biological activity of a target protein. In certain embodiments, the biological activity is kinase activity. For example, an inhibitor may competitively bind to the ATP-binding site of a kinase and inhibit its kinase activity, or it may allosterically block the kinase activity. In certain embodiments, an inhibitor causes increased degradation of a target protein. In particular embodiments, the inhibitor inhibits any of the target genes or proteins identified in Table 1, Table 2, Table 6, Table 7, Table 8, or Table 9, or any component or subunit of any of the complexes identified in Table 3 or Table 4 or pathways identified in Table 5. Methods for determining the expression level or the activity of a target gene or polypeptide are known in the art and include, e.g.. RT-PCR and FACS.

In particular embodiments, an inhibitor directly inhibits expression of or an activity of a target gene, mRNA, or protein, e.g., it may directly bind to the target gene, mRNA or protein. In some embodiments, the inhibitor indirectly inhibits expression of or an activity of a target gene, mRNA, or protein, e.g.. it may bind to and inhibit a protein that mediates expression of the target gene, mRNA, or protein (such as a transcription factor), or it may bind to and inhibit expression of an activity of another protein involved in the activity of the target protein (such as another protein present in a complex with the target protein).

In certain embodiments, the inhibitor inhibits SPOP or a protein complex to which SPOP is permanently or transiently associated. In certain embodiments, the protein complex is an SPOP-associated E3 ubiquitin ligase complex. In particular embodiments, the complex comprises Core histone macro-H2A.1 (H2AFY), SPOP, and CUL3: DNA damage-binding protein 1 (DDB1). DNA damage-binding protein 2 (DDB2), Cullin-4A (CUL4A), Cullin-4B (CUL4B), and E3 ubiquitin protein ligase RBX1 (RBX); or Polycomb complex protein BMI-1 (BMI1), SPOP, and CUL3; SPOP, Death domain-associated protein 6 (DAXX), and CUL3; Core histone macro-H2A.1 (H2AFY), SPOP, and CUL3; or BMI1, SPOP, and CUL3. In particular embodiments, the inhibitor inhibits one or more component of any of these complexes. In some embodiments, the inhibitor inhibits expression of SPOP, while in other embodiments, the inhibitor inhibits an activity of SPOP.

In certain embodiments, the inhibitor inhibits CUL3 or a protein complex to which CUL3 is permanently or transiently associated. In certain embodiments, the protein complex is a CUL3-associated E3 ubiquitin ligase complex. In certain embodiments, the CUL3-associated protein complex is a D(4) dopamine receptor (DRD4)-Kelch like protein 12 (KLH12)-CUL3. In certain embodiments, the CUL3-associated protein complex is a coiled coil domain containing protein 22 (CCDC22)-COMM domain containing protein 8 (COMMD8)-CUL3 complex. In certain embodiments, the CUL3-associated protein complex is a Cullin associated NEDD8 dissociated protein (CAND1)-CUL3-E3 ubiquitin protein ligase RBX1 (RBX1). In some embodiments, the complex comprises SPOP, Death domain-associated protein 6 (DAXX), and CUL3; Core histone macro-H2A.1 (H2AFY), SPOP, and CUL3; DNA damage-binding protein 1 (DDB1), DNA damage-binding protein 1 (DDB2), Cullin-4A (CUL4A), Cullin-4B (CUL4B), and E3 ubiquitin-protein ligase RBX1 (RBX1); Polycomb complex protein BMI-1 (BMI1), SPOP, and CUL3: COP9 signalosome complex subunit 1 (CSN1), COP9 signalosome complex subunit 8 (CSN8). Hairy/enhancer-of-split related with YRPW motif protein 1 (HRT1), S-phase kinase-associated protein 1 (SKP1). S-phase kinase-associated protein 2 (SKP2), Cullin-1 (CUL1), Cullin-2 (CUL2). and CUL3; CUL3, Kelch-like protein 3 (KLHL3), and Serine/threonine-protein kinase WNK4 (WNK4); CUL3. KLHL3, and Serine/threonine-protein kinase WNK1 (WNK1); CUL3 and KLHL3. In particular embodiments, the inhibitor inhibits one or more component of any of these complexes. In some embodiments, the inhibitor inhibits expression of CUL3, while in other embodiments, the inhibitor inhibits an activity of CUL3.

In one embodiment, a method of increasing the amount of fetal hemoglobin in a cell, tissue, organ or subject comprises contacting the cell, tissue, organ, or subject with an agent that results in a reduced amount of one or more target genes, mRNAs, or proteins in a cell. In certain embodiments, the agent inhibits the expression or activity of one or more target gene, mRNA. or polypeptide in a cell or tissue. In certain embodiments, the agent causes increased degradation of one or more target gene, mRNA, or polypeptide. In particular embodiments, the cell or tissue is contacted with an amount of the agent effective to reduce the expression or activity of one or more target genes. mRNAs. or polypeptides in the cell or tissue. In certain embodiments, the cell or tissue is contacted with an amount of the agent effective to reduce the amount of active target protein in the cell or tissue. In particular embodiments, the cells are hematopoietic cells, e.g., red blood cells. In certain embodiments, the cells are terminally differentiated, e.g., terminally differentiated red blood cells.

In certain embodiments of any of the methods disclosed herein, the cells comprise one or more mutations associated with a blood cell disorder, e.g., SCD or β-thalassemia. In certain embodiments of any of the methods disclosed herein, the cells have a reduced amount of functionally active HbA as compared to a control cell, e.g., a non-disease cell. In particular embodiments, the cells are associated with a blood cell disorder, e.g., SCD or β-thalassemia. For example, the cells may be derived from or obtained from cells or tissue from a subject diagnosed with the blood cell disorder. In particular embodiments, the methods are practiced on a subject diagnosed with a blood cell disorder, e.g., SCD or β-thalassemia. Methods disclosed herein may be practiced in vitro or in vivo.

In a related aspect. the disclosure includes a method of treating or preventing a blood cell disease or disorder associated with reduced amounts of functionally active HbA (or total HbA) in a subject in need thereof, comprising providing to a subject an agent that inhibits the expression or activity of one or more target protein in the subject, or in certain cells or tissue of the subject, wherein the treatment results in an increased amount of HbF in the subject or one or more cells or tissues of the subject e.g., hematopoietic cell, e.g.. an erythrocyte or red blood cell. In certain embodiments, the agent is present in a pharmaceutical composition. In some embodiments, the subject is provided with one or more (e.g., two, three, or more) agents that inhibits the expression or activity of one or more target protein in the subject, or in certain cells or tissue of the subject. In some embodiments the two or more agents inhibit the same target or target complex disclosed herein, whereas in other embodiments, the two or more agents inhibit different targets or target complexes disclosed herein. In certain embodiments, the cells are terminally differentiated, e.g., terminally differentiated red blood cells. In some embodiments, the agent inhibits the expression or activity of the one or more target protein. In certain embodiments, the agent induces degradation of the one or more target protein. In certain embodiments, the agent inhibits activity of the one or more target protein. In particular embodiments of any of the methods, the inhibitor reduces expression of one or more target genes, mRNAs or proteins in cells or tissue of the subject, e.g., hematopoietic cells, e.g., red blood cells. In particular embodiments, the inhibitor inhibits any of the target genes or proteins identified in Table 1, Table 2, Table 6, Table 7. Table 8, or Table 9, or any component or subunit of any of the complexes identified in Table 3 or Table 4 or pathways identified in Table 5.

In particular embodiments of methods of treatment disclosed herein, the blood disease or disorder is selected from Sickle Cell Disease. β-thalassemia, Beta thalassemia trait or beta thalassemia minor, Thalassemia intermedia, Thalassemia major or Cooley's Anemia.

In particular embodiments of any of the methods described herein, the pharmaceutical composition is provided to the subject parenterally.

Inhibitors and/or other agents and compositions (e.g., inhibitors) described herein can be formulated in any manner suitable for a desired administration route (e.g., parenteral or oral administration). In some embodiments, contacting an agent or composition with a cell and/or tissue is a result of administration of or providing an agent or composition to a subject. In some embodiments, an agent or composition (e.g., an inhibitor) is administered at least 1, 2. 3, 4, 5, 10, 15, 20, or more times. In some embodiments of combination therapies, administration of a first agent or composition is followed by or occurs overlapping with or concurrently with the administration of a second agent or composition. The first and second agent or composition may be the same or they may be different. In some embodiments, the first and second agents or compositions are administered by the same actor and/or in the same geographic location. In some embodiments, the first and second agents or compositions are administered by different actors and/or in different geographical locations. In some embodiments, multiple agents described herein are administered as a single composition.

A wide variety of administration methods may be used in conjunction with the inhibitors according to the methods disclosed herein. For example, inhibitors may be administered or coadministered topically, orally, intraperitoneally, intravenously, intraarterially, transdermally, sublingually, intramuscularly, rectally, transbuccally, intranasally, liposomally, via inhalation, vaginally, intraoccularly, via local delivery (for example by catheter or stent), subcutaneously, intraadiposally, intraarticularly, intrathecally, transmucosally, pulmonary, or parenterally, for example, by injection, including subcutaneous, intradermal, intramuscular, intravenous, intraarterial, intracardiac, intrathecal, intraspinal, intracapsular, subcapsular, intraorbital, intraperitoneal, intratracheal, subcuticular, intraarticular, subarachnoid, and intrasternal: by implant of a depot or reservoir, for example, subcutaneously or intramuscularly.

"Subjects" includes animals (e.g., mammals, swine, fish, birds, insects etc.). In some embodiments, subjects are mammals, particularly primates, especially humans. In some embodiments, subjects are livestock such as cattle, sheep, goats, cows, swine, and the like; poultry such as chickens, ducks, geese, turkeys, and the like; and domesticated animals such as dogs and cats. In some embodiments (e.g., particularly in research contexts) subjects are rodents (e.g., mice, rats, hamsters), rabbits, primates, or swine such as inbred pigs and the like. The terms "subject" and "patient" are used interchangeably herein.

"Tissue" is an ensemble of similar cells from the same origin that together carry out a specific function.

Methods disclosed herein may be practiced with any agent capable of inhibiting expression or activity of a target gene, mRNA or protein. e.g., an inhibitor of a gene, mRNA or protein, complex or pathway disclosed herein, e.g., in any of Tables 1-9.

In particular embodiments, methods disclosed herein result in a decrease in an expression level or activity of a target gene, mRNA or protein in one or more cells or tissues (e.g., within a subject), e.g., as compared to the expression level or activity in control cells or tissue not contacted with the inhibitor, or a reference level. "Decrease" refers to a decrease of at least 5%, for example, at least 5, 6, 7. 8, 9, 10, 15, 20, 25. 30, 35. 40, 45, 50. 55, 60. 65, 70, 75, 80, 85, 90, 95, 99 or 100%, for example, as compared to the reference level. Decrease also means decreases by at least 1-fold, for example, 1, 2, 3, 4, 5, 6, 7, 8, 9, 10, 15, 20, 30, 40, 50, 60. 70, 80, 90, 100, 200, 500. 1000-fold or more, for example, as compared to the level of a reference or control cells or tissue.

In particular embodiments, methods disclosed herein result in increased amounts of HbF or HBγ in one or more cells or tissues (e.g., within a subject), e.g., as compared to the expression level or activity in control cells or tissue not contacted with the inhibitor, or a reference level. In particular embodiments, methods disclosed herein result in increased expression of a hemoglobin gamma (e.g., HBG1 or HBG2) in one or more cells or tissues (e.g., within a subject), e.g., as compared to the expression level in control cells or tissue not contacted with the inhibitor, or a reference level. "Increase" refers to an increase of at least 5%, for example, at least 5, 6, 7, 8, 9. 10. 15, 20, 25. 30, 35, 40. 45, 50, 55, 60. 65, 70, 75. 80, 85, 90, 95, 99 or 100%, or an at least two-fold, three-fold, give-fold, ten-fold, 20-fold, 50-fold, 100-fold, 500-fold or 1000-fold increase, for example, as compared to the reference level or level in control cells or tissue.

Methods described herein may be practiced using any type of inhibitor that results in a reduced amount or level of a target gene, mRNA or protein, e.g., in a cell or tissue, e.g., a cell or tissue in a subject. In particular embodiments, the inhibitor causes a reduction in active target protein, a reduction in total target protein, a reduction in target mRNA levels, and/or a reduction in target protein activity, e.g., in a cell or tissue contacted with the inhibitor. In certain embodiments, the reduction is at least 5%, at least 10%, at least 20%, at least 30%, at least 40%, at least 50%, at least 60%, at least 70%, at least 80%, or at least 90%, as compared to the level in the same type of cell or tissue not contacted with the inhibitor or a reference level. Methods of measuring total protein or mRNA levels, or activity, in a cell are known in the art. In certain embodiments, the inhibitor inhibits or reduces target protein activity or expression, e.g.. mRNA and/or protein expression. In certain embodiments, the inhibitor causes increased degradation of the target protein, resulting in lower amounts of target protein in a cell or tissue.

Inhibitors that may be used to practice the disclosed methods include but are not limited to agents that inhibit or reduce or decrease the expression or activity of a biomolecule, such as but not limited to a target gene, mRNA or protein. In certain embodiments, an inhibitor can cause increased degradation of the biomolecule. In particular embodiments, an inhibitor can inhibit a biomolecule by competitive, uncompetitive, or non-competitive means. Exemplary inhibitors include, but are not limited to, nucleic acids, DNA, RNA. gRNA. shRNA, siRNA, modified mRNA (mRNA), microRNA (miRNA). proteins, protein mimetics, peptides, peptidomimetics, antibodies, small molecules, small organic molecules, inorganic molecules, chemicals, analogs that mimic the binding site of an enzyme, receptor, or other protein, e.g., that is involved in signal transduction, therapeutic agents, pharmaceutical compositions, drugs, and combinations of these. In some embodiments, the inhibitor can be a nucleic acid molecule including, but not limited to, siRNA that reduces the amount of functional protein in a cell. Accordingly, compounds or agents said to be "capable of inhibiting" a particular target protein comprise any type of inhibitor.

In particular embodiments, an inhibitor comprises a nucleic acid that binds to a target gene or mRNA. Accordingly, a nucleic acid inhibitor may comprise a sequence complementary to a target polynucleotide sequence, or a region thereof, or an antisense thereof. In particular embodiments, a nucleic acid inhibitor comprises at least 8, at least 10, at least 12, at least 14, at least 16, at least 20, at least 24, or at least 30 nucleotide sequence corresponding to or complementary to a target polynucleotide sequence or antisense thereof.

In certain embodiments, a nucleic acid inhibitor is an RNA interference or antisense RNA agent or a portion or mimetic thereof, or a morpholino, that decreases the expression of a target gene when administered to a cell. Typically, a nucleic acid inhibitor comprises at least a portion of a target nucleic acid molecule, or an ortholog thereof, or comprises at least a portion of the complementary strand of a target nucleic acid molecule. In some embodiments, expression of a target gene is reduced by at least about 10%, at least about 25%, at least about 50%, at least about 75%, or even 90-100%.

A "complementary" nucleic acid sequence is a nucleic acid sequence capable of hybridizing with another nucleic acid sequence comprised of complementary nucleotide base pairs. By "hybridize" is meant pair to form a double-stranded molecule between complementary nucleotide bases (e.g., adenine (A) forms a base pair with thymine (T), as does guanine (G) with cytosine (C) in DNA) under suitable conditions of stringency. (See, e.g., Wahl. G. M. and S. L. Berger (1987) Methods Enzymol. 152:399; Kimmel, A. R. (1987) Methods Enzymol. 152:507).

"Antisense" refers to a nucleic acid sequence, regardless of length, that is complementary to a nucleic acid sequence. In certain embodiments, antisense RNA refers to single stranded RNA molecules that can be introduced to an individual cell, tissue, or subject and results in decreased expression of a target gene through mechanisms that do not rely on endogenous gene silencing pathways. An antisense nucleic acid can contain a modified backbone, for example, phosphorothioate, phosphorodithioate, or others known in the art, or may contain non-natural internucleoside linkages. Antisense nucleic acid can comprise, e.g., locked nucleic acids (LNA).

"RNA interference" as used herein refers to the use of agents that decrease the expression of a target gene by degradation of a target mRNA through endogenous gene silencing pathways (e.g., Dicer and RNA-induced silencing complex (RISC)). RNA interference may be accomplished using various agents, including shRNA and siRNA. "Short hairpin RNA" or "shRNA'' refers to a double stranded, artificial RNA molecule with a hairpin turn that can be used to silence target gene expression via RNA interference (RNAi). Expression of shRNA in cells is typically accomplished by delivery of plasmids or through viral or bacterial vectors. shRNA is an advantageous mediator of RNAi in that it has a relatively low rate of degradation and turnover. Small interfering RNA (siRNA) is a class of double-stranded RNA molecules, usually 20-25 base pairs in length, similar to miRNA, and operating within the RNA interference (RNAi) pathway. It interferes with the expression of specific genes with complementary nucleotide sequences by degrading mRNA after transcription, preventing translation. In certain embodiments, an siRNA is 18, 19, 20, 21, 22, 23 or 24 nucleotides in length and has a 2 base overhang at its 3' end. siRNAs can be introduced to an individual cell and/or culture system and result in the degradation of target mRNA sequences. "114orpholino" as used herein refers to a modified nucleic acid oligomer wherein standard nucleic acid bases are bound to morpholine rings and are linked through phosphorodiamidate linkages. Similar to siRNA and shRNA, morpholinos bind to complementary mRNA sequences. However, morpholinos function through steric-inhibition of mRNA translation and alteration of mRNA splicing rather than targeting complementary mRNA sequences for degradation.

In certain embodiments, a nucleic acid inhibitor is a messenger RNA that may be introduced into a cell, wherein it encodes a polypeptide inhibitor of a target disclosed herein. In particular embodiments, the mRNA is modified, e.g., to increase its stability or reduce its immunogenicity, e.g., by the incorporation of one or more modified nucleosides. Suitable modifications are known in the art.

In certain embodiments, an inhibitor comprises an expression cassette that encodes a polynucleotide or polypeptide inhibitor of a target disclosed herein. In particular embodiments, the expression cassette is present in a gene therapy vector, for example a viral gene therapy vector. A variety of gene therapy vectors, including viral gene therapy vectors are known in the art, including, for example, AAV-based gene therapy vectors.

In some embodiments, an inhibitor is a polypeptide inhibitor. In particular embodiments, a polypeptide inhibitor binds to a target polypeptide, thus inhibiting its activity, e.g., kinase activity. Examples of polypeptide inhibitors include any types of polypeptides (e.g., peptides and proteins), such as antibodies and fragments thereof.

An "antibody" is an immunoglobulin (Ig) molecule capable of specific binding to a target, such as a carbohydrate, polynucleotide, lipid, or polypeptide, through at least one epitope recognition site, located in the variable region of the Ig molecule. As used herein, the term encompasses not only intact polyclonal or monoclonal antibodies, but also fragments thereof, such as dAb, Fab, Fab'. $F(ab')_2$, Fv, single chain (scFv), synthetic variants thereof, naturally occurring variants, fusion proteins comprising an antibody portion with an antigen-binding fragment of the required specificity, chimeric antibodies, nanobodies, and any other modified configuration of the immunoglobulin molecule that comprises an antigen-binding site or fragment of the required specificity.

"Fragment" refers to a portion of a polypeptide or nucleic acid molecule. This portion contains, preferably, at least 10%, 20%, 30%, 40%, 50%, 60%, 70%, 80%, or 90% of the entire length of the reference nucleic acid molecule or polypeptide. A fragment may contain 10, 20, 30, 40, 50, 60, 70, 80, 90, or 100, 200, 300, 400, 500, 600, 700, 800, 900, or 1000 nucleotides or amino acids. A "functional fragment" of an antibody is a fragment that maintains one or more activities of the antibody, e.g., it binds the same epitope and or possesses a biological activity of the antibody. In particular embodiments, a functional fragment comprises the six CDRs present in the antibody.

In certain embodiments, the inhibitor induces degradation of a target polypeptide. For example, inhibitors include proteolysis targeting chimeras (PROTAC), which induce selective intracellular proteolysis of target proteins. PROTACs include functional domains. which may be covalently linked protein-binding molecules: one is capable of engaging an E3 ubiquitin ligase, and the other binds to the target protein meant for degradation. Recruitment of the E3 ligase to the target protein results in ubiquitination and subsequent degradation of the target protein by the proteasome. In particular embodiments, an inhibitor is a PROTAC that targets any of the targets disclosed herein.

In certain embodiments, an inhibitor is a small molecule inhibitor, or a stereoisomer, enantiomer, diastereomer, isotopically-enriched, pro-drug, or pharmaceutically acceptable salt thereof. In certain embodiments the small molecule inhibitor of a target protein or protein complex that functions to regulate HbF expression targets SPOP. In certain embodiments the small molecule inhibitor of a target protein or protein complex that functions to regulate HbF expression targets CUL3. In certain embodiments, the CUL3 inhibitor is MLN4924 (CAS No: 905579-51-3), suramin (CAS NO: 145-63-1) or DI-591 (CAS No: 2245887-38-9).

In certain embodiments, the inhibitor comprises one or more components of a gene editing system. As used herein, the term "gene editing system" refers to a protein, nucleic acid, or combination thereof that is capable of modifying a target locus of an endogenous DNA sequence when introduced into a cell. Numerous gene editing systems suitable for use in the methods of the present invention are known in the art including, but not limited to, zinc-finger nuclease systems. TALEN systems, and CRISPR/Cas systems.

In some embodiments, the gene editing system used in the methods described herein is a CRISPR (Clustered Regularly Interspaced Short Palindromic Repeats)/Cas (CRISPR Associated) nuclease system, which is an engineered nuclease system based on a bacterial system that can be used for mammalian genome engineering. Generally, the system comprises a CRISPR-associated endonuclease (for example, a Cas endonuclease) and a guide RNA (gRNA). The gRNA is comprised of two parts: a crispr-RNA (crRNA) that is specific for a target genomic DNA sequence, and a trans-activating RNA (tracrRNA) that facilitates endonuclease binding to the DNA at the targeted insertion site. In some embodiments, the crRNA and tracrRNA may be present in the same RNA oligonucleotide, referred to as a single guide-RNA (sgRNA). In some embodiments, the crRNA and tracrRNA may be present as separate RNA oligonucleotides. In such embodiments, the gRNA is comprised of a crRNA oligonucleotide and a tracrRNA oligonucleotide that associate to form a crRNA:tracrRNA duplex. As used herein, the term "guide RNA" or "gRNA" refers to the combination of a tracrRNA and a crRNA, present as either an sgRNA or a crRNA:tracrRNA duplex.

In some embodiments, the CRISPR/Cas systems comprise a Cas protein, a crRNA, and a tracrRNA. In some embodiments, the crRNA and tracrRNA are combined as a duplex RNA molecule to form a gRNA. In some embodiments, the crRNA:tracrRNA duplex is formed *in vitro* prior to introduction to a cell. In some embodiments, the crRNA and tracrRNA are introduced into a cell as separate RNA molecules and crRNA:tracrRNA duplex is then formed intracellularly. In some embodiments, polynucleotides encoding the crRNA and tracrRNA are provided. In such embodiments, the polynucleotides encoding the crRNA and tracrRNA are introduced into a cell and the crRNA and tracrRNA molecules are then transcribed intracellularly. In some embodiments, the crRNA and tracrRNA are encoded by a single polynucleotides. In some embodiments, the crRNA and tracrRNA are encoded by separate polynucleotides.

In some embodiments, a Cas endonuclease is directed to the target insertion site by the sequence specificity of the crRNA portion of the gRNA, which may include a protospacer motif (PAM) sequence near the target insertion site. A variety of PAM sequences suitable for use with a particular endonuclease (*e.g.,* a Cas9 endonuclease) are known in the art (*See e.g.,* Nat Methods. 2013 Nov; 10(11): 1116-1121 and Sci Rep. 2014; 4: 5405).

The specificity of a gRNA for a target locus is mediated by the crRNA sequence, which comprises a sequence of about 20 nucleotides that are complementary to the DNA sequence at a target locus. e.g.. complementary to a target DNA sequence. In some embodiments, the crRNA sequences used in the methods of the present invention are at least 90% complementary to a DNA sequence of a target locus. In some embodiments, the crRNA sequences used in the methods of the present invention are at least 95%, 96%, 97%, 98%, or 99% complementary to a DNA sequence of a target locus. In some embodiments, the crRNA sequences used in the methods of the present invention are 100% complementary to a DNA sequence of a target locus. In some embodiments, the crRNA sequences described herein are designed to minimize off-target binding using algorithms known in the art (*e.g.*, Cas-OFF finder) to identify target sequences that are unique to a particular target locus or target gene.

In some embodiments, the endonuclease is a Cas protein or ortholog. In some embodiments, the endonuclease is a Cas9 protein. In some embodiments, the Cas9 protein is derived from *Streptococcus pyogenes* (*e.g.,* SpCas9), *Staphylococcus aureus* (*e.g.,* SaCas9), or *Neisseria meningitides* (NmeCas9). In some embodiments, the Cas endonuclease is a Cas9 protein or a Cas9 ortholog and is selected from the group consisting of SpCas9, SpCas9-HF1, SpCas9-HF2, SpCas9-HF3, SpCas9-HF4, SaCas9, FnCpf, FnCas9, eSpCas9, and NmeCas9. In some embodiments, the endonuclease is selected from the group consisting of C2C1, C2C3, Cpfl (also referred to as Cas12a), Cas1, CaslB, Cas2, Cas3, Cas4, Cas5, Cas6, Cas7, Cas8, Cas9 (also known as Csnl and Csx12), Cas10, Csy1, Csy2, Csy3, Cse1, Cse2, Csd, Csc2, Csa5, Csn2, Csm2, Csm3, Csm4, Csm5, Csm6, Cmrl, Cmr3, Cmr4, Cmr5, Cmr6, Csb1, Csb2, Csb3, Csxl7. Csxl4, Csx10, Csxl6, CsaX, Csx3, Csx1, Csxl5, Csf1, Csf2, Csf3, and Csf4. In some embodiments, the Cas9 is a Cas9 nickase mutant. Cas9 nickase mutants comprise only one catalytically active domain (either the HNH domain or the RuvC domain).

In particular aspects, the disclosure includes compositions, e.g., pharmaceutical compositions, comprising an inhibitor of a target disclosed herein, including any of the various classes of inhibitors described herein. The invention encompasses pharmaceutical compositions comprising an inhibitor and a pharmaceutically acceptable carrier, diluent or excipient. Any inert excipient that is commonly used as a carrier or diluent may be used in compositions of the present invention, such as sugars, polyalcohols, soluble polymers, salts and lipids. Sugars and polyalcohols which may be employed include, without limitation, lactose, sucrose. mannitol. and sorbitol. Illustrative of the soluble polymers which may be employed are polyoxyethylene, poloxamers. polyvinylpyrrolidone, and dextran. Useful salts include, without limitation, sodium chloride, magnesium chloride, and calcium chloride. Lipids which may be employed include, without limitation, fatty acids, glycerol fatty acid esters, glycolipids, and phospholipids.

In addition, the pharmaceutical compositions may further comprise binders (e.g., acacia, cornstarch, gelatin, carbomer, ethyl cellulose, guar gum, hydroxypropyl cellulose, hydroxypropyl methyl cellulose, povidone). disintegrating agents (e.g., cornstarch, potato starch, alginic acid, silicon dioxide, croscarmellose sodium, crospovidone, guar gum, sodium starch glycolate. Primogel), buffers (e.g., tris-HCL, acetate, phosphate) of various pH and ionic strength, additives such as albumin or gelatin to prevent absorption to surfaces, detergents (e.g., Tween 20, Tween 80, Pluronic F68, bile acid salts), protease inhibitors, surfactants (e.g., sodium lauryl sulfate), permeation enhancers. solubilizing agents (e.g., glycerol, polyethylene glycerol, cyclodextrins), a glidant (e.g., colloidal silicon dioxide), anti-oxidants (e.g., ascorbic acid, sodium metabisulfite, butylated hydroxyanisole), stabilizers (e.g., hydroxypropyl cellulose, hydroxypropylmethyl cellulose), viscosity increasing agents (e.g.. carbomer. colloidal silicon dioxide, ethyl cellulose, guar gum), sweeteners (e.g., sucrose, aspartame, citric acid), flavoring agents (e.g., peppermint, methyl salicylate, or orange flavoring), preservatives (e.g.. thimerosal, benzyl alcohol, parabens), lubricants (e.g., stearic acid, magnesium stearate, polyethylene glycol, sodium lauryl sulfate), flow-aids (e.g., colloidal silicon dioxide), plasticizers (e.g., diethyl phthalate, triethyl citrate), emulsifiers (e.g., carbomer, hydroxypropyl cellulose, sodium lauryl sulfate, methyl cellulose, hydroxyethyl cellulose, carboxymethylcellulose sodium), polymer coatings (e.g., poloxamers or poloxamines), coating and film forming agents (e.g., ethyl cellulose, acrylates, polymethacrylates) and/or adjuvants.

In one embodiment, the pharmaceutical compositions are prepared with carriers that will protect the inhibitor against rapid elimination from the body, such as a controlled release formulation, including implants and microencapsulated delivery systems. Biodegradable, biocompatible polymers can be used, such as ethylene vinyl acetate, polyanhydrides, polyglycolic acid, collagen, polyorthoesters, and polylactic acid. Methods for preparation of such formulations will be apparent to those skilled in the art. The materials can also be obtained commercially from Alza Corporation and Nova Pharmaceuticals, Inc. Liposomal suspensions (including liposomes targeted to infected cells with monoclonal antibodies to viral antigens) can also be used as pharmaceutically acceptable carriers. These can be prepared according to methods known to those skilled in the art, for example, as described in U.S. Pat. No. 4,522,811.

Additionally, the invention encompasses pharmaceutical compositions comprising any solid or liquid physical form of an inhibitor. For example, the inhibitor can be in a crystalline form, in amorphous form, and have any particle size. The particles may be micronized, or may be agglomerated, particulate granules, powders, oils, oily suspensions or any other form of solid or liquid physical form.

When inhibitors exhibit insufficient solubility, methods for solubilizing the compounds may be used. Such methods are known to those of skill in this art, and include, but are not limited to, pH adjustment and salt formation, using co-solvents, such as ethanol, propylene glycol, polyethylene glycol (PEG) 300, PEG 400, DMA (10-30%), DMSO (10-20%), NMP (10-20%), using surfactants, such as polysorbate 80, polysorbate 20 (1-10%), cremophor EL, Cremophor RH40, Cremophor RH60 (5-10%), Pluronic F68/Poloxamer 188 (20-50%), Solutol HS15 (20-50%), Vitamin E TPGS. and d-a-tocopheryl PEG 1000 succinate (20-50%), using complexation such as HP β-CD and SBE β-CD (10-40%), and using advanced approaches such as micelles, addition of a polymer, nanoparticle suspensions, and liposome formation.

Inhibitors may also be administered or coadministered in slow release dosage forms. Inhibitors may be in gaseous, liquid, semi-liquid or solid form, formulated in a manner suitable for the route of administration to be used. For oral administration, suitable solid oral formulations include tablets, capsules, pills, granules, pellets, sachets and effervescent, powders, and the like. Suitable liquid oral formulations include solutions, suspensions, dispersions, syrups, emulsions, oils and the like. For parenteral administration, reconstitution of a lyophilized powder is typically used.

Suitable doses of the inhibitors for use in treating the diseases or disorders described herein can be determined by those skilled in the relevant art. Therapeutic doses are generally identified through a dose ranging study in humans based on preliminary evidence derived from the animal studies. Doses should be sufficient to result in a desired therapeutic benefit without causing unwanted side effects. Mode of administration, dosage forms and suitable pharmaceutical excipients can also be well used and adjusted by those skilled in the art. All changes and modifications are envisioned within the scope of the present patent application.

In certain embodiments, the disclosure includes unit dosage forms of a pharmaceutical composition comprising an agent that inhibits expression or activity of a target polypeptide (or results in reduced levels of a target protein) and a pharmaceutically acceptable carrier, diluent or excipient, wherein the unit dosage form is effective to increase expression of a hemoglobin gamma in one or more tissue in a subject to whom the unit dosage form is administered.

In particular embodiments, the unit dosage forms comprise an effective amount, an effective concentration, and/or an inhibitory concentration, of an inhibitor to treat a blood cell disease or disorder, e.g., one associated with mutant or aberrant hemoglobin beta, including any of the diseases or disorders disclosed herein, e.g., SCD or β-thalasscmias.

"Pharmaceutical compositions" include compositions of one or more inhibitors disclosed herein and one or more pharmaceutically acceptable carrier, excipient, or diluent.

"Pharmaceutically acceptable" is employed herein to refer to those compounds, materials, compositions, and/or dosage forms which are, within the scope of sound medical judgment, suitable for use in contact with the tissues of human beings and animals without excessive toxicity, irritation, allergic response, or other problem or complication, commensurate with a reasonable benefit/risk ratio.

"Pharmaceutically acceptable carrier" includes without limitation any adjuvant, carrier, excipient, glidant, sweetening agent, diluent, preservative, dyc/colorant, flavor enhancer, surfactant, wetting agent, dispersing agent, suspending agent, stabilizer, isotonic agent, solvent, surfactant, and/or emulsifier which has been approved by the United States Food and Drug Administration as being acceptable for use in humans and/or domestic animals. Exemplary pharmaceutically acceptable carriers include, but are not limited to, to sugars, such as lactose, glucose and sucrose; starches, such as corn starch and potato starch; cellulose, and its derivatives, such as sodium carboxymethyl cellulose, ethyl cellulose and cellulose acetate; tragacanth: malt; gelatin; talc: cocoa butter, waxes, animal and vegetable fats, paraffins, silicones, bentonites, silicic acid, zinc oxide; oils, such as peanut oil, cottonseed oil, safflower oil, sesame oil, olive oil, corn oil and soybean oil; glycols, such as propylene glycol; polyols, such as glycerin, sorbitol, mannitol and polyethylene glycol; esters, such as ethyl oleate and ethyl laurate; agar; buffering agents, such as magnesium hydroxide and aluminum hydroxide: alginic acid; pyrogen- free water; isotonic saline; Ringer's solution; ethyl alcohol: phosphate buffer solutions; and any other compatible substances employed in pharmaceutical formulations. Except insofar as any conventional media and/or agent is incompatible with the agents of the present disclosure, its use in therapeutic compositions is contemplated. Supplementary active ingredients also can be incorporated into the compositions.

"Effective amount" as used herein refers to an amount of an agent effective in achieving a particular effect, e.g., increasing levels of fetal hemoglobin (or a hemoglobin gamma) in a cell, tissue, organ or subject. In certain embodiments, the increase is at least 10%, at least 20%, at least 30%, at least 40%, at least 50%, at least 60%, or at least 70%, as compared to the amount prior to or without treatment. In the context of therapeutic treatment of a subject, an effective amount may be, e.g., an amount effective or sufficient to reduce one or more disease symptoms in the subject, e.g., a subject with sickle cell disease.

"Effective Concentration" as used herein refers to the minimum concentration (mass/volume) of an agent and/or composition required to result in a particular physiological effect. As used herein, effective concentration typically refers to the concentration of an agent required to increase, activate, and/or enhance a particular physiological effect.

"Inhibitory Concentration" "Inhibitory Concentration" is the minimum concentration (mass/volume) of an agent required to inhibit a particular physiological effect. As used herein, inhibitory concentration typically refers to the concentration of an agent required to decrease, inhibit, and/or repress a particular physiological effect.

In some embodiments, an agent or compound described herein may be administered at a dosage from about 1 mg/kg to about 300 mg/kg. In another embodiment, an agent or compound described herein may be administered at a dosage from about 1 mg/kg to about 20 mg/kg. For example, the agent or compound may be administered to a subject at a dosage of 1, 2. 3, 4, 5, 6, 7, 8, 9, 10, 11, 12, 13, 14, 15, 16, 17, 18, 19 or 20 mg/kg, or within a range between any of the proceeding values, for example, between about 10 mg/kg and about 15 mg/kg, between about 6 mg/kg and about 12 mg/kg, and the like. In another embodiment, an agent or compound described herein is administered at a dosage of ≤15 mg/kg. For example, an agent or compound may be administered at 15 mg/kg per day for 7 days for a total of 105 mg/kg per week. For example, a compound may be administered at 10 mg/kg twice per day for 7 days for a total of 140 mg/kg per week.

In many embodiments, the dosages described herein may refer to a single dosage, a daily dosage, or a weekly dosage. In one embodiment, an agent or compound may be administered once per day. In another embodiment, a compound may be administered twice per day. In some embodiments, an agent or compound may be administered three times per day. In some embodiments, a compound may be four times per day. In some embodiments, an agent or compound described herein may be administered 1, 2, 3, 4, 5, 6, 7, 8, 9, 10, 11, 12, 13, 14, 15, 16, 17, 18, 19, 20, 21, 22, 23, or 24 times per week. In other embodiments, the compound is administered once biweekly.

In some embodiments, an agent or compound described herein may be administered orally. In some embodiments, an agent or compound described herein may be administered orally at a dosage of ≤15 mg/kg once per day.

The actual dosage employed may be varied depending upon the requirements of the patient and the severity of the condition being treated. Determination of the proper dosage regimen for a particular situation is within the skill of the art. For convenience, the total daily dosage may be divided and administered in portions during the day as required.

The dosage regimen utilizing the disclosed compound is selected in accordance with a variety of factors including type, species, age, weight, sex and medical condition of the patient; the severity of the condition to be treated; the route of administration: the renal or hepatic function of the patient; and the particular disclosed compound employed. A physician or veterinarian of ordinary skill in the art can readily determine and prescribe the effective amount of the drug required to prevent, counter or arrest the progress of the condition.

The amount and frequency of administration of the compounds of the invention and/or the pharmaceutically acceptable salts thereof will be regulated according to the judgment of the attending clinician considering such factors as age, condition and size of the patient as well as severity of the symptoms being treated.

## Examples

### Example 1

### TARGET IDENTIFICATION METHODS

Factors that upregulate HbF protein in the erythroid lineage were identified using a pooled CRISPR screening approach, as diagramed in FIG. 1. HUDEP2 cells, an erythroid progenitor model derived from CD34+ cells isolated from human umbilical cord blood, was used as a cellular model to study HbF reactivation, because the HBB/HBβ globin is the predominant β-like globin expressed.

A pool of CRISPR gRNAs was introduced into proliferating HUDEP2 cells via lentiviral delivery methods at an MOI ~0.1. Depending on the library construction, this was either a one-vector system (vector encoding both the gRNA and Cas9) or a two-vector system (vector encoding the gRNA). For the two-vector system, the lentiviral pool was delivered to HUDEP2 cells constitutively expressing Cas9 protein. One day following lentiviral transduction, the cells were grown in HUDEP2 proliferation media (StemSpan SFEM, StemCell Technologies: 50ng/ml SCF; 3 IU/ml erythropoietin; luM dexamethasone; lug/ml doxycycline) containing 500ng/ml puromycin to select for cells that received the CRISPR constructs. Selection in proliferation media + puromycin occurred for 2 days. The selected cells were then expanded for an additional 7 days in proliferation media and then shifted to HUDEP2 differentiation media (Iscove's Modified Dulbecco's Medium; 1% L-glutamine; 2% Penicillin/streptomycin; 330 ug/ml holo-human transferrin: 2 IU/ml heparin; 10ug/ml recombinant human insulin; 3 IU/ml erythropoietin: 100ng/ml SCF; 4% fetal calf serum) for 10 days.

An HbF fluorescence-activated cell sorting (FACs) assay (Invitrogen, HFH01) was used to isolate cells with elevated levels of HbF. HbF high cells were selected using HUDEP2 cells transduced with a negative control gRNA (sgGFP) as a gating threshold. Cells were also collected following the 3-day puromycin selection (post-selection sample) and prior to FACs sorting (FACs input sample) and used for downstream analyses to identify hits.

Genomic DNA was isolated from HbF high isolated cells, post-selection sample, and FACs input sample. The gRNA present at in the genomic DNA was amplified using nested PCR amplification. The second round of PCR amplification was performed to also incorporate Illumina sequencing adaptors onto the sample. Illumina sequencing was done to quantify the gRNAs present in each sample. The gRNAs were identified using conserved identifiers and were subsequently mapped to the human reference genome to identify the gRNA target gene to provide the relationship between the target gene and genetic perturbation that led to HbF upregulation.

The results of the screens are shown in FIG. 2 (CRISPR Library #1) and FIG. 3 (CRISPR Library #2). For each figure, the left panel plots the level of HbF (X-axis) and β-Actin (Y-axis) for each event, and the line "L" indicates the HbF threshold for HbF high cells. The right panel represents the same data in a one-dimensional plot showing the HbF levels (X-axis) and Events (Y-axis), and the line "C" indicates the HbF threshold for HbF high cells. Any cell above the HbF threshold was collected in the HbF high population. In both FIG. 2 and FIG. 3, the darker shaded cells at the left of each panel are HUDEP2 cells transduced with control sgGFP, and the lighter shaded cells at the right of each panel are HUDEP2 cells transduced with the CRISPR library.

Example 2

COMPUTATIONAL METHODS TO IDENTIFY GRNAs THAT UPREGULATE HBF

Illumina sequencing was used to sequence the libraries of gRNAs in the post-selection samples, FACs input samples, and HbF high samples. Each read was searched for the conserved identifiers either in the 5' or the 3' regions, and only reads that contained the conserved identifiers were retained. The 20bp gRNA sequence between the conserved identifiers was extracted from the retained reads and mapped to the human genome (hg19). A single retained read with a given gRNA represented one count for that gRNA in each sample. The counts were converted to RPM (reads per millions) to normalize for sequencing depth and to enable comparison across different gRNA libraries. The RPM for a gRNA was calculated as follows:

$$gRNA_{rpm} = \frac{gRNA_{count}}{N} * 1000000$$

In the above definition, N is the total number of reads in the library. Four different statistical methods were used to identify hits among the HbF high sample. The bioinformatics analysis performed using method 2 described below is summarized in FIG. 4A. FIG. 4B shows the distribution of guide abundance in different samples from two different screening libraries (Library #1 and Library #2), and FIG. 4C shows Z-score differences across samples for Library #1.

*Method 1: A Z score based approach in HbF high samples:*

In this approach, a Z score was calculated based on the distribution *of* $gRNA_{rpm}$ values in the HbF sample. More formally, the following formula was used to calculate the Z score

$$gRNA_{HbF+} = \frac{gRNA_{rpm,Hbf+} - \mu_{Hbf+}}{\sigma_{Hbf+}}$$

In the above equation $gRNA_{HbF+}$ is the Z score in HbF+ samples. $gRNA_{rpm,Hbf+}$ is the abundance. $\mu_{Hbf+}$, and $\sigma_{Hbf+}$ are the mean and standard-deviation of $gRNA_{rpm,Hbf+}$ in HbF+ samples. Similarly Z scores were calculated in the Input ($gRNA_{input}$) and post-selected ($gRNA_{post\text{-}selected}$) samples for all guides. gRNAs that led to a negative impact on cell health or proliferation were identified by performing a gRNA dropout analysis. More formally, all guides with $|gRNA_{input} - gRNA_{post\text{-}selected}| \geq 1$ were removed in this dropout analysis. All the remaining gRNAs with $gRNA_{HbF+} > 3$ were considered as enriched in HbF+ samples. Using this approach, a total of 174 hits were identified that contained at least one enriched gRNA.

*Method 2: A Z score difference based approach in HbF high and FACs Input:*

In this approach, the same dropout analysis (as performed in method 1) was performed. All gRNAs with $gRNA_{HbF+} - gRNA_{input} > 2.5$ were considered as enriched in HbF+ samples. Using this approach, a total of 307 hits were identified that contained at least one enriched gRNA. These are provided in Table 1.

Table 1: List of targets that upregulate HbF protein

| Gene Name | Uniprot ID | Description |
|---|---|---|
| CSNK1G2 | P78368 | casein kinase 1 gamma 2 |
| HIST1H2AA | Q96QV6 | histone cluster 1 H2A family member a |
| CDYL2 | Q8N8U2 | chromodomain Y like 2 |
| CAT | P04040 | catalase |
| KDMSA | P29375 | lysine demethylase 5A |
| PRKDC | P78527 | protein kinase, DNA-activated, catalytic polypeptide |
| SIM1 | P81133 | single-minded family bHLH transcription factor 1 |
| CCDC77 | Q9BR77 | coiled-coil domain containing 77 |
| SMYD1 | Q8NB12 | SET and MYND domain containing 1 |
| ASS1 | QST6L4 | argininosuccinate synthase 1 |
| CROT | Q9UKG9 | carnitine O-octanoyltransferase |
| CUL3 | Q13618 | cullin 3 |
| L3MBTL3 | Q96JM7 | L3MBTL3, histone methyl-lysine binding protein |
| GDNF | P39905 | glial cell derived neurotrophic factor |
| SAP130 | Q9H0E3 | Sin3A associated protein 130 |
| CDKN1C | P49918 | cyclin dependent kinase inhibitor 1C |
| ATPSFLC | P36542 | ATP synthase F1 subunit gamma |
| EID1 | Q9Y6B2 | EP300 interacting inhibitor of differentiation 1 |
| DNAJC1 | Q96KC8 | DnaJ heat shock protein family (Hsp40) member C1 |
| EXOSC1 | Q9Y3B2 | exosome component 1 |
| PGAM4 | Q8N0Y7 | phosphoglycerate mutase family member 4 |
| CHD1 | O14646 | chromodomain helicase DNA binding protein 1 |
| TSHZ3 | Q63HKS | teashirt zinc finger homeobox 3 |
| TADA3 | O75528 | transcriptional adaptor 3 |
| HIBADH | P31937 | 3-hydroxyisobutyrate dehydrogenase |
| WRB | O00258 | tryptophan rich basic protein |
| IKZF2 | Q9U KS7 | IKAROS family zinc finger 2 |
| TK2 | O00142 | thymidine kinase 2, mitochondrial |
| LDHB | Q5U077 | lactate dehydrogenase B |
| SIRT3 | Q9NTG7 | sirtuin 3 |
| HIST1H1T | P22492 | histone cluster 1 H1 family member t |
| ROCK2 | Q14DU5 | Rho associated coiled-coil containing protein kinase 2 |
| DIP2C | Q9Y2E4 | disco interacting protein 2 homolog C |
| NAP1L4 | Q99733 | nucleosome assembly protein 1 like 4 |
| PRKD3 | O94806 | protein kinase D3 |
| KDM3B | Q7LBC6 | lysine demethylase 38 |
| C22orf39 | Q6PSXS | chromosome 22 open reading frame 39 |
| ADCY8 | P40145 | adenylate cyclase 8 |
| HIRA | P54198 | histone cell cycle regulator |

(continued)

| Gene Name | Uniprot ID | Description |
|---|---|---|
| USP3 | Q9Y6I4 | ubiquitin specific peptidase 3 |
| MSL3 | Q8N5Y2 | MSL complex subunit 3 |
| HIST1H1B | P16401 | histone cluster 1 H1 family member b |
| HMG20B | Q9P0W2 | high mobility group 20B |
| BMX | P51813 | BMX non-receptor tyrosine kinase |
| KDM4E | B2RXH2 | lysine demethylase 4E |
| EEF2K | O00418 | eukaryotic elongation factor 2 kinase |
| PYGB | P11216 | glycogen phosphorylase B |
| MTA2 | O94776 | metastasis associated 1 family member 2 |
| SLC2A8 | Q9NY64 | solute carrier family 2 member 8 |
| NADK | O95544 | NAD kinase |
| PRMT1 | H7C2I1 | protein arginine methyltransferase 1 |
| HIST1H3D | P68431 | histone cluster 1 H3 family member d |
| PRKAR2B | P31323 | protein kinase cAMP-dependent type II regulatory subunit beta |
| ROS1 | P08922 | ROS proto-oncogene 1, receptor tyrosine kinase |
| ITPKC | Q96DU7 | inositol-trisphosphate 3-kinase C |
| AK1 | Q6FGX9 | adenylate kinase 1 |
| SSRP1 | Q08945 | structure specific recognition protein 1 |
| PADI4 | Q9UM07 | peptidyl arginine deiminase 4 |
| RB1 | Q92728 | RB transcriptional corepressor 1 |
| RRM2 | P31350 | ribonucleotide reductase regulatory subunit M2 |
| CDK10 | Q9UHL7 | cyclin dependent kinase 10 |
| G6PC3 | Q9BUM1 | glucose-6-phosphatase catalytic subunit 3 |
| GRK5 | P34947 | G protein-coupled receptor kinase 5 |
| BARD1 | Q99728 | BRCA1 associated RING domain 1 |
| MYLK2 | Q9H1R3 | myosin light chain kinase 2 |
| YWHAE | V9HW98 | tyrosine 3-monooxygenase/tryptophan 5-monooxygenase activation protein epsilon |
| GCDH | Q92947 | glutaryl-CoA dehydrogenase |
| TPI1 | V9HWK1 | triosephosphate isomerase 1 |
| PDK1 | Q15118 | pyruvate dehydrogenase kinase 1 |
| DCK | P27707 | deoxycytidine kinase |
| UBR2 | Q8IWV8 | ubiquitin protein ligase E3 component n-recognin 2 |
| IDH3G | P51553 | isocitrate dehydrogenase 3 (NAD(+)) gamma |
| SLC13A2 | Q13183 | solute carrier family 13 member 2 |
| TOP2A | P11388 | DNA topoisomerase II alpha |
| PDP1 | Q9P0J1 | pyruvate dehyrogenase phosphatase catalytic subunit 1 |
| PRPS1 | P60891 | phosphoribosyl pyrophosphate synthetase 1 |
| PHF7 | Q9BWX1 | PHD finger protein 7 |
| FBL | P22087 | fibrillarin |

(continued)

| Gene Name | Uniprot ID | Description |
|---|---|---|
| LDHAL6A | Q6ZMR3 | lactate dehydrogenase A like 6A |
| TEX14 | Q8IWB6 | testis expressed 14, intercellular bridge forming factor |
| PCCA | P05165 | propionyl-CoA carboxylase alpha subunit |
| PDK3 | Q15120 | pyruvate dehydrogenase kinase 3 |
| FADS1 | A0A0A0MR51 | fatty acid desaturase 1 |
| ATXN7L3 | Q14CW9 | ataxin 7 like 3 |
| RPS6KA4 | O75676 | ribosomal protein S6 kinase A4 |
| PC | P11498 | pyruvate carboxylase |
| GPX5 | V9HWN8 | glutathione peroxidase 5 |
| GPX6 | P59796 | glutathione peroxidase 6 |
| ARID4A | P29374 | AT-rich interaction domain 4A |
| USP16 | Q9YSTS | ubiquitin specific peptidase 16 |
| ITGB3 | Q16157 | integrin subunit beta 3 |
| RMI1 | Q9H9A7 | RecQ mediated genome instability 1 |
| SLC27A5 | Q9Y2P5 | solute carrier family 27 member 5 |
| PANK4 | Q9NVE7 | pantothenate kinase 4 |
| GALM | Q96C23 | galactose mutarotase |
| SRC | P12931 | SRC proto-oncogene, non-receptor tyrosine kinase |
| ADCY1 | Q08828 | adenylate cyclase 1 |
| RNF17 | Q9BXT8 | ring finger protein 17 |
| PFKFB4 | Q66S35 | 6-phosphofructo-2-kinase/fructose-2,6-biphosphatase 4 |
| COTL1 | Q14019 | coactosin like F-actin binding protein 1 |
| PHIP | Q8WWQ0 | pleckstrin homology domain interacting protein |
| BRWD1 | Q9NSI6 | bromodomain and WD repeat domain containing 1 |
| MBD3 | O95983 | methyl-CpG binding domain protein 3 |
| GCK | Q53Y25 | glucokinase |
| TYRO3 | Q06418 | TYRO3 protein tyrosine kinase |
| BCAT1 | P54687 | branched chain amino acid transaminase 1 |
| SMARCC1 | Q92922 | SWI/SNF related, matrix associated, actin dependent regulator of chromatin subfamily c member 1 |
| CBX4 | O00257 | chromobox 4 |
| ULK4 | Q96C45 | unc-51 like kinase 4 |
| GCLC | Q14TF0 | glutamate-cysteine ligase catalytic subunit |
| LYN | P07948 | LYN proto-oncogene, Src family tyrosine kinase |
| EZH2 | S4S3R8 | enhancer of zeste 2 polycomb repressive complex 2 subunit |
| FXR2 | P51116 | FMR1 autosomal homolog 2 |
| MGAM | O43451 | maltase-glucoamylase |
| CDKSR1 | Q15078 | cyclin dependent kinase 5 regulatory subunit 1 |
| PHF13 | Q86YI8 | PHD finger protein 13 |
| MAPK13 | O15264 | mitogen-activated protein kinase 13 |

(continued)

| Gene Name | Uniprot ID | Description |
|---|---|---|
| DGUOK | Q16854 | deoxyguanosine kinase |
| TNK1 | Q13470 | tyrosine kinase non receptor 1 |
| TET3 | O43151 | tet methylcytosine dioxygenase 3 |
| NAP1L2 | Q9ULW6 | nucleosome assembly protein 1 like 2 |
| SMARCB1 | Q12824 | SWI/SNF related, matrix associated, actin dependent regulator of chromatin, subfamily b, member 1 |
| L3MBTL1 | Q9Y468 | L3MBTL1, histone methyl-lysine binding protein |
| CAMK2G | Q8WU40 | calcium/calmodulin dependent protein kinase II gamma |
| SETD1A | O15047 | SET domain containing 1A |
| PHF3 | Q92576 | PHD finger protein 3 |
| CUL4B | Q13620 | cullin 4B |
| EPHAS | P54756 | EPH receptor A5 |
| BDH2 | Q9BUT1 | 3-hydroxybutyrate dehydrogenase 2 |
| FLT4 | P35916 | fms related tyrosine kinase 4 |
| CAMK2B | Q13554 | calcium/calmodulin dependent protein kinase II beta |
| PHF12 | Q96QT6 | PHD finger protein 12 |
| CCDC169 | A6NNP5 | coiled-coil domain containing 169 |
| AMT | P48728 | aminomethyltransferase |
| TRIB3 | Q96RU7 | tribbles pseudokinase 3 |
| AUH | Q13825 | AU RNA binding methylglutaconyl-CoA hydratase |
| NOC2L | Q9Y3T9 | NOC2 like nucleolar associated transcriptional repressor |
| UQCRC1 | P31930 | ubiquinol-cytochrome c reductase core protein 1 |
| STK36 | Q9NRP7 | serine/threonine kinase 36 |
| HDGF | P51858 | heparin binding growth factor |
| INSRR | P14616 | insulin receptor related receptor |
| MCAT | Q8IVS2 | malonyl-CoA-acyl carrier protein transacylase |
| AURKA | O14965 | aurora kinase A |
| USP46 | P62068 | ubiquitin specific peptidase 46 |
| FGFR1 | P11362 | fibroblast growth factor receptor 1 |
| RLIM | Q9NVW2 | ring finger protein, LIM domain interacting |
| MYBBP1A | Q9BQG0 | MYB binding protein 1a |
| MAPK4 | P31152 | mitogen-activated protein kinase 4 |
| RPS6KA3 | P51812 | ribosomal protein S6 kinase A3 |
| ULK2 | Q8IYT8 | unc-51 like autophagy activating kinase 2 |
| NPM2 | Q86SE8 | nucleophosmin/nucleoplasmin 2 |
| CDKN18 | Q6I9V6 | cyclin dependent kinase inhibitor 1B |
| EHHADH | Q08426 | enoyl-CoA hydratase and 3-hydroxyacyl CoA dehydrogenase |
| ADCK2 | Q7Z695 | aarF domain containing kinase 2 |
| PRMT2 | P55345 | protein arginine methyltransferase 2 |
| PRPF4B | Q13523 | pre-mRNA processing factor 4B |

(continued)

| Gene Name | Uniprot ID | Description |
|---|---|---|
| AMD1 | QSVXNS | adenosylmethionine decarboxylase 1 |
| ECI2 | O75521 | enoyl-CoA delta isomerase 2 |
| SBK1 | Q52WX2 | SH3 domain binding kinase 1 |
| MAP4K4 | O95819 | mitogen-activated protein kinase kinase kinase kinase 4 |
| HIF1AN | Q9NWT6 | hypoxia inducible factor 1 alpha subunit inhibitor |
| ALDOA | V9HWN7 | aldolase, fructose-bisphosphate A |
| INO80C | Q6PI98 | INO80 complex subunit C |
| SIRT7 | Q9NRC8 | sirtuin 7 |
| AIRE | O43918 | autoimmune regulator |
| SRSF3 | P84103 | serine and arginine rich splicing factor 3 |
| BDH1 | Q02338 | 3-hydroxybutyrate dehydrogenase 1 |
| SETD4 | Q9NVD3 | SET domain containing 4 |
| CDKN1A | P38936 | cyclin dependent kinase inhibitor 1A |
| TAF6L | Q9Y6J9 | TATA-box binding protein associated factor 6 like |
| ADCY9 | O60503 | adenylate cyclase 9 |
| PHF1 | O43189 | PHD finger protein 1 |
| BEX3 | Q00994 | brain expressed X-linked 3 |
| USP21 | Q9UK80 | ubiquitin specific peptidase 21 |
| SMYD2 | Q9NRG4 | SET and MYND domain containing 2 |
| G6PC | P35575 | glucose-6-phosphatase catalytic subunit |
| PHC2 | Q8IXK0 | polyhomeotic homolog 2 |
| FBXO43 | Q4G163 | F-box protein 43 |
| CDK8 | P49336 | cyclin dependent kinase 8 |
| HMGCS1 | Q01581 | 3-hydroxy-3-methylglutaryl-CoA synthase 1 |
| SPEN | Q96TS8 | spen family transcriptional repressor |
| ELP2 | Q6IA86 | elongator acetyltransferase complex subunit 2 |
| FFAR2 | O15552 | free fatty acid receptor 2 |
| RNF8 | O76064 | ring finger protein 8 |
| ZNF266 | Q14584 | zinc finger protein 266 |
| MST1 | G3XAK1 | macrophage stimulating 1 |
| PHF19 | QST6S3 | PHD finger protein 19 |
| IGF1R | P08069 | insulin like growth factor 1 receptor |
| MARK1 | Q9P0L2 | microtubule affinity regulating kinase 1 |
| FES | P07332 | FES proto-oncogene, tyrosine kinase |
| SMARCA1 | P28370 | SWI/SNF related, matrix associated, actin dependent regulator of chromatin, subfamily a, member 1 |
| ADCY7 | P51828 | adenylate cyclase 7 |
| PGLS | O95336 | 6-phosphogluconolactonase |
| SPOP | O43791 | speckle type BTB/POZ protein |
| ATF7IP | Q6VMQ6 | activating transcription factor 7 interacting protein |

(continued)

| Gene Name | Uniprot ID | Description |
|---|---|---|
| KDMSD | Q9BY66 | lysine demethylase 5D |
| TADA1 | Q96BN2 | transcriptional adaptor 1 |
| IKZF3 | Q9UKT9 | IKAROS family zinc finger 3 |
| IKZF1 | R9R4D9 | IKAROS family zinc finger 1 |
| MGST2 | Q99735 | microsomal glutathione S-transferase 2 |
| CALM1 | Q96HY3 | calmodulin 1 |
| TPK1 | Q9H3S4 | thiamin pyrophosphokinase 1 |
| MYO3A | Q8NEV4 | myosin IIIA |
| SIN3A | Q96ST3 | SIN3 transcription regulator family member A |
| AOX1 | Q06278 | aldehyde oxidase 1 |
| NME7 | Q9Y5B8 | NME/NM23 family member 7 |
| PARP1 | P09874 | poly(ADP-ribose) polymerase 1 |
| SCYL3 | Q8IZE3 | SCY1 like pseudokinase 3 |
| PASK | Q96RG2 | PAS domain containing serine/threonine kinase |
| MEAF6 | Q9HAF1 | MYST/Esa1 associated factor 6 |
| STK17A | Q9UEE5 | serine/threonine kinase 17a |
| ACADVL | P49748 | acyl-CoA dehydrogenase very long chain |
| PKN3 | Q6PSZ2 | protein kinase N3 |
| ACACB | O00763 | acetyl-CoA carboxylase beta |
| ZCWPW2 | Q504Y3 | zinc finger CW-type and PWWP domain containing 2 |
| FUK | Q8N0W3 | fucokinase |
| ADH5 | Q6IRT1 | alcohol dehydrogenase 5 (class III), chi polypeptide |
| CIR1 | Q86X95 | corepressor interacting with RBPJ, 1 |
| GOLGA5 | Q8TBA6 | golgin A5 |
| APOBEC3G | Q9HC16 | apolipoprotein B mRNA editing enzyme catalytic subunit 3G |
| PRDM11 | Q9NQV5 | PR/SET domain 11 |
| HLCS | P50747 | holocarboxylase synthetase |
| OBSCN | QSVST9 | obscurin, cytoskeletal calmodulin and titin-interacting RhoGEF |
| APOBEC3H | M4W6S4 | apolipoprotein B mRNA editing enzyme catalytic subunit 3H |
| ADH4 | P08319 | alcohol dehydrogenase 4 (class II), pi polypeptide |
| HIST3H3 | Q16695 | histone cluster 3 H3 |
| HMG20A | Q9NP66 | high mobility group 20A |
| FAM208A | Q9UK61 | family with sequence similarity 208 member A |
| SRP72 | V9HWK0 | signal recognition particle 72 |
| TAF5L | O75529 | TATA-box binding protein associated factor 5 like |
| MVK | Q03426 | mevalonate kinase |
| HIST4H4 | P62805 | histone cluster 4 H4 |
| SRPK2 | P78362 | SRSF protein kinase 2 |
| RPL27 | P61353 | ribosomal protein L27 |

(continued)

| Gene Name | Uniprot ID | Description |
|---|---|---|
| FLT3 | P36888 | fms related tyrosine kinase 3 |
| CS | O75390 | citrate synthase |
| GUCY2D | Q02846 | guanylate cyclase 2D, retinal |
| CPT18 | Q92523 | carnitine palmitoyltransferase 1B |
| EGFR | Q504U8 | epidermal growth factor receptor |
| MAST3 | O60307 | microtubule associated serine/threonine kinase 3 |
| MAGI2 | Q86UL8 | membrane associated guanylate kinase, WW and PDZ domain containing 2 |
| SLCSA1 | P13866 | solute carrier family 5 member 1 |
| IRAK4 | Q9NWZ3 | interleukin 1 receptor associated kinase 4 |
| NAP1L1 | P55209 | nucleosome assembly protein 1 like 1 |
| MAGI1 | Q96QZ7 | membrane associated guanylate kinase, WW and PDZ domain containing 1 |
| GAPDH | V9HVZ4 | glyceraldehyde-3-phosphate dehydrogenase |
| PRDM6 | Q9NQX0 | PR/SET domain 6 |
| PARP2 | Q9UGNS | poly(ADP-ribose) polymerase 2 |
| MYBL1 | P10243 | MYB proto-oncogene like 1 |
| NASP | QST626 | nuclear autoantigenic sperm protein |
| CTBP1 | X5D8Y5 | C-terminal binding protein 1 |
| NFYC | Q13952 | nuclear transcription factor Y subunit gamma |
| PIK3C2A | O00443 | phosphatidylinositol-4-phosphate 3-kinase catalytic subunit type 2 alpha |
| PRKAA2 | P54646 | protein kinase AMP-activated catalytic subunit alpha 2 |
| CUL4A | Q13619 | cullin 4A |
| SLC2AS | P22732 | solute carrier family 2 member 5 |
| TAF10 | Q12962 | TATA-box binding protein associated factor 10 |
| RRP8 | O43159 | ribosomal RNA processing 8 |
| DTYMK | Q6FGU2 | deoxythymidylate kinase |
| YWHAZ | P63104 | tyrosine 3-monooxygenase/tryptophan 5-monooxygenase activation protein zeta |
| SUCLG1 | P53597 | succinate-CoA ligase alpha subunit |
| KMT2C | Q8NEZ4 | lysine methyltransferase 2C |
| TTBK2 | Q8IWY7 | tau tubulin kinase 2 |
| SIRT2 | Q8IXJ6 | sirtuin 2 |
| DAB2IP | Q5VWQ8 | DAB2 interacting protein |
| CAMK1G | Q96NXS | calcium/calmodulin dependent protein kinase IG |
| PAK5 | Q9P286 | p21 (RAC1) activated kinase 5 |
| TXNDC12 | O95881 | thioredoxin domain containing 12 |
| TESK2 | Q96S53 | testis-specific kinase 2 |
| MAPK11 | Q15759 | mitogen-activated protein kinase 11 |
| MAG 13 | A0A024R0H3 | membrane associated guanylate kinase, WW and PDZ domain containing 3 |
| MAP2K5 | Q13163 | mitogen-activated protein kinase kinase 5 |
| BPGM | P07738 | bisphosphoglycerate mutase |

(continued)

| Gene Name | Uniprot ID | Description |
|---|---|---|
| PIK3CB | Q68DL0 | phosphatidylinositol-4,5-bisphosphate 3-kinase catalytic subunit beta |
| YEATS2 | Q9ULM3 | YEATS domain containing 2 |
| EXOSC9 | Q06265 | exosome component 9 |
| NEK1 | Q96PY6 | NIMA related kinase 1 |
| MYLK | Q15746 | myosin light chain kinase |
| CYP4A11 | Q02928 | cytochrome P450 family 4 subfamily A member 11 |
| AKT1 | P31749 | AKT serine/threonine kinase 1 |
| SETDB1 | Q15047 | SET domain bifurcated 1 |
| CDK17 | Q00537 | cyclin dependent kinase 17 |
| HLTF | Q14527 | helicase like transcription factor |
| IDH2 | P48735 | isocitrate dehydrogenase (NADP(+)) 2, mitochondrial |
| LRWD1 | Q9UFC0 | leucine rich repeats and WD repeat domain containing 1 |
| CPT2 | P23786 | carnitine palmitoyltransferase 2 |
| PRKACB | P22694 | protein kinase cAMP-activated catalytic subunit beta |
| ZNF687 | Q8N1G0 | zinc finger protein 687 |
| UBE2H | P62256 | ubiquitin conjugating enzyme E2 H |
| HMGN2 | P05204 | high mobility group nucleosomal binding domain 2 |
| ACAD10 | Q6JQN1 | acyl-CoA dehydrogenase family member 10 |
| TBK1 | Q9UHD2 | TANK binding kinase 1 |
| PRDM8 | Q9NQV8 | PR/SET domain 8 |
| ERBB3 | P21860 | erb-b2 receptor tyrosine kinase 3 |
| ARID1A | O14497 | AT-rich interaction domain 1A |
| DNMT1 | P26358 | DNA methyltransferase 1 |
| CAMK2D | Q13557 | calcium/calmodulin dependent protein kinase II delta |
| EPHB3 | P54753 | EPH receptor B3 |
| MBD4 | O95243 | methyl-CpG binding domain 4, DNA glycosylase |
| PRMT8 | Q9NR22 | protein arginine methyltransferase 8 |
| MTF2 | Q96G26 | metal response element binding transcription factor 2 |
| GLYR1 | Q49A26 | glyoxylate reductase 1 homolog |
| FRK | P42685 | fyn related Src family tyrosine kinase |
| ACAD8 | Q9UKU7 | acyl-CoA dehydrogenase family member 8 |
| RIMKLB | Q9ULI2 | ribosomal modification protein rimK like family member B |
| ACADS | P16219 | acyl-CoA dehydrogenase short chain |
| SMARCAL1 | Q9NZC9 | SWI/SNF related, matrix associated, actin dependent regulator of chromatin, subfamily a like 1 |

*Method 3: A fold-change based approach in HbF high and FACs Input:*

In this approach, the dropout and the hit calling was performed using fold-changes of RPM values. More formally, $\left|log\left(\frac{gRNA_{rpm,input}}{gRNA_{rpm,post-slected}}\right)\right| \geq 2$ was used as the criteria forgRNA dropout. After the dropout filtration, all the remaining gRNAs with $\left|log\left(\frac{gRNA_{rpm,Hbg+}}{gRNA_{rpm,input}}\right)\right| \geq 3$ were considered as enriched in HbF+ samples. Using this approach, a total of 314 hits were identified that contained at least one enriched gRNA.

*Number of gRNA hits per gene:*

In this approach, method 2 was used to identify enriched gRNAs. Genes with at least two enriched gRNAs were considered as hits. Using this approach 39 hits were identified. These are listed in FIG. 5A. A list of hits and associated gRNAs is summarized in Table 2.

Table 2: List of illustrative gRNAs for targets that upregulate HbF

| **Gene** | **Guide Sequence** | **Seq ID No.** |
|---|---|---|
| MTA2 | GCAAAGGAACGGCTACGACC | 5 |
| AK1 | TTGAAACGTGGAGAGACCAG | 6 |
| AK1 | GCTGTCGGAAATCATGGAGA | 7 |
| AKT1 | GCAGGATGTGGACCAACGTG | 8 |
| ARID4A | TGAGCCTGCCTACCTGACAG | 9 |
| UBE2H | CAGTCCGGGCAAGAGGCGGA | 10 |
| BEX3 | GACTTGCCCCTAATTTTCGA | 11 |
| COTL1 | TGCACTGCTGGATGAAGTGC | 12 |
| CROT | GGAGCGAACTCGATGGGCTA | 13 |
| CROT | ACTACTGGCCTCCAAAGGAA | 14 |
| DAB2IP | TGTGTGAGCTCAGGGAGCTG | 15 |
| ADH4 | GTTTGTGAAGGCTAAAGCCC | 16 |
| EEF2K | GGGGACAGCGACGATGAGGA | 17 |
| EEF2K | ATGGTGCGCTACCACGAGGG | 18 |
| FES | GGAGGGCATGAGAAAGTGGA | 19 |
| FXR2 | GGTTTAGTGCGTTCCAGGGG | 20 |
| CAMK1G | GCTGCATGACCAGGTAGTAG | 21 |
| GOLGA5 | GGAGAGCTATAAACAGATGC | 22 |
| GOLGA5 | TCTTTTGGGAGCCAAACCCA | 23 |
| GPX6 | TCTCAAAGAGCTGGAAACTG | 24 |
| SLC5A1 | GAGGAGGGAGATGACCACGA | 25 |
| IKZF1 | ATAAGGTCTCACCTGAAACT | 26 |
| IKZF1 | AGGCCCCGCACTGATTGCAC | 27 |
| RNF17 | AATAAGGCTCCAAAAGACCA | 28 |
| INO80C | GCAATGCCCTTTCAGAAGCG | 29 |
| KDM3B | GTAGACAGTAATGGGAGCGA | 30 |
| TET3 | GAGGCTGGGAACAACAGCAG | 31 |
| LYN | GTTTGGCCACATAGTTGCTG | 32 |
| MTA2 | GGTGCTGTGTCGGGATGAGA | 33 |
| MYLK | TCGCGATTTAGAAGTTGTGG | 34 |

(continued)

| Gene | Guide Sequence | Seq ID No. |
|---|---|---|
| MYLK | AATGAGCTCTGCTGTGCAGG | 35 |
| TAF6L | GAACCTGGCACCTCAAGGAT | 36 |
| PDK3 | TAAGAGCCCTGAGGATCCAT | 37 |
| PFKFB4 | GGGTTCTGTGTCAATTCCCG | 38 |
| UBE2H | GCCCGGACTGGGAGATGACA | 39 |
| SLC27A5 | GCCATACCTCCCCTACACCA | 40 |
| RNF17 | GCCTTGATGAAGCACTGCAG | 41 |
| RPS6KA3 | CCCGTGGCAGAAGATGGCTG | 42 |
| RPS8KA3 | ACATCTCTTGCAAACAGAGT | 43 |
| SIN3A | GGTGTGTGAGGCTGGACCGG | 44 |
| SLC27A5 | GGGGCTGCTGCTGACCAAGG | 45 |
| SLC27A5 | GCTCAGCACAGAGTGCGCCA | 46 |
| SLC5A1 | CACCATGGACATCTACGCCA | 47 |
| SMYD1 | TGAGCGGGCTTATTCCGCAG | 48 |
| SPOP | GTTTGTGCAAGGCAAAGACT | 49 |
| SPOP | TAACTTTAGCTTTTGCCGGG | 91 |
| SPOP | CGGGCATATAGGTTTGTGCA | 92 |
| SPOP | GTTTGCGAGTAAACCCCAAA | 93 |
| TADA1 | AGTGGGAAGCATCATTGTGT | 50 |
| TADA1 | ACTGGGCTAACCTAAAGCTG | 51 |
| TADA1 | GACCTTTGTGAGCGAGCTGG | 52 |
| TADA1 | AGATCGTACATGTTCACCGG | 53 |
| TAF6L | GGACACTGCCCACCAGACAG | 54 |
| TET3 | GGCACCTCTGAGCTGAGGAG | 55 |
| TOP2A | GAAGAGAGGGCCAGTTGTGA | 56 |
| UBE2H | GAGGCGGATGGACACGGACG | 57 |
| UBE2H | CAAATTCATTAAGTCCTCCC | 58 |
| ACAD10 | GAGGTCTTCGATCAGTGGGG | 59 |
| ACAD10 | GCTGGGAATCCCTGCTGCAG | 80 |
| ADH4 | CAAGCCCCTTTGCATTGAAG | 61 |
| CAMK1G | TGGCAGGGAGTGCTACACTG | 62 |
| AKT1 | GACAACCGCCATCCAGACTG | 63 |
| ARID4A | GAAAAGGCTGGTGAAAGTTA | 64 |
| BEX3 | GAAGACCGCCCTTTGGGAGG | 65 |
| C22orf39 | GAAGCCTTGCACAGAGCCTG | 66 |
| C22orf39 | GAGTCTTGAAGATATCAGGA | 87 |
| CAMK1G | GCGGGGTGTCTACACAGAGA | 68 |
| TOP2A | TAATCAGCAAGCCTTTGATG | 69 |
| COTL1 | ACTCCGCTCCCTGCTCGCCG | 70 |

(continued)

| Gene | Guide Sequence | Seq ID No. |
|---|---|---|
| DAB2IP | GGAGTTGATGATCTTGCAGA | 71 |
| FES | GCATTTGCTGCAGGACCCCG | 72 |
| FXR2 | ATAATGACAAGAAGAACCCC | 73 |
| GPX6 | CCTAAAGCCTCAAAATAGGA | 74 |
| HIRA | GAAGCCTTGCACAGAGCCTG | 75 |
| HIRA | GAGTCTTGAAGATATCAGGA | 76 |
| INO80C | TTAGCTGGCTTAAAGGATGG | 77 |
| KDM3B | GGAATGCCAGTGGAGAGCCA | 78 |
| LYN | TGAAAGACAAGTCGTCCGGG | 79 |
| SPOP | GTAGCACCAACTCTCAGCTA | 80 |
| MTA2 | GGCCCTAGAGAAGTATGGGA | 81 |
| NPM2 | GGAGGACAAGAAGATGCAGC | 82 |
| NPM2 | GGGAAATGCGCACCATGGGG | 83 |
| PDK3 | TAAGAGCCCTGAGGATCCAC | 84 |
| PFKFB4 | GAGCTACGTGGTGAACCGTG | 85 |
| RPS6KA3 | GGATGAACCTATGGGAGAGG | 86 |
| SIN3A | GCAGATGCCAGCAAACATGG | 87 |
| SMYD1 | AGGAGGAGCAGAAGGACCTG | 88 |
| TPK1 | GGCACTTAGTAAAGTCAGTG | 89 |
| TPK1 | AAGGCTGTCCAACAGGAATA | 90 |
| CUL3 | GAGCATCTCAAACACAACGA | 94 |
| CUL3 | CGAGATCAAGTTGTACGTTA | 95 |
| CUL3 | TCATCTACGGCAAACTCTAT | 96 |

Example 3

BIOINFORMATIC ANALYSIS OF TARGET GENE HITS THAT UPREGULATE HBF

Multiple bioinformatic analyses were used to identify specific pathways, complexes and tissue specific expression patterns that were enriched in the top targets that significantly upregulate HbF protein levels.

*Protein complex analysis:*

To identify protein complexes with multiple targets that upregulate HbF, top targets identified by the methods described above were overlapped with existing protein complex annotations (CORUM protein complex annotations (Giurgiu M et al, Nucleic Acids Research)). This analysis identified several complexes with multiple targets. These complexes and the number of targets identified as components of each complex are provided in FIG. 6. The overlap of complex annotations and targets identified using methods 2 and 3 are displayed in Table 3 and Table 4.

Table 3: Protein complexes with multiple subunits identified as targets (method 2) that upregulate HbF

| **Complex Name** | **hits_in_complex** |
|---|---|
| STAGA_complex_SPT3-linked | TADA3;TAF6L;TADA1;TAF5L;ATXN7L3;TAF10 ;SAP130 |
| STAGA_complex | TADA3;TAF6L;TADA1 ;TAF5L;TAF10 |
| SAGA_complex_GCN5-linked | TADA3;TAF6L;TAF5L;ATXN7L3;TAF10 |
| LARC complex_(LCR-associated_remodeling_complex) | MBD3;SMARCB1 ;SMARCC1 ;ARID1A;MTA2 |
| ALL-1_supercomplex | SIN3A;MBD3;SMARCB1;SMARCC1;MTA2 |
| TFTC_complex_(TATA-binding_protein-free_TAF-II-containing_complex) | TADA3;TAF6L;TAF5L:TAF10 |
| SIN3-ING1b_complex_II | SIN3A;SMARCB1;SMARCC1;ARID1A |
| PCAF _complex | TADA3;TAF6L;TAF5L;TAF1 0 |
| Nop56p-associated_pre-rRNA_complex | MYBBP1A;FBL;NAP1L1 ;RPL27 |
| BRM-SIN3A_complex | SIN3A;SMARCB1;SMARCC1;ARID1A |
| BRM-SIN3A-HDAC_complex | SIN3A;SMARCB1;SMARCC1;ARID1A |
| BRG1-SIN3A_complex | SIN3A;SMARCB1;SMARCC1;ARID1A |
| p300-CBP-p270-SWI/SNF_complex | SMARCB1;SMARCC1;ARID1A |
| WINAC_complex | SMARCB1;SMARCC1;ARID1A |
| USP22-SAGA_complex | TADA3;ATXN7L3;TAF10 |
| Spliceosome | SPEN;SRSF3;PRPF4B |
| SWI-SNF_chromatin_remodeling-related-BRCA1_complex | SMARCB1;SMARCC1:ARID1A |
| RNA_polymerase_II_complex,_ incomplete_(CDK8_complex),_chromati n_structure_modifying | CDK8;SMARCB1 ;SMARCC1 |
| RNA_polymerase_II_complex,_ chromatin_structure_modifying | CDK8;SMARCB1 ;SMARCC1 |
| NUMAC_complex_(nucleosom al_methylation_activator_complex) | SMARCB1;SMARCC1;ARID1A |
| MTA2_complex | SIN3A;MBD3;MTA2 |
| LSD1_complex | HMG20B;HMG20A;CTBP1 |
| Kinase_maturation_complex_1 | MAP2K5;YWHAE;YWHAZ |
| ING2_complex | SIN3A;ARID4A;SAP130 |
| GCN5-TRRAP_histone_acetyltransferase_com plex | TADA3;TAF5L;TAF10 |
| EBAFa_complex | SMARCB1;SMARCC1;ARID1A |

(continued)

| Complex Name | hits_in_complex |
|---|---|
| CEN_complex | FBL;SSRP1;CUL4A |
| BAF_complex | SMARCB1;SMARCC1;ARID1A |
| Anti-HDAC2_complex | HMG20B;SIN3A;MTA2 |
| ZNF304-corepressor_complex | DNMT1;SETDB1 |
| Ubiquitin_E3_ligase_(SPOP,_D AXX,_CUL3) | SPOP;CUL3 |
| Ubiquitin_E3_ligase_(H2AFY,_ SPOP,_CUL3) | SPOP;CUL3 |
| Ubiquitin_E3_ligase_(DDB1,_D DB2, CUL4A, CUL4B,_RBX1) | CUL4B;CUL4A |
| Ubiquitin_E3_ligase_(BMI1,_S POP,_CUL3) | SPOP;CUL3 |
| Toposome | SSRP1;TOP2A |
| SNF2h-cohesin-NuRD complex | MBD3;MTA2 |
| SIN3-SAP25_complex | SIN3A;SAP130 |
| SHARP-CtBP_complex | CTBP1;SPEN |
| SHARP-CtBP1-CtIP_complex | CTBP1;SPEN |
| SHARP-CtBP1-CtIP-RBP-Jkappa corepressor complex | CTBP1;SPEN |
| SETDB1-containing HMTase complex | ATF7IP;SETDB1 |
| Polycomb_repressive_complex 1_(PRC1,_hPRC-H) | PHC2;CBX4 |
| PBAF _complex_(Polybromo- and BAF containing complex) | SMARCB1;SMARCC1 |
| NCOR1_complex | SMARCB1;SMARCC1 |
| NCOA6-DNA-PK-Ku-PARP1_complex | PARP1;PRKDC |
| Mi2/NuRD_complex | MBD3;MTA2 |
| Mi-2/NuRD-MTA2_complex | MBD3;MTA2 |
| MeCP1_complex | MBD3;MTA2 |
| MLL1-WDRS_complex | INO80C;MGAM |
| MBD1-MCAF1-SETDB1_complex | ATF7IP;SETDB1 |
| ITGAV-ITGB3-EGFR_complex | EGFR;ITGB3 |
| ITGA2b-ITGB3-CD47-SRC complex | ITGB3;SRC |

(continued)

| Complex Name | hits_in_complex |
|---|---|
| Histone_H3.3_complex | NASP;HIRA |
| HDAC2-asscociated_core_complex | MBD3;MTA2 |
| HDAC1-associated protein complex | MBD3;MTA2 |
| HDAC1-associated_core_complex_cII | MBD3;MTA2 |
| HCF-1_complex | SIN3A;SETD1A |
| FIB-associated protein complex | FBL;PRMT1 |
| Exosome | EXOSC1;EXOSC9 |
| Emerin_complex_52 | HDGF;YWHAE |
| Emerin_complex_32 | SMARCB1;SMARCC1 |
| Emerin_complex_25 | YWHAE;SAP130 |
| Emerin_complex_24 | RB1;SAP130 |
| EGFR-containing_signaling_complex | EGFR;PIK3C2A |
| EBAFb_complex | SMARCB1;SMARCC 1 |
| CtBP _complex | CTBP1;CBX4 |
| CDC5L_complex | PRKDC;TOP2A |
| ATAC_complex,_YEATS2-linked | TADA3;YEATS2 |
| ATAC_complex,_GCN5-linked | TADA3;YEATS2 |
| ARC_complex | CDK8;ACAD8 |
| pRb2/p130-multimolecular_complex_(DNMT1,_E2F 4,_SuV39H1,_HDAC1,_RBL2) | DNMT1 |
| p32-CBF-DNA_complex | NFYC |
| p300-CBP-p270_complex | ARID1A |
| p27-cyclinE-Cdk2_-_Ubiquitin_E3_ligase_(SKP1A,_SKP2,_ CUL1, CKS1B,_RBX1) complex | CDKN1B |
| p27-cyclinE-CDK2_complex | CDKN1B |
| p21 (ras)GAP-Fyn-Lyn-Yes_complex_thrombin_stimulated | LYN |
| p130Cas-ER-alpha-cSrc-kinase- PI3-kinase_p85-subunit_complex | SRC |
| hNURF_complex | SMARCA1 |

(continued)

| Complex Name | hits_in_complex |
|---|---|
| eNOS-HSP90-AKT_complex,_VEGF_induced | AKT1 |
| c-Abl-cortactin-nmMLCK_complex | MYLK |
| anti-BHC110_complex | HMG20B |
| WRN-Ku70-Ku80-PARP1 complex | PARP1 |
| WDR20-USP46-UAF1_complex | USP46 |
| Vigilin-DNA-PK-Ku_antigen_complex | PRKDC |
| VEcad-VEGFR_complex | FLT4 |
| Ubiquitin_E3_ligase_(DET1,_D DB1, CUL4A, RBX1,_COP1) | CUL4A |
| Ubiquitin_E3_ligase_(DDIT4,_D DB1, BTRC,_CUL4A) | CUL4A |
| Ubiquitin_E3_ligase_(DDB1,_C UL4A,_RBX1) | CUL4A |
| Ubiquitin_E3_ligase_(CUL3,_K LHL3,_ WNK4) | CUL3 |
| Ubiquitin_E3_ligase_(CUL3,_K LHL3,_WNK1) | CUL3 |
| Ubiquitin_E3_ligase_(CUL3,_K LHL3) | CUL3 |
| Ubiquitin_E3_ligase_(CSN1,_C SN8_HRT1 ,_SKP1,_SKP2,_CUL1 ,_C UL2,_CUL3) | CUL3 |
| Ubiquitin_E3_ligase_(CHEK1,_ CUL4A) | CUL4A |
| Ubiquitin E3 ligase_(CDT1 _D DB1, CUL4A, RBX1) | CUL4A |
| Ubiquitin_E3_ligase_(AHR,_AR NT, DDB1,_TBL3,_CUL4B,_RBX1) | CUL4B |
| UTX-MLL2/3_complex | KMT2C |
| USP46-UAF1_complex | USP46 |
| ULK2-ATG13-RB1CC1. complex | ULK2 |
| Tacc1-chTOG-AuroraA_complex | AURKA |
| TRIM27-RB1_complex | RB1 |
| TRIB3-DDIT3_complex | TRIB3 |
| TRBP_containing_complex_(DI CER,_RPL7A,_EIF6,_MOV10_and_sub units_of_the_60S_ribosomal_particle) | RPL27 |
| TNF-alpha/NF-kappa_B_signaling_complex_6 | FBL |
| TNF-alpha/NF-kappa_B_signaling_complex_10 | TBK1 |

(continued)

| Complex Name | hits_in_complex |
|---|---|
| TIP5-DNMT-HDAC1_complex | DNMT1 |
| TFIID_complex_B-cell_specific | TAF10 |
| TFIID_complex | TAF10 |
| TFIID-beta_complex | TAF10 |
| TCL1 (trimer)-AKT1_complex | AKT1 |
| Succinyl-CoA_synthetase,_GDP-forming | SUCLG1 |
| Succinyl-CoA synthetase. ADP-forming | SUCLG1 |
| Set1A_complex | SETD1A |
| SWI/SNF_chromatin-remodeling_complex | SIN3A |
| SNX_complex_(SNX1a,_SNX2, SNX4,_EGFR) | EGFR |
| SNF2L-RSF1_complex | SMARCA1 |
| SMCC_complex | CDK8 |
| SMAR1-HDAC1-SIN3A-SIN3B repressor complex | SIN3A |
| SMAR1-HDAC1-SIN3A-SIN3B-p107-p130_repressor_complex | SIN3A |
| SMAD3-cSKI-SIN3A-HDAC1_complex | SIN3A |
| SKI-NCOR1-SIN3A-HDAC1 complex | SIN3A |
| SIN3_complex | SIN3A |
| SIN3-ING1 b_complex_I | SIN3A |
| SHARP-CtIP-RBP-Jkappa, complex | SPEN |
| SH3KBP1-CBLB-EGFR complex | EGFR |
| SETDB1-DNMT3B_complex | SETDB1 |
| SETDB1 -DNMT3A_ complex | SETDB1 |
| SERCA2a-alphaKAP-CaM-CaMKII_complex | CALM1 |
| Ribosome,_cytoplasmic | RPL27 |
| Replication-coupled_CAF-1-MBD1-ETDB1_complex | SETDB1 |
| Rb-tal-1-E2A-Lmo2-Ldb1_complex | RB1 |

(continued)

| Complex Name | hits_in_complex |
|---|---|
| Rb-HDAC1_complex | RB1 |
| RasGAP-AURKA-survivin complex | AURKA |
| Rap1_complex | PARP1 |
| RSmad_complex | SMARCC1 |
| RIN1-STAM2-EGFR_complex,_EGF_stimulated | EGFR |
| REST-CoREST-mSIN3A_complex | SIN3A |
| RC_complex_during_S-phase_of_cell_cycle | PARP1 |
| RC_complex_during_G2/M-phase_of_cell_cycle | PARP1 |
| RBP-Jkappa-SHARP _complex | SPEN |
| RB1-TFAP2A_complex | RB1 |
| RB1-HDAC1-BRG1_complex | RB1 |
| RB1 (hypophosphorylated)-E2F4 complex | RB1 |
| RB-E2F1_complex | RB1 |
| RAF1-MAP2K1-YWHAE_complex | YWHAE |
| Polycystin-1_multiprotein_complex_(ACTN1,_CDH 1,_SRC,_JUP,_VCL,_CTNNB1,_PXN,_ BCAR1, PKD1,_PTK2. TLN1) | SRC |
| Polycomb_repressive_complex _4_(PRC4) | EZH2 |
| Polycomb_repressive_complex 2_(PRC2) | EZH2 |
| Polycomb_repressive_complex | CBX4 |
| Phosphorylase_kinase_comple x | CALM1 |
| PU.1-SIN3A-HDAC_complex | SIN3A |
| PTIP-HMT_complex | KMT2C |
| PTEN-NHERF1-EGFR_complex | EGFR |
| PRMT2_homo-oligomer complex | PRMT2 |
| PRMT1_complex | PRMT1 |
| PLC-gamma-2-SLP-76-Lyn-Grb2_complex | LYN |
| PLC-gamma-2-Lyn-FcR-gamma complex | LYN |

(continued)

| Complex Name | hits_in_complex |
|---|---|
| PKA_(RII-alpha and RII-beta)-AKAP5-ADRB1 complex | PRKAR2B |
| PCNA_complex | CDKN1A |
| PCNA-p21_complex | CDKN1A |
| P53-BARD1-Ku70_complex | BARD1 |
| NuRD.1_complex | MBD3 |
| NuA4/Tip60_HAT_complex | MEAF6 |
| NuA4/Tip60-HAT_complex_A | MEAF6 |
| NRP2-VEGFR3_complex | FLT4 |
| NK-3-Groucho-HIPK2-SIN3A-RbpA48-HDAC1 complex | SIN3A |
| NCOR2_complex | SIN3A |
| NCOR-SIN3-RPD3_complex | SIN3A |
| NCOR-SIN3-HDAC1_complex | SIN3A |
| NCOR-SIN3-HDAC-HESX1 complex | SIN3A |
| NAT_complex | CDK8 |
| Mi2/NuRD-BCL6-MTA3, complex | MBD3 |
| Mediator_complex | CDK8 |
| MeCP2-SIN3A-HDAC_complex | SIN3A |
| MTA1_complex | MBD3 |
| MSL_complex | MSL3 |
| MRG15-PAM14-RB_complex | RB1 |
| MLL3_complex | KMT2C |
| MGC1-DNA-PKcs-Ku_complex | PRKDC |
| MBD1-MCAF_complex | ATF7IP |
| MAP2K1-BRAF-RAF1-YWHAE-KSR1_complex | YWHAE |
| MAD1-mSin3A-HDAC2 complex | SIN3A |
| Kinase_maturation_complex_2 | TBK1 |

(continued)

| Complex Name | hits_in_complex |
|---|---|
| ITGB3-ITGAV-VTN_complex | ITGB3 |
| ITGB3-ITGAV-CD47_complex | ITGB3 |
| ITGAV-ITGB3_complex | ITGB3 |
| ITGAV-ITGB3-THBS1_complex | ITGB3 |
| ITGAV-ITGB3-SPP1_complex | ITGB3 |
| ITGAV-ITGB3-SLC3A2 complex | ITGB3 |
| ITGAV-ITGB3-PXN-PTK2b, complex | ITGB3 |
| ITGAV-ITGB3-PPAP2b_complex | ITGB3 |
| ITGAV-ITGB3-NOV_complex | ITGB3 |
| ITGAV-ITGB3-LAMA4_complex | ITGB3 |
| ITGAV-ITGB3-COL4A3 complex | ITGB3 |
| ITGAV-ITGB3-CD47-FCER2 complex | ITGB3 |
| ITGAV-ITGB3-ADAM23_complex | ITGB3 |
| ITGAV-ITGB3-ADAM15_complex | ITGB3 |
| ITGA5-ITGB3-COL6A3 complex | ITGB3 |
| ITGA2b-ITGB3-TLN1_complex | ITGB3 |
| ITGA2b-ITGB3-CD9_complex | ITGB3 |
| ITGA2b-ITGB3-CD9-GP1b-CD47 complex | ITGB3 |
| ITGA2b-ITGB3-CD4 7-FAK_complex | ITGB3 |
| ITGA2B-ITGB3_complex | ITGB3 |
| ITGA2B-ITGB3-ICAM4_complex | ITGB3 |
| ITGA2B-ITGB3-FN1-TGM2_complex | ITGB3 |
| ITGA2B-ITGB3-F11R_complex | ITGB3 |
| ITGA2B-ITGB3-CIB1_complex | ITGB3 |
| ITAGV-ITGB3-F11R_complex | ITGB3 |
| INO80_chromatin_remodeling_ complex | INO80C |

(continued)

| Complex Name | hits_in_complex |
|---|---|
| ING5_complex | MEAF6 |
| ING4_complex_(ING4,_MYST2 ,_C1orf149,_PHF17) | MEAF6 |
| ING4_complex_(ING4,_MYST2 ,_C1orf149,_PHF16) | MEAF6 |
| ING4_complex_(ING4,_MYST2 ,_C1orf149,_PHF15) | MEAF6 |
| IGF1R-CXCR4-GNAI2-GNB1 complex | IGF1R |
| Histone_H3.1_complex | NASP |
| HUIC_complex | BARD1 |
| HSP90-CIP1-FKBPL_complex | CDKN1A |
| HMGB1-HMGB2-HSC70-ERP60-GAPDH complex | GAPDH |
| HES1_promoter-Notch enhancer complex | CDK8 |
| HERP1/HEY2-NCOR-SIN3A_complex | SIN3A |
| HBO1_complex | MEAF6 |
| H2AX_complex_I | PARP1 |
| H2AX_complex,_isolated_from cells_without_IR_exposure | SSRP1 |
| G_alpha-13-Hax-1-cortactin-Rac_complex | AKT1 |
| GAIT_complex | GAPDH |
| FGFR2-c-Cbl-Lyn-Fyn_complex | LYN |
| FGFR1c-KL_complex | FGFR1 |
| FGF23-FGFR1c-KL_complex | FGFR1 |
| FGF21-FGFR1c-KLB_complex | FGFR1 |
| FE65-TSHZ3-HDAC1_complex | TSHZ3 |
| FA_complex_(Fanconi_anemia _complex) | RMI1 |
| **FACT complex _UV-activated** | SSRP1 |
| FACT_complex | SSRP1 |
| FACT-NEK9_complex | SSRP1 |
| F1F0-ATP_synthase,_mitochondrial | ATP5F1C |

(continued)

| **Complex Name** | **hits_in_complex** |
|---|---|
| Elongator_holo_complex | ELP2 |
| Ecsit_complex_(ECSIT,_MT-CO2,_GAPDH,_TRAF6,_NDUFAF1) | GAPDH |
| ETS2-SMARCA4-INI1_complex | SMARCB1 |
| ERBB3-SPG1_complex | ERBB3 |
| EGFR-CBL-GRB2_complex | EGFR |
| EED-EZH_polycomb_complex | EZH2 |
| EED-EZH2_complex | EZH2 |
| EED-EZH-YY1_polycomb_complex | EZH2 |
| DRD4-KLHL12-CUL3_complex | CUL3 |
| DNMT3B_complex | SIN3A |
| DNMT1-G9a_complex | DNMT1 |
| DNMT1-G9a-PCNA_complex | DNMT1 |
| DNA_synthesome_complex_(1 7_subunits) | TOP2A |
| DNA-PK-Ku_complex | PRKDC |
| DNA-PK-Ku-eIF2-NF90-NF45_complex | PRKDC |
| DHX9-ADAR-vigilin-DNA-PK-Ku, antigen, complex | PRKDC |
| DDN-MAGI2-SH3KBP1_complex | MAGI2 |
| DDB2_complex | CUL4A |
| DA_complex | TAF10 |
| DAB_complex | TAF10 |
| CyclinD3-CDK4-CDK6-p21_complex | CDKN1A |
| Condensin_I-PARP-1-XRCC1_complex | PARP1 |
| CoREST-HDAC_complex | HMG20B |
| Cell_cycle_kinase_complex_C DK5 | CDKN1A |
| Cell_cycle_kinase_complex_C DK4 | CDKN1A |
| Cell_cycle_kinase_complex_C DK2 | CDKN1A |

(continued)

| Complex Name | hits_in_complex |
|---|---|
| Cell_cycle_kinase_complex_C DC2 | CDKN1A |
| C_complex_spliceosome | PRPF4B |
| CUL4B-DDB1-WDR26_complex | CUL4B |
| CUL4B-DDB1-TLE3_complex | CUL4B |
| CUL4B-DDB1-TLE2complex | CUL4B |
| CUL4B-DDB1-TLE1_complex | CUL4A |
| CUL4B-DDB1-GRWD1_complex | CUL4B |
| CUL4B-DDB1-DTL-CSN_complex | CUL4B |
| CUL4A-DDB1-WDR61_complex | CUL4A |
| CUL4A-DDB1-WDR5_complex | CUL4A |
| CUL4A-DDB1-WDR5B complex | CUL4A |
| CUL4A-DDB1-WDR57_complex | CUL4A |
| CUL4A-DDB1-RBBP5_complex | CUL4A |
| CUL4A-DDB1-EED_complex | CUL4A |
| CUL4A-DDB1-DTL_complex | CUL4A |
| CSA_complex | CUL4A |
| CSA-POLIIa_complex | CUL4A |
| CS-MAP3K7IP1-MAP3K7IP2_complex | CS |
| CNK1-SRC-RAF1_complex | SRC |
| CHTOP-methylosome_complex | PRMT1 |
| CERF_complex_(CECR2-containing_remodeling_factor_complex | SMARCA1 |
| CEP164-TTBk2_complex | TTBK2 |
| CEBPE-E2F1-RB1_complex | RB1 |
| CDK8-CyclinC-Mediator_complex | CDK8 |
| CD20-LCK-LYN-FYN-p75/80_complex,_(Raji_human_B_cell_ line) | LYN |
| CCDC22-COMMD8-CUL3_complex | CUL3 |

(continued)

| Complex Name | hits_in_complex |
|---|---|
| CBF-DNA_complex | NFYC |
| CAS-SRC-FAK_complex | SRC |
| CAND1-CUL4B-RBX1_complex | CUL4B |
| CAND1-CUL4A-RBX1_complex | CUL4A |
| CAND1-CUL3-RBX1_complex | CUL3 |
| CALM1-KCNQ4(splice_variant_2)_complex | CALM1 |
| CALM1-KCNQ4(splice_variant_1)_complex | CALM1 |
| BRMS1-SIN3-HDACcomplex | SIN3A |
| BRCA1_C_complex | BARD1 |
| BRCA1_B_complex | BARD1 |
| BRCA1_A_complex | BARD1 |
| BRCA1-CtIP-CtBP_complex | CTBP1 |
| BRCA1-BARD1-UbcH7c complex | BARD1 |
| BRCA1-BARD1-UbcH5c_complex | BARD1 |
| BRCA1-BARD1-POLR2A_complex | BARD1 |
| BRCA1-BARD1-BRCA2-DNA_damage_complex_III | BARD1 |
| BRCA1-BARD1-BACH1-DNA_damage_complex II | BARD1 |
| BRCA1-BARD1-BACH1-DNA_damage_complex I | BARD1 |
| BRAFT_complex | RMI1 |
| BRAF53-BRCA2_complex | HMG20B |
| BRAF-RAF1 -14-3-3_ complex | YWHAZ |
| BRAF-MAP2K1-MAP2K2-YWHAE_complex | YWHAE |
| BMI1-HPH1-HPH2_complex | PHC2 |
| BLM_complex_III | RMI1 |
| BLM_complex_II | RMI1 |
| BHC_complex | HMG20B |

(continued)

| Complex Name | hits_in_complex |
|---|---|
| BARD1-BRCA1-CSTF complex | BARD1 |
| BARD1-BRCA1-CSTF64 complex | BARD1 |
| B-WICH_complex | MYBBP1A |
| Artemis-DNA-PK_complex | PRKDC |
| Akt-PHLPP1-PHLPP2-FANCI-FANCD2-USP1-UAF1_complex | AKT1 |
| AURKA-INPP5E_complex | AURKA |
| AURKA-HDAC6_cilia-disassembly_complex | AURKA |
| ASF1-interacting_protein_complex | HIRA |
| ASF1-histone_containing_complex | NASP |
| ASCOM_complex | KMT2C |
| ARC92-Mediator_complex | CDK8 |
| ARC-L_complex | CDK8 |
| AR-AKT-APPL_complex | AKT1 |
| AMY-1-S-AKAP84-RII-beta_complex | PRKAR2B |
| 60S_ribosomal_subunit_cytopl asmic | RPL27 |
| 17S_U2_snRNP | HMG20B |

Table 4: Protein complexes with multiple subunits identified as targets (method 3) that upregulate HbF

| Complex Name | hits_in_complex |
|---|---|
| STAGA_complex,_SPT3-linked | TAF6L;TADA1;KAT2A;ATXN7L3;SAP130;TRRAP |
| NuA4/Tip60_HAT_complex | KAT5;BRD8;MEAF6;EPC1;YEATS4;TRRAP |
| NuA4/Tip60-HAT_complex_A | KAT5;BRD8;MEAF6;EPC1;YEATS4;TRRAP |
| WINAC_complex | SUPT16H;SMARCB1;SMARCD1;ARID1A;BAZ1B |
| UTX-MLL2/3_complex | N4BP2;KMT2C;RBBPS;KMT2D;ASH2L |
| Spliceosome | CDK12;PPM1G;SRSF1;SRSF3;PRPF4B |
| Nop56p-associated_pre-rRNA complex | MYBBP1A;FBL;NAP1L1;RPL27;H1FX |
| LARC_complex_(LCR-associated_remodeling_complex) | MBD3;GATAD2B;SMARCB1;ARID1A;MTA2 |
| BRM-SIN3A_complex | SIN3A;SMARCB1;SMARCD1;SMARCD3;ARID1A |
| BRG1-SIN3A_complex | SIN3A;SMARCB1;SMARCD1;SMARCD3;ARID1A |
| ALL-1_supercomplex | SIN3A;MBD3;RBBP5;SMARCB1;MTA2 |
| STAGA_complex | TAF6L;TADA1; KAT2A;TRRAP |
| SIN3-ING1b_compex_II | SIN3A;SMARCB1;SMARCD1;ARID1A |
| SAGA_complex,_GCN5-linked | TAF6L;KAT2A;ATXN7L3;TRRAP |
| MLL1-WDR5_complex | INO80C;E2F6;RBBP5;ASH2L |
| BRM-SIN3A-HDAC_complex | SIN3A;SMARCB1;SMARCD1;ARID1A |
| ASCOM_complex | KMT2C;RBBP5;KMT2D;ASH2L |
| p300-CBP-p270-SWI/SNF_complex | CREBBP;SMARCB1;ARID1A |
| USP22-SAGA_complex | KAT2A;ATXN7L3;TRRAP |
| TFTC_complex_(TATA-binding_protein-free_TAF-II-containing_complex) | TAF6L;KAT2A;TRRAP |
| Set1B_complex | CXXC1;RBBP5;ASH2L |
| Set1A_complex | CXXC1;RBBP5;ASH2L |
| SNF2h-cohesin-NuRD_complex | BAZ1A;MBD3;MTA2 |
| RNA_polymerase_II_complex,_chromat in_structure_modifying | CREBBP;SMARCB1;SMARCD1 |
| PTIP-HMT_complex | KMT2C;RBBP5;ASH2L |
| PBAF_complex_(Polybromo-_and_BAF_containing_complex) | PBRM1;SMARCB1;SMARCD1 |
| NuA4/Tip60-HAT_complex_B | KAT5;EPC1;TRRAP |
| NUMAC_complex_(nucleosomal_methy lation_activator _complex) | SMARCB1;SMARCD1;ARID1A |
| MeCP1_complex | MBD3;GATAD2B;MTA2 |
| MTA2_complex | SIN3A;MBD3;MTA2 |
| MLL4_complex | RBBP5;KMT2D;ASH2L |
| MLL3_complex | KMT2C;RBBP5;ASH2L |
| MLL2_complex | RBBP5;KMT2D;ASH2L |
| MBD1-MCAF1-SETDB1_complex | MBD1;ATF7IP;SETDB1 |
| HDAC2-asscociated_core_complex | MBD3;GATAD2A;MTA2 |
| HDAC1-associated_core_complex_cll | MBD3;GATAD2A;MTA2 |
| HCF-1_complex | SIN3B;SIN3A;ASH2L |

(continued)

| Complex Name | hits_in_complex |
|---|---|
| EBAFa_complex | SMARCB1;SMARCD1;ARID1A |
| DMAP1-associated_complex | BRD8;EPC1;TRRAP |
| CEN_complex | SUPT16H;FBL;SSRP1 |
| CDC5L_complex | PRKDC;SFPQ;SRSF1 |
| BAF_complex | SMARCB1;SMARCD1;ARID1A |
| Anti-HDAC2_complex | SIN3A;ZMYM3;MTA2 |
| p300-CBP-p270_complex | CREBBP;ARID1A |
| WRA_complex_(WDR5,_RBBP5,_ASH2L) | RBBP5;ASH2L |
| WRAD_complex_(WDRS,_RBBPS,_ASH 2L,_DPY30) | RBBP5;ASH2L |
| Ubiquitin_E3 ligase_(CSN1,_CSN8,_HRT 1,_SKP1,_SKP2,_CUL1,_CUL2,_CUL3) | SKP1;CUL3 |
| TIP6_ histone_acetylase_complex | KATS;TRRAP |
| TFTC-type_histone_acetyl_transferase_comp lex | KAT2A;TRRAP |
| SWI-SNF_chromatin_remodeling-related-BRCA1_complex | SMARCB1;ARID1A |
| SRC-3_complex | CREBBP;NCOA3 |
| SMAR1-HDAC1-SIN3A-SIN3B_repressor_complex | SIN3B;SIN3A |
| SMAR1-H DAC1-SIN3A-SIN3B-p107-p130_repressor_complex | SIN3B;SIN3A |
| SKI-NCOR1-SIN3A-HDAC1_complex | SIN3A;NCOR1 |
| SIN3-SAP25_complex | SIN3A;SAP130 |
| SETDB1-containing_HMTase_complex | ATF7IP;SETDB1 |
| SERCA2a-alphaKAP-CaM-CaMKII_complex | CALM1;CAMK2A |
| Ribosome,_cytoplasmic | RPL27;RPS4X |
| Replication-coupled_CAF-1-MBD1-ETDB1_complex | MBD1;SETDB1 |
| RSmad_complex | CREBBP;NCOA3 |
| RC_complex_during_S-phase_of_cell_cycle | PARP1;POLD1 |
| RC_complex_during_G2/M-phase_of_cell_cycle | PARP1;POLD1 |
| Polycomb_repressive_complex_4_(PRC 4) | EZH2;EED |
| Polycomb repressive complex 2_(PRC 2) | EZH2;EED |
| PCAF_complex | TAF6L;TRRAP |
| NIF1-ASH2L-RBBP5-WDR5_complex | RBBP5;ASH2L |
| NCOR2_complex | SIN3A;NCOR1 |
| NCOR1_complex | SMARCB1;NCOR1 |
| NCOR-SIN3-RPD3_complex | SIN3B;SIN3A |
| NCOR-SIN3-HDAC-HESX1_complex | SIN3B;SIN3A |
| NCOA6-DNA-PK-Ku-PARP1_complex | PARP1;PRKDC |
| Multisubunit_ACTR_coactivator_compl ex | CREBBP;NCOA3 |
| Mi2/NuRD_complex | MBD3;MTA2 |
| Mi-2/NuRD-MTA2_complex | MBD3;MTA2 |
| Menin-associated_histone_methyltransferase_ complex | RBBP5;ASH2L |

(continued)

| Complex Name | hits_in_complex |
|---|---|
| MLL1_core_complex | RBBP5;ASH2L |
| MLL1_complex | RBBP5;ASH2L |
| MLL-HCF_complex | RBBP5;ASH2L |
| MBD1-MCAF_complex | MBD1;ATF7IP |
| Kinase_maturation_complex_1 | YWHAE;YWHAZ |
| INO80_chromatin_remodeling_complex | INO80C;INO80 |
| ING4_complex_(ING4,_MYST2,_C1orf14 9,_PHF17) | ING4;MEAF6 |
| ING4_complex_(ING4,_MYST2,_C1orf14 9,_PHF16) | ING4;MEAF6 |
| ING4_complex_(ING4,_MYST2,_C1orf14 9,_PHF15) | ING4;MEAF6 |
| ING2_complex | SIN3A;SAP130 |
| HDAC1-associated_protein_complex | MBD3;MTA2 |
| HBO1_complex | ING4;MEAF6 |
| H2AX_complex,_isolated_from_cells_wi thout_IR_exposure | SUPT16H;SSRP1 |
| GCN5-TRRAP_histone_acetyltransferase_com plex | KAT2A;TRRAP |
| FIB-associated_protein_complex | FBL;PRMT1 |
| FACT_complex,_UV-activated | SUPT16H;SSRP1 |
| FACT_complex | SUPT16H;SSRP1 |
| FACT-NEK9_complex | SUPT16H;SSRP1 |
| Exosome | EXOSC1;EXOSC9 |
| Emerin_complex_52 | HDGF;YWHAE |
| Emerin_complex_25 | YWHAE;SAP130 |
| EED-EZH_polycomb complex | EZH2;EED |
| EED-EZH2_complex | EZH2;EED |
| EED-EZH-YY1_polycomb_complex | EZH2;EED |
| EBAFb_complex | SMARCB1;SMARCD1 |
| E2F-6_complex | E2F6;PCGF6 |
| CyclinD3-CDK4-CDK6_complex | CDK4;CDK6 |
| CyclinD3-CDK4-CDK6-p21_complex | CDK4;CDK6 |
| C_complex_spliceosome | SRSF1;PRPF4B |
| BRMS1-SIN3-HDAC_complex | SIN3B;SIN3A |
| BRCA1-BARD1-BRCA2-DNA_damage_complex_III | BARD1;BRCA2 |
| BCOR_complex | BCOR;SKP1 |
| B-WICH_complex | MYBBP1A;BAZ1B |
| ATAC_complex,_YEATS2-linked | AC118549.1;KAT2A |
| ATAC_complex,_GCN5-linked | AC118549.1;KAT2A |
| transcription_factor_IIIC_multisubunit_ complex | GTF3C4 |
| snRNP-free_U1A_(SF-A)-CtIP-CtBP_complex | SFPQ |
| p54(nrb)-PSF-matrin3_complex | SFPQ |
| p400-associated_complex | TRRAP |

(continued)

| Complex Name | hits_in_complex |
|---|---|
| p34(SEI-1)-CDK4-CyclinD2_complex | CDK4 |
| p27-cyclinE-Cdk2_-_Ubiquitin_E3_ligase_(SKP1A,_SKP2,_CUL1,_CKS1B_RBX1)_complex | SKP1 |
| p21(ras)GAP-Fyn-Lyn-Yes_complex,_thrombin_stimulated | LYN |
| p130Cas-ER-alpha-cSrc-kinase-_PI3-kinase_p85-subunit_complex | SRC |
| c-MYC-ATPase-helicase_complex | TRRAP |
| anti-BHC110_complex | ZMYM3 |
| ZO1-(beta)cadherin-(VE)cadherin-VEGFR2_complex | KDR |
| ZN F304-corepressor_complex | SETDB1 |
| XFIM_complex | ZMYM3 |
| WRN-Ku70-Ku80-PARP1_complex | PARP1 |
| WICH_complex | BAZ1B |
| Vigilin-DNA-PK-Ku_antigen_complex | PRKDC |
| VEcad-VEGFR_complex | KDR |
| VEGFR2-S1PR5-ERK1/2-PKC-alpha_complex | KDR |
| VEGFR2-S1PR3-ERK1/2-PKC-alpha_complex | KDR |
| VEGFR2-S1PR2-ERK1/2-PKC-alpha_complex | KDR |
| VEGFR2-S1PR1-ERK1/2-PKC-alpha_complex | KDR |
| VEGFA(165)-KDR-NRP1_complex | KDR |
| Ubiquitin_E3 ligase_(SPOP,_DAXX, CUL 3) | CUL3 |
| Ubiquitin_E3 ligase_(SMAD3, BTRC, C UL1,_SKP1A,_RBX1) | SKP1 |
| Ubiquitin_E3_ligase_(SKP1A,_SKP2,_CU L1,_RBX1) | SKP1 |
| Ubiquitin_E3_ligase_(SKP1A,_SKP2,_CU L1,_CKS1B,_RBX1) | SKP1 |
| Ubiquitin E3 ligase_(SKP1A, SKP2, CU L1) | SKP1 |
| Ubiquitin E3_ligase_(SKP1A, FBXW8,_ CUL7,_RBX1) | SKP1 |
| Ubiquitin E3_ligase_(SKP1A, FBXW2,_ CUL1) | SKP1 |
| Ubiquitin_E3 ligase_(SKP1A, BTRC, CU L1) | SKP1 |
| Ubiquitin_E3_ligase_(SIAH1,_SIP,_SKP1 A,_TBL1X) | SKP1 |
| Ubiquitin_E3 ligase_(NIPA, SKP1A, CU L1,_RBX1) | SKP1 |
| Ubiquitin_E3_ligase_(NFKBIA,_FBXW11, _BTRC,_CUL1,_SKP1A) | SKP1 |
| Ubiquitin_E3_ligase_(H2AFY,_SPOP,_CU L3) | CUL3 |
| Ubiquitin_E3_ligase_(GLMN,_FBXWB,_S KP1A,_RBX1) | SKP1 |
| Ubiquitin_E3_ligase_(FBXW7,_CUL1,_S KP1A,_RBX1) | SKP1 |
| Ubiquitin_E3 ligase_(FBXV1/11,_SKP1A, _CUL1,_RBX1) | SKP1 |
| Ubiquitin_E3_ligase_(FBXO31,_SKP1A,_ CUL1,_RBX1) | SKP1 |
| Ubiquitin_E3_ligase_(FBXO18,_SKP1A,_ CUL1,_RBX1) | SKP1 |
| Ubiquitin_E3_ligase_(CUL3,_KLHL3,_W NK4) | CUL3 |

(continued)

| Complex Name | hits_in_complex |
|---|---|
| Ubiquitin E3 ligase_(CUL3,_KLHL3, W NK1) | CUL3 |
| Ubiquitin_E3 ligase_(CUL3, KLHL3) | CUL3 |
| Ubiquitin_E3_ligase_(CUL1,_RBX1,_SKP 1) | SKP1 |
| Ubiquitin_E3 ligase_(CRY2,_SKP1A,_CU L1,_FBXL3) | SKP1 |
| Ubiquitin_E3_ligase_(CRY1,_SKP1A,_CU L1,_FBXL3) | SKP1 |
| Ubiquitin_E3_lligase_(CDC34,_NEDD8,_ BTRC,_CUL1,_SKP1A,_RBX1) | SKP1 |
| Ubiquitin_E3_ligase_(BMI1,_SPOP,_CUL 3) | CUL3 |
| URI_complex_(Unconventional_prefoldi n_RPBS Interactor) | SKP1 |
| ULK2-ATG13-RB1CC1_complex | ULK2 |
| Toposome | SSRP1 |
| Ternary_complex_(LRRC7,_CAMK2a,_A CTN4) | CAM K2A |
| TRRAP-BAF53-HAT_complex | TRRAP |
| TRIB3-DDIT3_complex | TRIB3 |
| TRBP_containing_complex_(DICER,_RPL 7A,_EIFS,_MOV10_and_subunits_of_th e_60S_ribosomal_particle) | RPL27 |
| TNF-alpha/NF-kappa_B_signaling_complex_6 | FBL |
| TNF-alpha/NF-kappa_B_signaling_complex_5 | SKP1 |
| TNF-alpha/NF-kappa_B_signaling_complex_10 | TBK1 |
| TNF-alpha/NF-kappa_B_signaling_complex_(CHUK,_B TRC,_NFK82,_PPP6C,_REL,_CUL1,_IKBK E,_SAPS2,_SAPS1,_ANKRD28,_RELA,_SK P1) | SKP1 |
| TFIIIC_containing-TOP1-SUB1_complex | GTF3C4 |
| TCF4-CTNNB1-CREBBP_complex | CREBBP |
| TBPIP/HOP2-MND1_complex | PSMC3IP |
| Succinyl-CoA_synthetase,_GDP-forming | SUCLG2 |
| Statl-alpha-dimer-CBP_DNA-protein_complex | CREBBP |
| Set/TAF-I_beta-TAF-I_alpha-PP32_complex | ANP32A |
| SWI/SNF_chromatin-remodeling_complex | SIN3A |
| STAGA_core_complex | KAT2A |
| SRCAP-associated_chromatin_remodeling_co mplex | YEATS4 |
| SRC-1_complex | CREBBP |
| SNF2H-BAZ1A_complex | BAZ1A |
| SMAD4-SKI-NCOR_complex | NCOR1 |
| SMAD3-cSKI-SIN3A-HDAC1_complex | SIN3A |
| SMAD3-SMAD4-FOXO1_complex | FOXO1 |
| SMAD3-SKI-NCOR_complex | NCOR1 |
| SMAD2-SKI-NCOR_complex | NCOR1 |
| SMAD1-CBP_complex | CREBBP |
| SIN3_complex | SIN3A |

(continued)

| Complex Name | hits_in_complex |
|---|---|
| SIN3-ING1b_complex_I | SIN3A |
| SETDB1-DNMT3B_complex | SETDB1 |
| SETDB1-DNMT3A_complex | SETDB1 |
| Rap1_complex | PARP1 |
| RNA_polymerase_II_complex,_incompl ete_(CDK8_complex),_chromatin_struct ure_modifying | SMARCB1 |
| REST-CoREST-mSIN3A_complex | SIN3A |
| RAF1-MAP2K1-YWHAE_complex | YWHAE |
| RAD6A-KCMF1-UBR4_complex | UBE2A |
| Prune/Nm23-H1_complex | NME1 |
| Protein_phosphatase_4_complex | PPP4C |
| Polycystin-1_multiprotein_complex_(ACTN1,_CDH 1,_SRC,_JUP,_VCL,_CTNNB1,_PXN,_BCA R1,_PKD1,_PTK2,_TLN1) | SRC |
| Phosphorylase_kinase_complex | CALM1 |
| Phosphatidylinositol_3-kinase_(PIK3CA,_PIK3R1) | PIK3CA |
| Paf_complex | PAF1 |
| PU.1-SIN3A-HDAC_complex | SIN3A |
| PSF-p54(nrb)_complex | SFPQ |
| PRMT1_complex | PRMT1 |
| PPP4C-PPP4R2-Gemin3-Gemin4_complex | PPP4C |
| POLR2A-CCNT1-CDK9-NCL-LEM6-CPSF2_complex | PPARGC1A |
| PLC-gamma-2-SLP-76-Lyn-Grb2_complex | LYN |
| PLC-gamma-2-Lyn-FcR-gamma_complex | LYN |
| PKA_(RII-alpha and RII-beta)-AKAPS-ADRB1_complex | PRKAR2B |
| PGC-1-SRp40-SRp55-SRp75_complex | PPARGC1A |
| PCNA_complex | CDK4 |
| PCNA-DNA_polymerase delta complex | POLD1 |
| P53-BARD1-Ku70_complex | BARD1 |
| OCT2-TLE4_complex | TLE4 |
| NuRD.1_complex | MBD3 |
| Neddylin_ligase_(FBXO11,_SKP1,_CUL1, _RBX1) | SKP1 |
| NK-3-Groucho-HIPK2-SIN3A-RbpA48-HDAC1_complex | SIN3A |
| NDPKA-AMPKalpha1_complex | NME1 |
| NCOR_complex | NCOR1 |
| NCOR-SIN3-HDAC1_complex | SIN3A |
| NCOR-HDAC3_complex | NCOR1 |
| Mi2/NuRD-BCL6-MTA3_complex | MBD3 |
| MeCP2-SIN3A-HDAC_complex | SIN3A |
| MTA1_complex | MBD3 |

(continued)

| Complex Name | hits_in_complex |
|---|---|
| MSL_complex | MSL3 |
| MRN-TRRAP complex_(MRE11A-RAD50-NBN-TRRAP_complex) | TRRAP |
| MGC1-DNA-PKcs-Ku_complex | PRKDC |
| MEP50-PRMTS-ICLN_complex | CLNS1A |
| MCM8-ORC2-CDC6_complex | CDC6 |
| MBD1-Suv39h1-HP1_complex | MBD1 |
| MAP2K1-BRAF-RAF1-YWHAE-KSR1_complex | YWHAE |
| MAK-ACTR-AR_complex | NCOA3 |
| MAD1-mSin3A-HDAC2_complex | SIN3A |
| Kinase_maturation_complex_2 | TBK1 |
| Kaiso-NCOR_complex | NCOR1 |
| JBP1-plCln_complex | CLNS1A |
| ITGAV-ITGB3-SLC3A2_complex | SLC3A2 |
| ITGA2b-ITGB3-CD47-SRC_complex | SRC |
| ING5_complex | MEAF6 |
| IKK-alpha--ER-alpha-AIB1_complex | NCOA3 |
| IGF1R-CXCR4-GNAI2-GNB1_complex | IGF1R |
| HuCHRAC_complex | BAZ1A |
| HUIC_complex | BARD1 |
| HMGB1-HMGB2-HSC70-ERP60-GAPDH_complex | GAPDH |
| HES1_promoter_corepressor_complex | CREBBP |
| HES1_promoter-Notch_enhancer_complex | SUPT16H |
| HERP1/HEY2-NCOR-SIN3A_complex | SIN3A |
| H2AX_complex_I | PARP1 |
| GAIT_complex | GAPDH |
| FOXO3-CBP_complex | CREBBP |
| FOXO1-FHL2-SIRT1_complex | FOXO1 |
| FGFR2-c-Cbl-Lyn-Fyn_complex | LYN |
| FGFR1c-KL_complex | FGFR1 |
| FGF23-FGFR1c-KL_complex | FGFR1 |
| FGF21-FGFR1c-KLB_complex | FGFR1 |
| FE65-TSHZ3-HDAC1_complex | TSHZ3 |
| F1F0-ATP_synthase,_mitochondrial | ATP5F1C |
| Ezh2_methyltransferase_complex,_cyto solic | EED |
| Emerin_complex_32 | SMARCB1 |
| Emerin_complex_24 | SAP130 |
| Elongator_holo_complex | ELP2 |
| Ecsit_complex_(ECSIT,_MT-CO2,_GAPDH,_TRAF6,_NDUFAF1) | GAPDH |

(continued)

| Complex Name | hits_in_complex |
|---|---|
| ETS2-SMARCA4-INI1_complex | SMARCB1 |
| ESR1-RELA-BCL3-NCOA3_complex | NCOA3 |
| ERBB3-SPG1_complex | ERBB3 |
| DSS1_complex | BRCA2 |
| DRD4-KLHL12-CUL3_complex | CUL3 |
| DNTTIP1-ZNF541-HDAC1-HDAC2_complex | ZNF541 |
| DNMT3B_complex | SIN3A |
| DNA_synthesome_complex_(17_subuni ts) | POLD1 |
| DNA-PK-Ku_complex | PRKDC |
| DNA-PK-Ku-eIF2-NF90-NF45_complex | PRKDC |
| DHX9-ADAR-vigilin-DNA-PK-Ku_antigen_complex | PRKDC |
| DA_complex | TAF3 |
| DAXX-MDM2-USP7_complex | USP7 |
| DAB_complex | TAF3 |
| Cytochrome_c_oxidase,_mitochondrial | COX4I1 |
| Condensin_I-PARP-1-XRCC1_complex | PARP1 |
| Cell_cycle_kinase_complex_CDK4 | CDK4 |
| CUL4A-DDB1-RBBP5_complex | RBBP5 |
| CUL4A-DDB1-EED_complex | EED |
| CS-MAP3K7IP1-MAP3K7IP2_complex | CS |
| CREBBP-SMAD3_hexameric_complex | CREBBP |
| CREBBP-SMAD3-SMAD4_pentameric_complex | CREBBP |
| CREBBP-SMAD2_hexameric_complex | CREBBP |
| CREBBP-SMAD2-SMAD4_pentameric_complex | CREBBP |
| CREBBP-KAT2B-MYOD1_complex | CREBBP |
| CNK1-SRC-RAF1_complex | SRC |
| CHTOP-methylosome_complex | PRMT1 |
| CF_IIAm_complex_(Cleavage_factor_IIA m_complex) | SFPQ |
| CEP164-TTBK2_complex | TTBK2 |
| CDC7-DBF7_complex | CDC7 |
| CD98-LAT2-ITGB1_complex | SLC3A2 |
| CD20-LCK-LYN-FYN-p75/80 complex,_(Raji human_B cell_ line) | LYN |
| CCND3-CDK6_complex | CDK6 |
| CCND3-CDK4_complex | CDK4 |
| CCND2-CDK6_complex | CDK6 |
| CCND2-CDK4_complex | CDK4 |
| CCND1-CDK6_complex | CDK6 |
| CCND1-CDK4_complex | CDK4 |
| CCDC22-COM M D8-CU L3_complex | CUL3 |

(continued)

| Complex Name | hits_in_complex |
|---|---|
| CBP-RARA-RXRA-DNA_complex,_ligand_stimulated | CREBBP |
| CAS-SRC-FAK_complex | SRC |
| CAND1-CUL3-RBX1_complex | CUL3 |
| CALM1-_KCNQ4(splice_variant_2)_complex | CALM1 |
| CALM1-KCNQ4(splice_variant_1)_complex | CALM1 |
| BRCC_complex | BRCA2 |
| BRCA1_C_complex | BARD1 |
| BRCA1_B_complex | BARD1 |
| BRCA1_A_complex | BARD1 |
| BRCA1-IRIS-pre-replication_complex | CDC6 |
| BRCA1-BARD1-UbcH7c_complex | BARD1 |
| BRCA1-BARD1-UbcH5c_complex | BARD1 |
| BRCA1-BARD1-POLR2A_complex | BARD1 |
| BRCA1-BARD1-BACH1-DNA_damage_complex_II | BARD1 |
| BRCA1-BARD1-BACH1-DNA_damage_complex_I | BARD1 |
| BRAF53-BRCA2_complex | BRCA2 |
| BRAF-RAF1-14-3-3_complex | YWHAZ |
| BRAF-MAP2K1-MAP2K2-YWHAE_complex | YWHAE |
| BARD1-BRCA1-CSTF_complex | BARD1 |
| BARD1-BRCA1-CSTF64_complex | BARD1 |
| Artemis-DNA-PK_complex | PRKDC |
| Anti-Sm_protein_complex | CLNS1A |
| ASF1-histone_containing_complex | CHEK2 |
| ARC_complex | ACAD8 |
| ANKS6-NEK8-INVS-NPHP3_complex | NPHP3 |
| AMY-1-S-AKAP84-RII-beta_complex | PRKAR2B |
| AJUBA-GFI1-HDAC3_complex | GFI1 |
| AJUBA-GFI1-HDAC2_complex | GFI1 |
| AJUBA-GFI1-HDAC1_complex | GFI1 |
| 9b-1-1_complex | HUS1 |
| 9-1-1_complex | HUS1 |
| 9-1-1-RHINO_complex | HUS1 |
| 9-1-1-RAD17-RFC_complex | HUS1 |
| 9-1-1-POLB_complex | HUS1 |
| 9-1-1-LIG1_complex | HUS1 |
| 9-1-1-FEN1_complex | HUS1 |
| 9-1-1-APE1_complex | HUS1 |
| 6S _methyltransferase_complex | CLNS1A |
| 6S_methyltransferase_and_RG-containing_Sm_protein-s_complex | CLNS1A |

(continued)

| Complex Name | hits_in_complex |
|---|---|
| 60S_ribosomal_subunit,_cytoplasmic | RPL27 |
| 5S-DNA-TFIIIA-TFIIIC2_subcomplex | GTF3C4 |
| SS-DNA-TFIIIA-TFIIIC2-TFIIIB_subcomplex | GTF3C4 |
| 40S_ribosomal_subunit,_cytoplasmic | RPS4X |
| 20S_methyltransferase_core_complex | CLNS1A |
| 20S_methylosome_and_RG-containing_Sm_protein_complex | CLNS1A |
| 20S_methylosome-SmD_complex | CLNS1A |
| 17S_U2_snRNP | SRSF1 |

*Molecular pathway analysis:*

To identify top molecular pathways enriched with multiple targets, the top targets were overlapped with KEGG pathway maps using the clusterProfiler R package. Top pathways are shown in Table 5 derived from hits identified using method 2.

Table 5: Molecular pathways associated with targets that upregulate HbF

| ID | Description | geneID | p.adjust | qvalue |
|---|---|---|---|---|
| hsa04922 | Glucagon signaling pathway | 32/207/801/808/816/817/818/1375/2538/92579/2645/160287/3945/441531/5563/5567/3276/5834 | 1.32E-08 | 7.39E-09 |
| hsa01200 | Carbon metabolism | 35/128/226/275/847/1431/1962/2597/2645/3418/3421/5091/5095/441531/25796/5631/8802/7167 | 5.10E-08 | 2.85E-08 |
| hsa04921 | Oxytocin signaling pathway | 107/113/114/115/801/808/57172/816/817/818/1026/29904/1956/5607/4638/85366/5563/5567/9475/6714 | 8.08E-08 | 4.51E-08 |
| hsa00010 | Glycolysis / Gluconeoge nesis | 127/128/226/669/2538/92579/130589/2597/2645/160287/3945/441531/7167 | 5.57E-07 | 3.11E-07 |
| hsa01522 | Endocrine resistance | 107/113/114/115/207/1026/1027/1956/3480/5600/5603/5291/5567/5925/6714 | 5.68E-07 | 3.18E-07 |
| hsa04912 | GnRH signaling pathway | 107/113/114/115/801/808/816/817/818/1956/5600/5603/5567/6714 | 2.16E-06 | 1.20E-06 |
| hsa04114 | Oocyte meiosis | 107/113/114/115/6790/801/808/816/817/818/286151/3480/5567/6197/7531/7534 | 2.16E-06 | 1.20E-06 |
| hsa00071 | Fatty acid degradation | 35/37/127/128/1375/1376/1579/10455/1962/2639 | 2.69E-06 | 1.50E-06 |
| hsa04750 | Inflammator y mediator regulation of TRP channels | 107/113/114/115/801/808/816/817/818/5600/5603/5291/5567/6714 | 2.79E-06 | 1.56E-06 |
| hsa04015 | Rap1 signaling pathway | 107/113/114/115/207/801/808/1956/2260/2324/3480/3690/9223/9863/260425/5600/5603/5291/23683/6714 | 4.14E-06 | 2.31E-06 |
| hsa04971 | Gastric acid secretion | 107/113/114/115/801/808/816/817/818/4638/85366/5567 | 6.06E-06 | 3.39E-06 |

(continued)

| ID | Description | geneID | p.adjust | qvalue |
|---|---|---|---|---|
| hsa04611 | Platelet activation | 107/113/114/115/207/3690/4067/5600/5603/4638/85366/5291/5567/9475/6714 | 7.03E-06 | 3.93E-06 |
| hsa05214 | Glioma | 207/801/808/816/817/818/1026/1956/3480/5291/5925 | 2.29E-05 | 1.28E-05 |
| hsa04722 | Neurotrophi n signaling pathway | 207/27018/801/808/816/817/818/51135/5607/5600/5603/5291/6197/7531 | 2.34E-05 | 1.31E-05 |
| hsa01230 | Biosynthesis of amino acids | 226/445/586/1431/2597/3418/3421/5091/441531/5631/7167 | 3.03E-05 | 1.69E-05 |
| hsa00280 | Valine, leucine and isoleucine degradation | 27034/35/316/549/586/1962/11112/3157/5095 | 3.44E-05 | 1.92E-05 |
| hsa04213 | Longevity regulating pathway - multiple species | 107/113/114/115/207/847/3480/5291/5563/5567 | 3.71E-05 | 2.07E-05 |
| hsa04925 | Aldosterone synthesis and secretion | 107/113/114/115/801/808/57172/816/817/818/5567/23683 | 5.60E-05 | 3.13E-05 |
| hsa04914 | Progesteron e-mediated oocyte maturation | 107/113/114/115/207/6790/3480/5600/5603/5291/5567/6197 | 7.36E-05 | 4.11E-05 |
| hsa04066 | HIF-1 signaling pathway | 207/226/816/817/818/1026/1027/1956/2597/3480/5163/5291 | 7.77E-05 | 4.34E-05 |
| hsa04012 | ErbB signaling pathway | 207/816/817/818/1026/1027/1956/2065/57144/5291/6714 | 8.70E-05 | 4.86E-05 |
| hsa04714 | Thermogene sis | 107/113/114/115/8289/509/1375/1376/2260/51780/5600/5603/5563/5567/6197/6598/6599/7384 | 0.000164 951 | 9.22E-05 |
| hsa04068 | FoxO signaling pathway | 207/847/1026/1027/1956/2538/92579/3480/5600/5603/5291/5563/3276 | 0.000246 041 | 0.000137 489 |
| hsa05230 | Central carbon metabolism in cancer | 207/1956/2260/2322/2645/5163/441531/5291/23410 | 0.000302 864 | 0.000169 242 |
| hsa04720 | Long-term potentiation | 107/114/801/808/816/817/818/5567/619 7 | 0.000364 175 | 0.000203 503 |
| hsa05205 | Proteoglyca ns in cancer | 207/816/817/818/1026/1956/2065/2260/3480/3690/5600/5603/5291/5567/9475/6 714 | 0.000364 175 | 0.000203 503 |
| hsa04020 | Calcium signaling pathway | 107/113/114/115/801/808/816/817/818/1956/2065/80271/4638/85366/5567 | 0.000412 694 | 0.000230 615 |
| hsa04261 | Adrenergic signaling in cardiomyoc ytes | 107/113/114/115/207/801/808/816/817/818/5600/5603/5567 | 0.000508 051 | 0.000283 901 |
| hsa04931 | Insulin resistance | 32/207/1375/2538/92579/5291/5563/5834/6197/10998/57761 | 0.000553 97 | 0.000309 561 |
| hsa04211 | Longevity regulating pathway | 107/113/114/115/207/847/3480/5291/5563/5567 | 0.000553 97 | 0.000309 561 |

(continued)

| ID | Description | geneID | p.adjust | qvalue |
|---|---|---|---|---|
| hsa04973 | Carbohydrat e digestion and absorption | 207/2538/92579/8972/5291/6518/6523 | 0.000746 2 | 0.000416 98 |
| hsa00640 | Propanoate metabolism | 32/1962/160287/3945/5095/8802 | 0.000899 319 | 0.000502 544 |
| hsa04713 | Circadian entrainment | 107/113/114/115/801/808/816/817/818/ 5567 | 0.000960 425 | 0.000536 69 |
| hsa04910 | Insulin signaling pathway | 32/207/801/808/2538/92579/2645/5291/ 5563/5567/5577/5834 | 0.001081 413 | 0.000604 298 |
| hsa01212 | Fatty acid metabolism | 35/37/1375/1376/1962/3992/27349 | 0.001167 221 | 0.000652 248 |
| hsa05418 | Fluid shear stress and atherosdero sis | 207/445/801/808/3690/5607/5600/5603/ 4258/5291/5563/6714 | 0.001172 205 | 0.000655 034 |
| hsa04916 | Melanogene sis | 107/113/114/115/801/808/816/817/818/ 5567 | 0.001309 791 | 0.000731 917 |
| hsa04270 | Vascular smooth muscle contraction | 107/113/114/115/801/808/1579/4638/85 366/5567/9475 | 0.001317 487 | 0.000736 218 |
| hsa04911 | Insulin secretion | 107/113/114/115/816/817/818/2645/556 7 | 0.001562 467 | 0.000873 113 |
| hsa04923 | Regulation of lipolysis in adipocytes | 107/113/114/115/207/5291/5567 | 0.002165 171 | 0.001209 907 |
| hsa04926 | Relaxin signaling pathway | 107/113/114/115/207/1956/5600/5603/5 291/5567/6714 | 0.002287 353 | 0.001278 183 |
| hsa04024 | cAMP signaling pathway | 107/113/114/115/207/801/808/816/817/ 818/2867/5291/5567/9475 | 0.002397 555 | 0.001339 764 |
| hsa00480 | Glutathione metabolism | 2729/2880/257202/3418/4258/6241/510 60 | 0.002486 55 | 0.001389 495 |
| hsa04934 | Cushing's syndrome | 107/113/114/115/816/817/818/1026/102 7/1956/5567/5925 | 0.002486 55 | 0.001389 495 |
| hsa04725 | Cholinergic synapse | 107/113/114/115/207/816/817/818/5291 /5567 | 0.002502 724 | 0.001398 533 |
| hsa00650 | Butanoate metabolism | 35/622/56898/1962/3157 | 0.003003 088 | 0.001678 139 |
| hsa04371 | Apelin signaling pathway | 107/113/114/115/207/801/808/4638/853 66/5563/5567 | 0.003058 932 | 0.001709 345 |
| hsa04915 | Estrogen signaling pathway | 107/113/114/115/207/801/808/1956/529 1/5567/6714 | 0.003058 932 | 0.001709 345 |
| hsa00310 | Lysine degradation | 1962/2146/2639/58508/93166/9739/986 9 | 0.003065 186 | 0.001712 839 |
| hsa05215 | Prostate cancer | 207/1026/1027/1956/2260/3480/3645/52 91/5925 | 0.003271 725 | 0.001828 254 |
| hsa00020 | Citrate cycle (TCA cycle) | 1431/3418/3421/5091/8802 | 0.003771 776 | 0.002107 685 |

(continued)

| ID | Description | geneID | p.adjust | qvalue |
|---|---|---|---|---|
| hsa00270 | Cysteine and methionine metabolism | 262/586/1786/2729/160287/3945 | 0.003799 784 | 0.002123 336 |
| hsa04152 | AMPK signaling pathway | 32/207/1375/29904/2538/92579/3480/5210/5291/5563 | 0.003914 111 | 0.002187 222 |
| hsa01210 | 2-Oxocarboxyl ic acid metabolism | 586/1431/3418/3421 | 0.003949 829 | 0.002207 182 |
| hsa00052 | Galactose metabolism | 2538/92579/130589/2645/8972 | 0.004086 582 | 0.002283 6 |
| hsa04913 | Ovarian steroidogen esis | 107/113/114/115/3480/5567 | 0.005577 474 | 0.003116 717 |
| hsa04540 | Gap junction | 107/113/114/115/1956/5607/5567/6714 | 0.006503 423 | 0.003634 141 |
| hsa00072 | Synthesis and degradation of ketone bodies | 622/56898/3157 | 0.006781 885 | 0.003789 748 |
| hsa00500 | Starch and sucrose metabolism | 2538/92579/2645/8972/5834 | 0.007588 73 | 0.004240 616 |
| hsa04976 | Bile secretion | 107/113/114/115/5567/10998/6523 | 0.007588 73 | 0.004240 616 |
| hsa05218 | Melanoma | 207/1026/1956/2260/3480/5291/5925 | 0.008097 086 | 0.004524 688 |
| hsa04918 | Thyroid hormone synthesis | 107/113/114/115/2880/257202/5567 | 0.009333 62 | 0.005215 668 |

*Consistency across two different CRISPR libraries:*

To gain more confidence on the identified targets, an additional CRISPR library (library #2) with different set of genes and corresponding gRNAs was used. Only the HbF+ and FACs input samples were sequenced with library #2. Hits in library #2 were identified using method 2 (cutoff changed to 1.0) and without the dropout filter. Using this approach, a total of 209 hits were identified (FIG. 6B). Several common hits were identified in both libraries (FIG. 5B and Table 6).

Table 6: Hits identified using independent CRISPR libraries

| Gene Name | Uniprot ID | Description |
|---|---|---|
| TK2 | 000142 | thymidine kinase 2, mitochondrial |
| HIST1H1B | P16401 | histone cluster 1 H1 family member b |
| BMX | P51813 | BMX non-receptor tyrosine kinase |
| G6PC3 | Q9BUM1 | glucose-6-phosphatase catalytic subunit 3 |
| IDH3G | P51553 | isocitrate dehydrogenase 3 (NAD(+)) gamma |
| PRPS1 | P60891 | phosphoribosyl pyrophosphate synthetase 1 |
| PDK3 | Q15120 | pyruvate dehydrogenase kinase 3 |
| MBD3 | O95983 | methyl-CpG binding domain protein 3 |
| TYRO3 | Q06418 | TYRO3 protein tyrosine kinase |
| EPHAS | P54756 | EPH receptor A5 |
| BDH2 | Q9BUT1 | 3-hydroxybutyrate dehydrogenase 2 |
| CDKN1B | Q6I9V6 | cyclin dependent kinase inhibitor 1B |
| PRMT2 | P55345 | protein arginine methyltransferase 2 |

(continued)

| Gene Name | Uniprot ID | Description |
|---|---|---|
| MAP4K4 | 095819 | mitogen-activated protein kinase kinase kinase kinase 4 |
| INO80C | Q6PI98 | INO80 complex subunit C |
| SRSF3 | P84103 | serine and arginine rich splicing factor 3 |
| ADCY7 | P51828 | adenylate cyclase 7 |
| TADA1 | Q96BN2 | transcriptional adaptor 1 |
| IKZF1 | R9R4D9 | IKAROS family zinc finger 1 |
| PARP1 | P09874 | poly(ADP-ribose) polymerase 1 |
| PKN3 | Q6P5Z2 | protein kinase N3 |
| MVK | Q03426 | mevalonate kinase |
| CTBP1 | X5D8Y5 | C-terminal binding protein 1 |
| CUL4A | Q13619 | cullin 4A |
| AKT1 | P31749 | AKT serine/threonine kinase 1 |
| GLYR1 | | glyoxylate reductase 1 homolog |
| ACAD8 | Q9UKU7 | acyl-CoA dehydrogenase family member 8 |

*Expression specificity of hits in blood tissue and erythroid lineage:*

Hits identified using method 2 were prioritized based on their expression in blood tissue, relevant to SCD. This was performed using GTEx gene expression data from 15,598 samples across 31 different tissues (The GTEx Consortium. Nature Genetics). A mean Z-score was calculated to identify genes with high blood specific expression. The blood Z-scores for hits were calculated as follows:

$$Z_{g,blood} = mean_{i \in blood}\left(\frac{g_i - \mu_g}{\sigma_g}\right)$$

In the above equation. $Z_{g,blood}$ is the mean Z-score of gene "g" in blood tissue, $g_i$ is the expression of gene "g" in sample "i", $\mu_g$ is the mean expression of gene "g" across all samples, and $\sigma_g$, is the standard deviation of gene "g" across all samples. In total, 32 hits were identified that had a $Z_{g,blood}$ greater than 1 (FIG. 7A and Table 7).

Table 7: Additional drug targets identified using blood-specific network

| Gene Name | Uniprot ID | Description | Blood_mean_Zscore |
|---|---|---|---|
| PGAM4 | Q8N0Y7 | phosphoglycerate mutase family member 4 | 1.165971631 |
| IKZF2 | Q9UKS7 | IKAROS family zinc finger 2 | 1.549012532 |
| USP3 | Q9Y6I4 | ubiquitin specific peptidase 3 | 1.198035702 |
| MSL3 | Q8N5Y2 | MSL complex subunit 3 | 2.809489699 |
| HIST1H1B | P16401 | histone cluster 1 H1 family member b | 1.266391878 |
| BMX | P51813 | BMX non-receptor tyrosine kinase | 1.82329169 |
| NADK | 095544 | NAD kinase | 2.357039301 |
| HIST1H3D | P68431 | histone cluster 1 H3 family member d | 1.940003256 |
| PADI4 | Q9UM07 | peptidyl arginine deiminase 4 | 3.284882803 |
| RRM2 | P31350 | ribonucleotide reductase regulatory subunit M2 | 1.58105877 |
| TPI1 | V9HWK1 | triosephosphate isomerase 1 | 1.110545454 |
| PDK3 | Q15120 | pyruvate dehydrogenase kinase 3 | 1.461996437 |

(continued)

| Gene Name | Uniprot ID | Description | Blood_mean_Zscore |
|---|---|---|---|
| PFKFB4 | Q66S35 | 6-phosphofructo-2-kinase/fructose-2,6-biphosphatase 4 | 3.170252799 |
| COTL1 | Q14019 | coactosin like F-actin binding protein 1 | 3.522557555 |
| LYN | P07948 | LYN proto-oncogene, Src family tyrosine kinase | 3.60867428 |
| MGAM | 043451 | maltase-glucoamylase | 2.203722836 |
| PHF12 | Q96QT6 | PHD finger protein 12 | 1.445134764 |
| SIRT7 | Q9NRC8 | sirtuin 7 | 1.011603642 |
| PHC2 | Q8IXK0 | polyhomeotic homolog 2 | 1.528946092 |
| FFAR2 | 015552 | free fatty acid receptor 2 | 3.013584729 |
| FES | P07332 | FES proto-oncogene, tyrosine kinase | 1.938512739 |
| ADCY7 | P51828 | adenylate cyclase 7 | 1.667462363 |
| IKZF3 | Q9U KT9 | IKAROS family zinc finger 3 | 2.223300296 |
| IKZF1 | R9R4D9 | IKAROS family zinc finger 1 | 2.970394101 |
| TPK1 | Q9H3S4 | thiamin pyrophosphokinase 1 | 1.798433907 |
| STK17A | Q9UEE5 | serine/threonine kinase 17a | 2.137292947 |
| APOBEC3G | Q9HC16 | apolipoprotein B mRNA editing enzyme catalytic subunit 3G | 2.766529254 |
| APOBEC3H | M4W6S4 | apolipoprotein B mRNA editing enzyme catalytic subunit 3H | 2.353495477 |
| MAST3 | 060307 | microtubule associated serine/threonine kinase 3 | 1.933987547 |
| IRAK4 | Q9NWZ3 | interleukin 1 receptor associated kinase 4 | 1.511622129 |
| GAPDH | V9HVZ4 | glyceraldehyde-3-phosphate dehydrogenase | 1.124617068 |
| BPGM | P07738 | bisphosphoglycerate mutase | 1.876857003 |

Blood tissue is heterogeneous with many different cell-types, which are not all relevant to SCD. To focus on erythroid lineage, which is primarily affected in SCD, hits were overlapped with lineage specific modules identified by DMAP project (Novershtem et al, Cell). Many hits were identified that were expressed in progenitor and late erythroid lineages (Table 8) (FIGS. 7B and 7C).

Table 8: Hits with specific induction pattern in erythroid lineage

| Hit | Induction_pattern |
|---|---|
| AKT1 | Earlt Mye, T/B-cell and GRANs |
| ROCK2 | Earlt Mye, T/B-cell and GRANs |
| TTBK2 | Earlt Mye, T/B-cell and GRANs |
| TBK1 | Earlt Mye, T/B-cell and GRANs |
| SUCLG1 | Earlt Mye, T/B-cell and GRANs |
| TAFSL | Earlt Mye, T/B-cell and GRANs |
| PGLS | Earlt Mye, T/B-cell and GRANs |
| SETDB1 | Earlt Mye, T/B-cell and GRANs |
| ADCY7 | Earlt Mye, T/B-cell and GRANs |
| NAP1L1 | Earlt Mye, T/B-cell and GRANs |
| RPL27 | Earlt Mye, T/B-cell and GRANs |
| HMGN2 | Earlt Mye, T/B-cell and GRANs |
| DGUOK | Earlt Mye, T/B-cell and GRANs |

(continued)

| Hit | Induction_pattern |
|---|---|
| SPEN | Earlt Mye, T/B-cell and GRANs |
| ARID4A | Earlt Mye, T/B-cell and GRANs |
| PRPF4B | Earlt Mye, T/B-cell and GRANs |
| MYBBP1A | Earlt Mye, T/B-cell and GRANs |
| FBL | Earlt Mye, T/B-cell and GRANs |
| PARP1 | Earlt Mye, T/B-cell and GRANs |
| ADH5 | Earlt Mye, T/B-cell and GRANs |
| SMARCC1 | Earlt Mye, T/B-cell and GRANs |
| CTBP1 | Earlt Mye, T/B-cell and GRANs |
| EXOSC9 | Earlt Mye, T/B-cell and GRANs |
| ARID1A | Earlt Mye, T/B-cell and GRANs |
| MTF2 | Earlt Mye, T/B-cell and GRANs |
| PRKDC | Earlt Mye, T/B-cell and GRANs |
| RNF8 | Earlt Mye, T/B-cell and GRANs |
| YEATS2 | Earlt Mye, T/B-cell and GRANs |
| ACACB | Earlt Mye, T/B-cell and GRANs |
| LDHB | Earlt Mye, T/B-cell and GRANs |
| PRKACB | Earlt Mye, T/B-cell and GRANs |
| BDH2 | Earlt Mye, T/B-cell and GRANs |
| PRKD3 | Earlt Mye, T/B-cell and GRANs |
| HMG20A | Earlt Mye, T/B-cell and GRANs |
| PIK3C2A | Earlt Mye, T/B-cell and GRANs |
| CHD1 | Earlt Mye, T/B-cell and GRANs |
| SRP72 | Earlt Mye, T/B-cell and GRANs |
| CS | Earlt Mye, T/B-cell and GRANs |
| HLTF | Earlt Mye, T/B-cell and GRANs |
| NASP | Earlt Mye, T/B-cell and GRANs |
| HMGCS1 | Earlt Mye, T/B-cell and GRANs |
| EHHADH | HSC, Early Mye |
| MAGI2 | HSC, Early Mye |
| HIST1H3D | HSC, Early Mye |
| EZH2 | HSC, Early Mye |
| NME7 | HSC, Early Mye |
| IKZF2 | HSC, Early Mye |
| IGF1R | HSC, Early Mye |
| IDH2 | HSC, Early Mye |
| SSRP1 | HSC, Early Mye |
| DTYMK | HSC, Early Mye |
| GAPDH | HSC, Early Mye |

(continued)

| Hit | Induction_pattern |
|---|---|
| PCCA | HSC, Early Mye |
| ALDOA | HSC, Early Mye |
| USP46 | HSC, Early Mye |
| TPI1 | HSC, Early Mye |
| PIK3CB | HSC, Early Mye |
| G6PC3 | HSC, Early Mye |
| MGST2 | HSC, Early Mye |
| FLT3 | HSC, Early Mye |
| CDKN1C | HSC, Early Mye |
| MYLK | HSC, Early Mye |
| BCAT1 | HSC, Early Mye |
| SMARCA1 | HSC, Early Mye |
| FADS1 | HSC, Early Mye |
| CUL3 | Late ERY, T/B-cell and GRANs |
| SAP130 | Late ERY, T/B-cell and GRANs |
| PRPS1 | Late ERY, T/B-cell and GRANs |
| NAP1L4 | Late ERY, T/B-cell and GRANs |
| GCLC | Late ERY, T/B-cell and GRANs |
| CUL4A | Late ERY, T/B-cell and GRANs |
| GCDH | Late ERY, T/B-cell and GRANs |
| NEK1 | Late ERY, T/B-cell and GRANs |
| HIRA | Late ERY, T/B-cell and GRANs |
| MST1 | Late ERY, T/B-cell and GRANs |
| SPOP | Late ERY, T/B-cell and GRANs |
| GOLGA5 | Late ERY, T/B-cell and GRANs |
| AUH | Late ERY, T/B-cell and GRANs |
| MAST3 | Late ERY, T/B-cell and GRANs |
| CDKN1B | Late ERY, T/B-cell and GRANs |
| UBR2 | Late ERY, T/B-cell and GRANs |
| MAP4K4 | Late ERY, T/B-cell and GRANs |
| TAF10 | Late ERY, T/B-cell and GRANs |
| HDGF | Late ERY, T/B-cell and GRANs |
| YWHAE | Late ERY, T/B-cell and GRANs |
| AMD1 | Late ERY, T/B-cell and GRANs |
| EID1 | Late ERY, T/B-cell and GRANs |
| HIF1AN | Late ERY, T/B-cell and GRANs |
| CDK8 | Late ERY, T/B-cell and GRANs |
| DCK | Late ERY, T/B-cell and GRANs |
| FXR2 | Late ERY, T/B-cell and GRANs |

(continued)

| Hit | Induction_pattern |
|---|---|
| UQCRC1 | Late ERY, T/B-cell and GRANs |
| TESK2 | Late ERY, T/B-cell and GRANs |
| ADCK2 | Late ERY, T/B-cell and GRANs |
| USP21 | Late ERY, T/B-cell and GRANs |
| CAMK2D | Late ERY, T/B-cell and GRANs |
| FGFR1 | Late ERY, T/B-cell and GRANs |
| PHC2 | Late ERY |
| UBE2H | Late ERY |
| BPGM | Late ERY |
| SIRT2 | Late ERY |
| SIRT3 | Late ERY |
| NFYC | Late ERY |
| CPT2 | Late ERY |
| ITGB3 | MYE |
| AURKA | MYE |
| RRM2 | MYE |
| PRKAR2B | MYE |
| TOP2A | MYE |
| WRB | MYE |
| CAT | MYE |
| RMI1 | MYE |

Table 9 provides a list of various components of complexes and pathways identified herein as targets for increasing expression of HbF. Any of these may be targeted according to any of the methods disclosed herein.

Table 9: Complexes associated with hits and the other complex subunits within hits

| ComplexName | hit_members | other_members |
|---|---|---|
| ALL-1 supercomplex | SIN3A;MBD3;S MARCB1;SMAR CC1;MTA2 | SAP18;CHD3;WDR5;KDM1A;HDAC1;HDAC2;KMT2A; CPSF2;RAN;RBBP4;RBBP5;RBBP7;SMARCA2;SMARC C2;TAF1;TAF6;TAF9;TAF12;TBP;SYMPK;SMARCAS;S AP30;EFTUD2 |
| Anti-HDAC2 complex | HMG20B;SIN3A ;MTA2 | CHD3;CHD4;KDM1A;RCOR1:GSE1;GTF2I;HDAC1;HD AC2;PHF21A;RBBP4;RBBP7;ZMYM2;MTA1;ZMYM3 |
| BAF complex | SMARCB1;SMA RCC1;ARID1A | ACTL6B;ARID1B;ACTB;SMARCA2;SMARCA4;SMARCC 2;SMARCD1;SMARCE1;ACTG1;ACTL6A |
| BRG1-SIN3A complex | SIN3A;SMARCB 1;SMARCC1;ARI D1A | PRMT5;HDAC2;RBBP4;SMARCA4;SMARCC2;SMARC D1;SMARCD2;SMARCD3;SMARCE1;ACTL6A |
| BRM-SIN3A complex | SIN3A;SMARCB 1;SMARCC1;ARI D1A | PRMT5;HDAC1;HDAC2;RBBP4;SMARCA2;SMARCC2; SMARCD1;SMARCD2;SMARCD3;SMARCE1;ACTL6A |
| BRM-SIN3A-HDAC complex | SIN3A;SMARCB 1;SMARCC1;ARI D1A | PRMTS;HDAC2;SMARCA2;SMARCC2;SMARCD1;SMA RCD2;SMARCE1;ACTL6A |
| EBAFa complex | SMARCB1;SMA RCC1;ARID1A | MLLT1;SMARCA4;SMARCC2;SMARCD1;SMARCD2;S MARCE1;ACTL6A |
| GCN5-TRRAP histone acetyltransferas e complex | TADA3;TAF5 L;T AF10 | KAT2A;MSH6;MSH2;BRCA1;TAF9;TRRAP;SUPT3H |
| ING2 complex | SIN3A;ARID4A;S AP130 | BRMS1;HDAC1;HDAC2;ING2;RBBP4;RBBP7;SUDS3;B RMS1L;SAP30 |
| Kinase maturation complex 1 | MAP2K5;YWHA E;YWHAZ | YWHAQ;CDC37;MARK2;HSPA4;HSP90AA1;HSP90AB 1;MAP3K3;PFDN2;YWHAB;YWHAG;YWHAH;PDRG1; TRAF7 |
| LARC complex (LCR-associated remodeling complex) | MBD3;SMARCB 1;SMARCC1;ARI D1A;MTA2 | CHD4;HDAC1;HDAC2;HNRNPC;GATAD2B;RBBP4;DP F2;ACTB;SMARCA4;SMARCC2;SMARCD2;SMARCE1; ACTL6A;MBD2 |
| LSD1 complex | HMG20B;HMG2 0A;CTBP1 | PHF21B;KDM1A:RCOR1;HDAC1;HSPA1A;HSPA1B;PH F21A;RCOR3;RREB1;ZMYM2;ZNF217 |
| MTA2 complex | SIN3A;MBD3;M TA2 | CHD4;HDAC1;HDAC2;RBBP4;RBBP7 |
| NUMAC complex (nucleosomal methylation activator complex) | SMARCB1;SMA RCC1;ARID1A | CARM1;SCYL1;ACTB;SMARCA4;SMARCC2;SMARCD1 ;SMARCE1 |
| PCAF complex | TADA3;TAF6L;T AFSL;TAF10 | TADA2A;TAF9;TAF12;TRRAP;SUPT3H;KAT2B |
| RNA polymerase II complex, chromatin structure modifying | CDK8;SMARCB1 ;SMARCC1 | DRAP1;CREBBP;ERCC3;GTF2B;GTF2E1;GTF2F1;GTF2 H1;GTF2H3;POLR2A;PCSK4;SMARCA2;SMARCA4;SM ARCC2;SMARCD1;SMARCE1;TBP;ACTL6A;KAT2B;CC NC;MED21 |

(continued)

| ComplexName | hit_members | other_members |
|---|---|---|
| RNA polymerase II complex, incomplete (CDK8 complex), chromatin structure modifying | CDK8;SMARCB1 ;SMARCC1 | GTF2F1;SMARCC2;CCNC;CCNH;MED21 |
| SAGA complex, GCNS-linked | TADA3;TAF6L;T AF5L;ATXN7L3; TAF10 | ADA;SGF29;ATXN7L2;ATXN7L1;USP22;KAT2A;TAF9B ;SUPT20H;TAF9;TAF12;TRRAP;SUPT3H;TADA2B;SUP T7L |
| SIN3-ING1b complex II | SIN3A;SMARCB 1;SMARCC1;ARI D1A | SAP18;HDAC1;HDAC2;ING1;ARID4B;RBBP4;RBBP7;S MARCA4;SMARCC2;SMARCD1;ACTL6A;SAP30 |
| STAGA complex | TADA3;TAF6L;T ADA1;TAF5L;TA F10 | SF3B3;KAT2A;ATXN7;TAF9;TAF12;TRRAP;SUPT3H;S UPT7L |
| STAGA complex, SPT3-linked | TADA3;TAF6L;T ADA1;TAF5L;AT XN7L3;TAF10;S AP130 | SGF29;USP22;KAT2A;SUPT20H;ENY2;ATXN7;TAF9;T AF12;TRRAP;SUPT3H;TADA2B;SUPT7L |
| SWI-SNF chromatin remodeling-related-BRCA1 complex | SMARCB1;SMA RCC1;ARID1A | SMARCA2;SMARCA4;SMARCC2;SMARCD2;SMARCE 1;BRCA1;ACTL6A |
| TFTC complex (TATA-binding protein-free TAF-II-containing complex) | TADA3;TAF6L;T AF5L;TAF10 | SF3B3;KAT2A;ATXN7;TAF2;TAF4;TAFS;TAF6;TAF7;T AF9;TAF12;TAF13;TRRAP;SUPT3H |
| USP22-SAGA complex | TADA3;ATXN7L 3;TAF10 | USP22;KAT2A;TAF9B;TRRAP;TADA2B |
| WINAC complex | SMARCB1;SMA RCC1;ARID1A | CHAF1A;SUPT16H;SMARCA2;SMARCA4;SMARCC2;S MARCD1;SMARCE1;TOP2B;VDR;ACTL6A;BAZ1B |
| p300-CBP-p270-SWI/SNF complex | SMARCB1;SMA RCC1;ARID1A | CREBBP;EP300;SMARCA4;SMARCC2 |

Example 4

SPOP AND CUL3 GENETIC VALIDATION IN PRIMARY CD34+ CELLS

SPOP and CUL3 were identified using pooled CRISPR screening in the HUDEP2 model as regulators of fetal hemoglobin expression. To further investigate the role of SPOP and CUL3 in fetal hemoglobin regulation, primary CD34+ cells from a healthy donor were used with CRISPR Cas9- and shRNA-mediated genetic perturbation approaches. The impact on HbF levels was studied in differentiated CD34+ cells using HbF immunocytochemistry (ICC) (FIG. 8A).

HbF levels were determined by HbF ICC using CRISPR Cas9-RNP-based loss of function. Cas9-RNP complexes were electroporated into proliferating CD34+ cells. Cells were then differentiated for 7 days down the erythroid lineage and HbF levels were quantified using HbF ICC. Non-target guide RNAs were used as negative controls and guide RNAs targeting BCL11A were used as positive controls in this experimental design. Genetically perturbing SPOP and CUL3 using either CRISPR-Cas9 or shRNA led to elevated HbF levels, as measured by percent F cells within the population of differentiated erythroid cells or mean HbF levels per cell. The gRNAs used for SPOP were TAACTTTAGCTTTTGCCGGG (SEQ ID NO: 91), CGGGCATATAGGTTTGUGCA (SEQ ID NO: 92). GTTTGCGAGTAAACCCCAAA (SEQ ID NO: 93) and the gRNAs used for CUL3 were GAGCATCTCAAACACAACGA (SEQ ID NO: 94), CGAGATCAAGTTGTACGTTA (SEQ ID NO: 95), TCATCTACGGCAAACTCTAT (SEQ ID NO: 96) using the CRISPR Cas9-RNA method via electroporation. The Cas9-gRNA complexes were made independently and the three complexes per target were pooled for the cellular assay. The shRNAs used for SPOP were CCGGCACAGATCAAGGTAGTGAA ATCTCGAGATTTCACTACCTTGATCTGTGTTT TTTG (SPOP shRNA #2) (SEQ ID NO: 97), CCGGCAAGGTAGTGA AATTCTCCTACTCGAGTAGGAGAATTTCACTACCTTGTTT TTTG (SPOP shRNA #4) (SEQ ID NO: 98), CCGGCAG ATGAGTTAGGAGGACTGTCTCGAGACAGTCCTCCTAACTCATCTGTTT TTTG (SPOP shRNA #1) (SEQ ID NO: 99), and CCGGCACAAGGCTATCTTAGCAGCTCTCGAGAGCTGCTAAGATAGCCTTGTGTTT TITG (SPOP shRNA #3) (SEQ ID NO: 100). The shRNAs used for CUL3 were CCGGGACTATATCCAGGGCTTATTGCTCGAGCAATAAGCCC TGGATATAGTCTTT TTG (CUL3 shRNA #1) (SEQ ID NO: 101). CCGGCGTAAGAATAACAGTGGTCTTCTCGAGAA GACCACTGTTATTCTTACGTTT TTG (CUL3 shRNA #3) (SEQ ID NO: 102), and CCGGCGTGTGCCAAATGGTTTG AAACTCGAGTTTCAAACCATTTGGCACACGTTT TTG (CUL3 shRNA #2) (SEQ ID NO: 103). HbF ICC allows for the quantification of percent F cell and HbF intensity on a per-cell basis. An F cell is an erythroid cell that has a detectable level of HbF beyond a defined threshold and the percent F cells is defined as the percent of cells among a population of cells that are defined as F cells. The percent F cells and mean HbF intensity cells were quantified for negative control, sgBCL11A, sgSPOP and sgCUL3. HbF levels determined by HbF ICC using shRNA-based loss of function. shRNA vectors were electroporated into proliferating CD34+ cells. Cells were then differentiated for 7 days down the erythroid lineage and HbF levels were quantified using ICC. The percent F cells (*FIG. 8B and FIG. 8D*) and mean HbF intensity (*FIG. 8C and FIG. 8E*) were quantified for individual shRNA constructs for negative control, shBCL11A, shSPOP and shCUL3.

Methods

*Cell Culture*

Human Mobilized Peripheral Blood Primary CD34+ cells were expanded from thaw by seeding 100,000 viable cells/mL in a culture flask containing CD34+ Phase 1 Media comprised of IMDM, 100 ng/mL hSCF. 5 ng/mL IL-3. 3 IU/mL EPO, 250 ug/mL transferrin, 2.5% normal human serum, 1% pen/strep, 10 ng/mL heparin, 10 ug/mL insulin. The cells were supplemented by adding an additional 1X culture volume of CD34+ Phase 1 Media on Day 3 after thaw. After 5 days of expansion. Primary CD34+ cells were transfected with RNP complex.

*Ccrs9-gRNA RNP Preparation and nucleofection*

TE buffer was used to resuspend lyophilized crRNA and tracrRNA. The crRNA and tracrRNA were added to annealing buffer and annealed in thermocycler. Multiple sgrRNAs per gene were pooled into a microcentrifuge tube. Each sgRNA was mixed with TrueCut Cas9 v2 and incubated for 10 minutes to generate RNP complex. After counting, 144,000 CD34+ cells were added to the transfection cuvette and combined with transfection solution (P3, RNP complex, glycerol). The cells were transfected using an Amaxa Nucleofector and then transferred to a 12-well plate with 1mL of prewarmed Phase 1 media.

*In Vitro differentiation*

The day after transfection, the cells are supplemented with an additional 0.5 mL of Phase 1 media. On the 5th day post transfection the cells were differentiated towards erythroid lineage by complete medium exchange into CD34+ Phase 2 Media comprised of IMDM, 100 ng/mL hSCF. 5 ng/mL IL-3, 3 IU/mL EPO, 250 ug/mL transferrin, 2.5% normal human serum, 1% pen/strep, 10 ng/mL heparin, 10 ug/mL insulin. Two days after changing to Phase 2 media the cells were centrifuged, and 1 mL of Phase 2 media exchanged with fresh Phase 2 media. After another 2 days, the cells were harvested for HbF analysis by ICC.

*HbF ICC Protocol*

To collect the CD34+ cells, 40 uL from each well were transferred to a 384-well plate in duplicate and the plate was centrifuged. First the plate was washed with 25 μL of PBS. Then the plate was fixed with 25 μL of 4% paraformaldehyde for 10 minutes at room temperature. The cells were then washed three times with 25 μL of PBS. Next the cells were permeabilized and blocked for 1 hour at room temperature in 25 μL of Penn/Block buffer comprised of 1X PBS, 1% bovine serum albumin, 10% fetal bovine serum, 0.3M glycine, and 0.1% tween-20. Then the cells were washed three times with 25 μL of 0.1% tween in PBS. After washing, the cells were incubated overnight at 4°C with 25 μL of HbF-488 Primary Antibody (ThennoFisher MHFH01-4) diluted 1:40 in 0.1% tween and Hoescht diluted 1:2000 in 0.1% tween. The next day the cells were again washed three times with 25 μL of 0.1% tween in PBS and foil sealed for imaging on the ThermoFisher Cellinsight CX7.

The plates were then scanned on the CX7 at 10x magnification, and 9 images were acquired per well. The software algorithm then identified nuclei and calculated a total nuclei count using the Hoechst staining on channel 1. After nuclei were identified, the algorithm calculated the average nuclear intensity of the HbF staining on channel 2.

**REFERENCES**

An international effort to cure a global health problem: A report on the 19th

Hemoglobin Switching Conference.
Blobel GA, Bodine D, Brand M, Crispino J, de Bruijn MF, Nathan D, Papayannopoulou T,
Porcher C, Strouboulis J. Zon L. Higgs DR. Stamatoyannopoulos G, Engel JD.
Exp Hematol. 2015 Oct;43(10):821-37. doi: 10.1016/j.exphem.2015.06.008. Epub 2015 Jul
2. Review. PMID:26143582
Control of globin gene expression during development and erythroid differentiation.
Stamatoyannopoulos G.
Exp Hematol. 2(X)5 Mar:33(3):259-7t. Review. PMID: 15730849
Fetal haemoglobin induction in sickle cell disease.
Paikari A, Sheehan VA.
Br J Haematol. 2018 Jan:180(2):189-200. doi: 10.1111/bjh.15021. Epub 2017 Nov
16. Review. PMID: 29143315
Fetal haemoglobin in sickle-cell disease: from genetic epidemiology to new therapeutic strategies.
Lettre G, Bauer DE.
Lancet. 2016 Jun 18;387(10037):2554-64. doi: 10.1016/S0140-6736(15)01341-0. Review. PMID: 27353686
Locus control regions.
Li Q. Peterson KR. Fang X, Stamatoyannopoulos G.
Blood. 2002 Nov 1;100(9):3077-86. Review. PMID: 12384402
Pomalidomide and lenalidomide regulate erythropoiesis and fetal hemoglobin production in human CD34+ cells.
Moutouh-de Parseval LA, Verhelle D, Glezer E, Jensen-Pergakes K, Ferguson GD, Corral
LG. Morris CL. Muller G. Brady H, Chan K.
J Clin Invest. 2008 Jan;118(1):248-58. PMID: 18064299
Augmentation of fetal-hemoglobin production in anemic monkeys by hydroxyurea.
Letvin NL, Linch DC, Beardsley GP. McIntyre KW. Nathan DG.
N Engl J Med. 1984 Apr 5;310(14):869-73. PMID: 619967
EHMT1 and EHMT2 inhibition induces fetal hemoglobin expression.
Rennevillc A, Van Galen P, Canver MC, McConkey M. Krill-Burger JM. Dorfman DM, Holson EB, Bernstein BE, Orkin SH, Bauer DE, Ebert BL.
Blood. 2015 Oct 15;126(16):1930-9. doi: 10.1182/blood-2015-06-649087. Epub 2015 Aug 28. PMID: 26320100
Lvsine-specific demethylase 1 is a therapeutic target for fetal hemoglobin induction.
Shi L. Cui S, Engel JD, Tanabe O.
Nat Med. 2013 Mar;19(3):291-4. doi: 10.1038/nm.3101. Epub 2013 Feb 17. PMID: 23416702

CORUM: the comprehensive resource of mammalian protein complexes-2009.
Giurgiu M. Reinhard J, Brauner B. Dunger-Kaltenbach 1, Fobo G, Frishman G, Montrone C. Ruepp A
Nucleic Acids Res. 2010 Jan;38(Database issue):D497-501. doi: 10.1093/nar/gkp914. Epub 2009 Nov 1. Pmid: 19884131
Densely interconnected transcriptional circuits control cell states in human hematopoiesis
Novershtern N, Subramanian A. Lawton L.N. Raymond H. M, Haining N, McConkey M. E, Habib N, Yosef N, Chang C. Y, Shay T, Frampton C. M, Drake A. C. B, Leskov I, Nilsson B. Proffer F. Dombkowski D, Evans J. W. Liefeld R, Smutko J. S, Chen J, Friedman N, Young R. A. Golub T. R. Regev A. Ebert B. L.
Cell. 2011 Jan 21;144(2):296-309. doi: 10.1016/j.cell.2011.01.004. PMID: 21241896
The Genotype-Tissue Expression (GTEx) project
The GTEx Consortium
Nat Genet. 2013 Jun;45(6):580-5. doi: 10.1038/ng.2653. PMID: 23715323

All publications and patent applications described herein are hereby incorporated by reference in their entireties.

While the present invention has been described in conjunction with the specific embodiments set forth above, many alternatives, modifications and other variations thereof will be apparent to those of ordinary skill in the art. All such alternatives, modifications and variations are intended to fall within the spirit and scope of the present invention.

The following section comprises numbered clauses which are not claims, but are additional statements of the invention.

1. A method for increasing expression of a fetal hemoglobin (HbF) in a cell, optionally a eukaryotic cell, comprising contacting a cell with an inhibitor of a target protein or target protein complex that functions to regulate HbF expression, optionally wherein the target protein is Cullin 3 (CUL3) or Speckle-type POZ protein (SPOP).

2. The method of clause 1, wherein the target protein is CUL3.

3. The method of clause 1 wherein the target protein is SPOP.

4. The method of any one of clauses 1-3, wherein the HbF comprises hemoglobin gamma and hemoglobin alpha.

5. The method of clause 4, wherein the hemoglobin gamma comprises hemoglobin gamma G1 (HBG1) and/or or hemoglobin gamma G2 (HBG2).

6. The method of any one of clauses 1-5, wherein the target protein or protein complex regulates HbF expression via a molecular signaling pathway listed in Table 5.

7. The method of clause 6, wherein the molecular signaling pathway is selected from the group consisting of glucagon signaling pathway, carbon metabolism, oxytocin signaling, glycolysis, gluconeogenesis, endocrine resistance, Gonadotropin-releasing hormone (GnRH) signaling, oocyte meiosis, fatty acid degradation, and inflammatory mediator regulation of Transient Receptor Potential (TRP) channels.

8. The method of any one of clauses 1-7, wherein the target protein is selected from those listed in Table 1 or Table 2.

9. The method of any one of clauses 1-8, wherein the target protein is permanently or transiently associated with a multi-protein complex that regulates HbF expression.

10. The method of clause 9, wherein the multi-protein complex is selected from those listed in Table 3 or Table 4.

11. The method of clause 9 or clause 10, wherein CUL3 is permanently or transiently associated with the multi-protein complex.

12. The method of clause 11, wherein the multi-protein complex is selected from D(4) dopamine receptor (DRD4)-Kelch like protein 12 (KLH12)-CUL3, ubiquitin E3 ligase. coiled coil domain containing protein 22 (CCDC22)-COMM domain containing protein 8 (COMMD8)-CUL3, or Cullin associated NEDD8 dissociated protein (CAND1)-CUL3- E3 ubiquitin protein ligase RBX1 (RBX).

13. The method of clause 9 or clause 10, wherein SPOP is permanently or transiently associated with the multi-protein complex.

14. The method of clause 13, wherein the multi-protein complex is a ubiquitin E3 ligase complex.

15. The method of any one of clauses 1-14, wherein the inhibitor target or binds a nucleotide sequence encoding the target protein or a protein in the protein complex, thereby inhibiting or preventing the expression of the target protein or a protein in the protein complex.

16. The method of clause 15, wherein the nucleotide sequence encoding the target protein or the protein in the protein complex is DNA.

17. The method of clause 15, wherein the nucleotide sequence encoding the target protein or the protein in the protein complex is RNA.

18. The method of clause 17, wherein the nucleotide sequence encodes CUL3, and optionally comprises or consists of a nucleic acid encoding the amino acid sequence of SEQ ID NO: 108 or an antisense sequence thereof.

19. The method of clause 17, wherein the nucleotide sequence encodes SPOP, and optionally comprises or consists of a nucleic acid encoding the amino acid sequence of SEQ ID NO: 109 or an antisense sequence thereof.

20. The method of any one of clauses 1-19, wherein the inhibitor is selected from the group consisting of a small molecule, a nucleic acid, a polypeptide, and a nucleoprotein complex.

21. The method of clause 20, wherein the nucleic acid is selected from the group consisting of DNA, RNA, shRNA, siRNA, microRNA, gRNA. and antisense oligonucleotide.

22. The method of clause 20, wherein the polypeptide is selected from the group consisting of: a protein, a peptide, a protein mimetic, a peptidomimetic, an antibody or functional fragment thereof, and an antibody-drug conjugate or a functional fragment thereof.

23. The method of clause 20, wherein the nucleoprotein complex is ribonucleoprotein complex (RNP) comprising:

a) a first sequence comprising a guide RNA (gRNA) that specifically binds a target sequence, wherein the target sequence comprises a regulator of HbF expression and
b) a second sequence encoding a CRISPR-Cas protein

wherein the CRISPR-Cas protein comprises a DNA-nuclease activity.

24. The method of any one of clauses 1-23, wherein the cell is a blood cell.

25. The method of clause 24, wherein the blood cell is an erythrocyte.

26. The methods of any one of clauses 1-25, wherein the contacting a cell occurs in vitro, in vivo, ex vivo, or in situ.

27. A pharmaceutical composition for increasing expression of fetal hemoglobin (HbF) in a subject in need thereof, comprising:

an inhibitor of a target protein or protein complex that functions to regulate HbF expression, and
a diluent, excipient, and carrier

wherein the composition is formulated for delivery to a subject in need thereof.

28. The pharmaceutical composition of clause 27, wherein the inhibitor is small molecule.

29. The pharmaceutical composition of clause 28, wherein the small molecular inhibitor targets CUL3.

30. The pharmaceutical composition of clause 29, wherein the CUL3 small molecule inhibitor is selected from the group consisting of: MLN4924, suramin, and DI-591.

31. The pharmaceutical composition of clause 27, wherein the inhibitor is a nucleic acid.

32. The pharmaceutical composition of clause 31, wherein the nucleic acid is selected from DNA, RNA, shRNA, siRNA, microRNA, gRNA, and antisense oligonucleotide.

33. The pharmaceutical composition of clause 27, wherein the inhibitor is a polypeptide.

34. The pharmaceutical composition of clause 33, wherein the polypeptide is selected from a protein, a peptide, a protein mimetic, a peptidomimetic, an antibody or functional fragment thereof, and an antibody-drug conjugate or a functional fragment thereof.

35. The pharmaceutical composition of any one of clauses 33-34, wherein the polypeptide specifically binds a regulator of HbF expression.

36. The pharmaceutical composition of clause 27, wherein the inhibitor is a ribonucleoprotein (RNP) complex comprising:

a) a first sequence comprising a guide RNA (gRNA) that specifically binds a target sequence, wherein the target sequence comprises a regulator of HbF expression and
b) a second sequence encoding a CRISPR-Cas protein

wherein the CRISPR-Cas protein comprises a DNA-nuclease activity.

37. The pharmaceutical composition of clause 36, wherein the gRNA binds a gene encoding the regulator of HbF expression.

38. The pharmaceutical composition of clause 36, wherein the target sequence is listed in any one of Tables 1, 3-4, and 6-7.

39. The pharmaceutical composition of clause 38, wherein the target sequence is CUL3.

40. The pharmaceutical composition of clause 38, wherein the target sequence is SPOP.

41. The pharmaceutical composition of clause 37, wherein the gRNA comprises any one of the sequences disclosed in Table 2 or a fragment thereof, or an antisense sequence of any of the foregoing.

42. The pharmaceutical composition of clause 41, wherein the gRNA binds a gene encoding CUL3, and optionally comprises or consists of GAGCATCTCAAACACAACGA (SEQ ID NO: 94). CGAGATCAAGTTGTACGTTA (SEQ ID NO: 95), or TCATCTACGGCAAACTCTAT (SEQ ID NO: 96).

43. The pharmaceutical composition of clause 41, wherein the gRNA binds a gene encoding SPOP, and optionally comprises or consists of TAACTTTAGCTTTTGCCGGG (SEQ ID NO: 91), CGGGCATATAGGTTTGTGCA (SEQ ID NO: 92), or GTTTGCGAGTAAACCCCAAA (SEQ ID NO: 93).

44. The pharmaceutical composition of clause 36 or clause 37, wherein the first sequence comprising the gRNA comprises a sequence encoding a promoter capable of expressing the gRNA in a eukaryotic cell.

45. The pharmaceutical composition of clause 36 or clause 37, wherein the second sequence comprising the CRISPR-Cas protein comprises a sequence capable of expressing the CRISPR-Cas protein in a eukaryotic cell.

46. The method of any of clauses 1-26 or the pharmaceutical composition of clause 44 or clause 45, wherein the eukaryotic cell is a mammalian cell.

47. The method of any of clauses 1-26 or the pharmaceutical composition of any one of clauses 44-46, wherein the eukaryotic cell is a blood cell.

48. The method of any of clauses 1-26 or the pharmaceutical composition of any one of clauses 44-46, wherein the eukaryotic cell is an erythrocyte.

49. The method of any one of clauses 1-26, wherein the inhibitor is delivered via a vector.

50. The method of clause 49, wherein the vector is a viral vector.

51. The method of clause 50, wherein the viral vector comprises a sequence isolated or derived from an adeno-associated virus (AAV).

52. A method of treating a disease or disorder associated with a defect in a hemoglobin protein activity or expression, comprising providing to a subject in need thereof the composition of any one of clauses 27-51.

53. The method of clause 52, wherein the disease or disorder is a blood disorder.

54. The method of clause 53, wherein the blood disorder is selected from a group consisting of: Sickle cell disease, β-thatassemia, β-thalessemia intermedia, β-thalessemia major, β-thalessemia minor, and Cooley's anemia.

55. The method of any one of clauses 52-54, wherein the hemoglobin protein is selected from hemoglobin-alpha and hemoglobin-beta.

56. The method of any one of clauses 52-55, wherein the defect in the hemoglobin protein activity or expression results from a mutation, substitution, deletion, insertion, frameshift, inversion, or transposition to a nucleotide sequence which encodes the hemoglobin protein.

## Claims

**1.** An *in vitro* or *ex vivo* method for increasing expression of a fetal hemoglobin (HbF) in a cell comprising contacting a cell with an inhibitor of a target protein or target protein complex that functions to regulate HbF expression.

**2.** The method of claim 1, wherein the target protein is selected from those listed in Table 1, for example, wherein the target protein is CUL3 or SPOP.

**3.** The method of claim 1 or 2, wherein the HbF comprises hemoglobin gamma and hemoglobin alpha; more preferably wherein the hemoglobin gamma comprises hemoglobin gamma G1 (HBG1) and/or hemoglobin gamma G2 (HBG2).

**4.** The method of any of claims 1 to 3, wherein the target protein or protein complex regulates HbF expression via a molecular signalling pathway listed in Table 5; preferably wherein the molecular signalling pathway is selected from the group consisting of glucagon signalling pathway, carbon metabolism, oxytocin signalling, glycolysis, gluconeogenesis, endocrine resistance, Gonadotropin-releasing hormone (GnRH) signalling, oocyte meiosis, fatty acid degradation, and inflammatory mediator regulation of Transient Receptor Potential (TRP) channels.

**5.** The method of any of claims 1 to 4, wherein the target protein is permanently or transiently associated with a multi-protein complex that regulates HbF expression; preferably (a) wherein the multi-protein complex is selected from those listed in Table 3 or Table 4; and/or (b) wherein the target protein is permanently or transiently associated with the multi-protein complex; optionally wherein the multi-protein complex is selected from D(4) dopamine receptor (DRD4)-Kelch like protein 12 (KLH12)-CUL3, ubiquitin E3 ligase, coiled coil domain containing protein 22 (CCDC22)-COMM domain containing protein 8 (COMMD8)-CUL3, or Cullin associated NEDD8 dissociated protein (CAND1)-CUL3- E3 ubiquitin protein ligase RBX1 (RBX).

**6.** The method of any preceding claim, wherein the inhibitor targets or binds a nucleotide sequence encoding the target protein or a protein in the protein complex, thereby inhibiting or preventing the expression of the target protein or a protein in the protein complex; preferably wherein

(a) the nucleotide sequence encoding the target protein or the protein in the protein complex is DNA; or
(b) the nucleotide sequence encoding the target protein or the protein in the protein complex is RNA: optionally (i) wherein the nucleotide sequence encodes a target protein, and optionally comprises or consists of a nucleic acid encoding the amino acid sequence of SEQ ID NO: 108 or an antisense sequence thereof.

**7.** The method of any preceding claim, wherein the inhibitor is selected from the group consisting of a small molecule, a nucleic acid, a polypeptide, and a nucleoprotein complex; preferably

(a) wherein the nucleic acid is selected from the group consisting of DNA, RNA, shRNA, siRNA, microRNA, gRNA, and antisense oligonucleotide; or
(b) wherein the polypeptide is selected from the group consisting of a protein, a peptide, a protein mimetic, a peptidomimetic, an antibody or functional fragment thereof, and an antibody-drug conjugate or a functional fragment thereof; or
(c) wherein the nucleoprotein complex is a ribonucleoprotein complex (RNP) comprising:

i) a first sequence comprising a guide RNA (gRNA) that specifically binds a target sequence, wherein the target sequence comprises a regulator of HbF expression and
ii) a second sequence encoding a CRISPR-Cas protein

wherein the CRISPR-Cas protein comprises a DNA-nuclease activity.

**8.** The method of any preceding claim, wherein the cell is a blood cell; preferably an erythrocyte.

**9.** A pharmaceutical composition for increasing expression of fetal hemoglobin (HbF) in a subject in need thereof, comprising:

an inhibitor of a target protein or protein complex that functions to regulate HbF expression, and
a diluent, excipient, and carrier

wherein the composition is formulated for delivery to a subject in need thereof.

**10.** The pharmaceutical composition of claim 9, wherein the target protein is selected from those listed in Table 1, for example, wherein the target protein is CUL3 or SPOP.

**11.** The pharmaceutical composition of claim 9 or 10, wherein the inhibitor is:

a) a small molecule; preferably wherein the small molecule inhibitor targets a target protein; more preferably wherein the small molecule inhibitor is selected from the group consisting of MLN4924, suramin, and DI-591; or
b) a nucleic acid; preferably wherein the nucleic acid is selected from DNA, RNA, shRNA, siRNA, microRNA, gRNA, and antisense oligonucleotide; or
c) a polypeptide; preferably wherein the polypeptide is selected from a protein, a peptide, a protein mimetic, a peptidomimetic, an antibody or functional fragment thereof, and an antibody-drug conjugate or a functional fragment thereof, and/or wherein the polypeptide specifically binds a regulator of HbF expression; or
d) a ribonucleoprotein (RNP) complex comprising:

(i) a first sequence comprising a guide RNA (gRNA) that specifically binds a target sequence, wherein the target sequence comprises a regulator of HbF expression and
(ii) a second sequence encoding a CRISPR-Cas protein wherein the CRISPR-Cas protein comprises a DNA-nuclease activity.

**12.** The pharmaceutical composition of claim 11(d), wherein the gRNA comprises any one of the sequences disclosed in Table 2 or a fragment thereof, or an antisense sequence of any of the foregoing; wherein the gRNA binds a gene encoding a target protein, and optionally comprises or consists of GAGCATCTCAAACACAACGA (SEQ ID NO: 94), CGAGATCAAGTTGTACGTTA (SEQ ID NO: 95), or TCATCTACGGCAAACTCTAT (SEQ ID NO: 96).

**13.** The pharmaceutical composition of claim 11(d) or claim 12, wherein (a) the first sequence comprising the gRNA comprises a sequence encoding a promoter capable of expressing the gRNA in a eukaryotic cell, optionally wherein the eukaryotic cell is a blood cell, e.g., an erythrocyte; or (b) wherein the second sequence comprising the CRISPR-Cas protein comprises a sequence capable of expressing the CRISPR-Cas protein in a eukaryotic cell, optionally wherein the eukaryotic cell is a blood cell, e.g., an erythrocyte.

**14.** The method of any of claims 1 to 8, wherein the inhibitor is delivered via a vector; preferably wherein the vector is a viral vector; more preferably wherein the viral vector comprises a sequence isolated or derived from an adeno-associated virus (AAV).

**15.** A composition of any of claims 9 to 13 for treating a disease or disorder associated with a defect in a hemoglobin protein activity or expression, wherein the hemoglobin protein is selected from hemoglobin-alpha and hemoglobm-beta; optionally wherein the defect in the hemoglobin protein activity or expression results from a mutation, substitution, deletion, insertion, frameshift, inversion, or transposition to a nucleotide sequence which encodes the hemoglobin protein, optionally wherein the disease or disorder is a blood disorder; optionally wherein the blood disorder is selected from a group consisting of Sickle cell disease, β-thatassemia, β-thalessemia intermedia, β-thalessemia major, β-thalessemia minor, and Cooley's anemia.

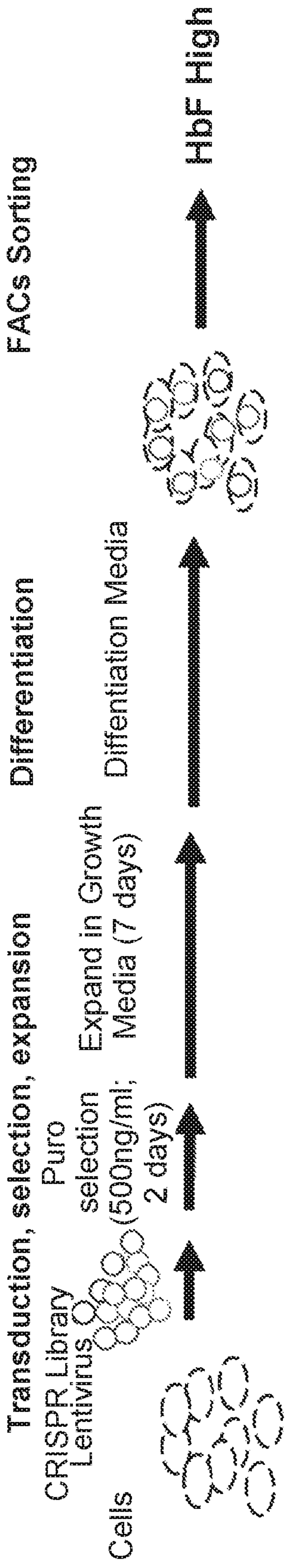
Transduction, selection, expansion
Differentiation
FACs Sorting
Cells
CRISPR Library Lentivirus
Puro selection (500ng/ml; 2 days)
Expand in Growth Media (7 days)
Diffentiation Media
HbF High

FIG. 1

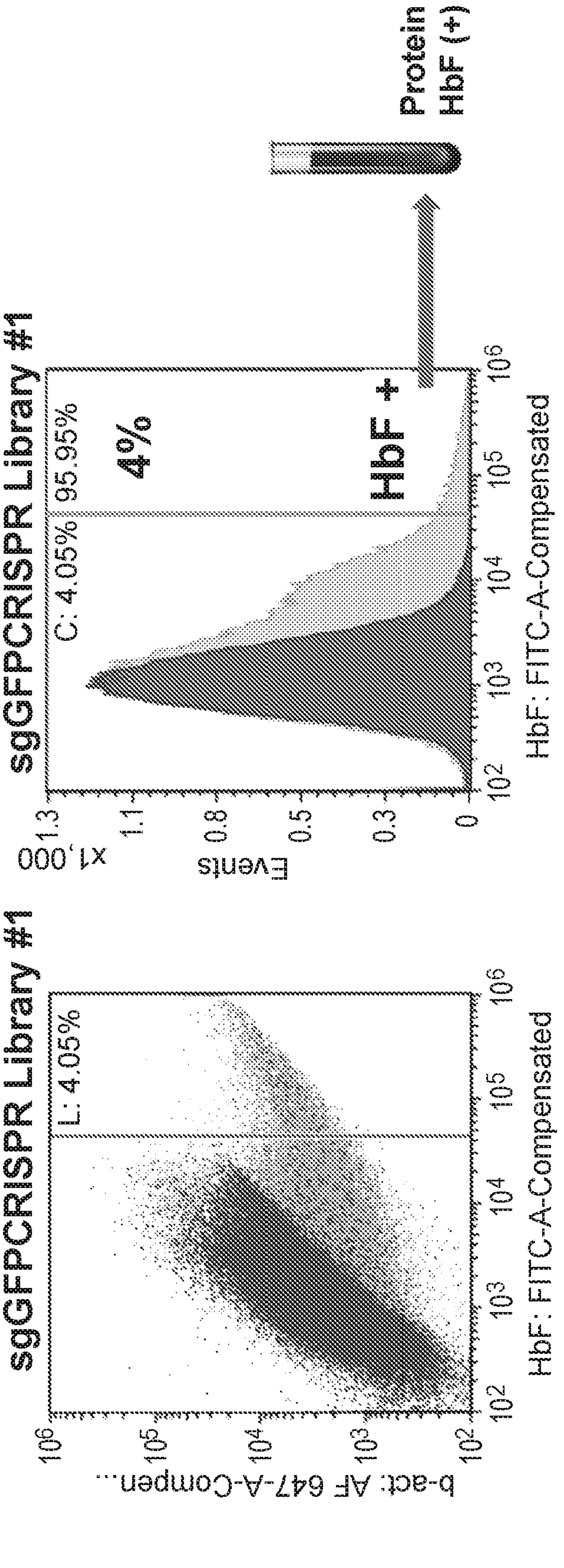
sgGFPCRISPR Library #1
L: 4.05%
b-act: AF 647-A-Compen...
HbF: FITC-A-Compensated
sgGFPCRISPR Library #1
C: 4.05%
95.95%
4%
HbF +
x1,000
Events
HbF: FITC-A-Compensated
Protein
HbF (+)

FIG. 2

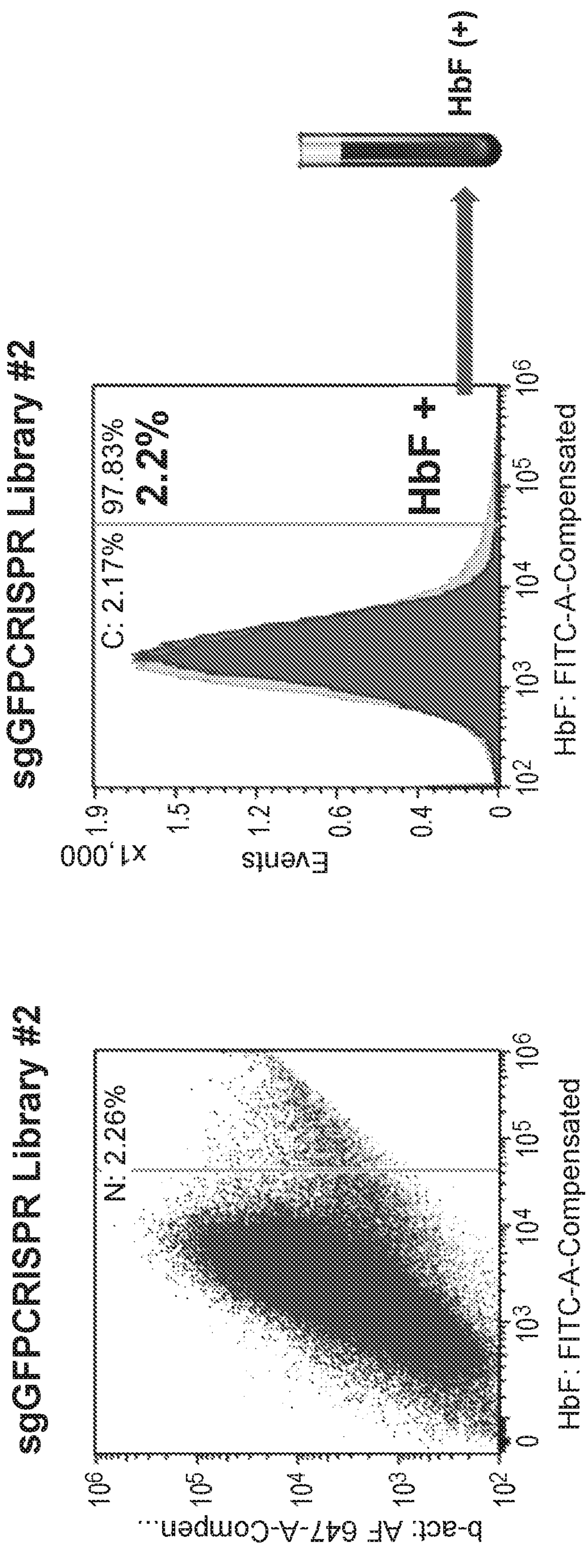
sgGFPCRISPR Library #2
N: 2.26%
b-act: AF 647-A-Compen...
HbF: FITC-A-Compensated
sgGFPCRISPR Library #2
C: 2.17%
97.83%
2.2%
HbF +
x1,000
Events
HbF: FITC-A-Compensated
HbF (+)

FIG. 3

| | | |
|---|---|---|
| Genome alignment (hg19)<br><br>Abundance estimation (RPM) | Hit calling<br><br>Zscore difference<br><br>Fold-change<br><br>no. of guides per gene<br><br>Concordance between Horizon and Desktop genetics | Protein complex membership<br><br>KEGG pathway enrichment<br><br>Whole blood expression specificity<br><br>Erythroid lineage expression specificity |

FIG. 4A

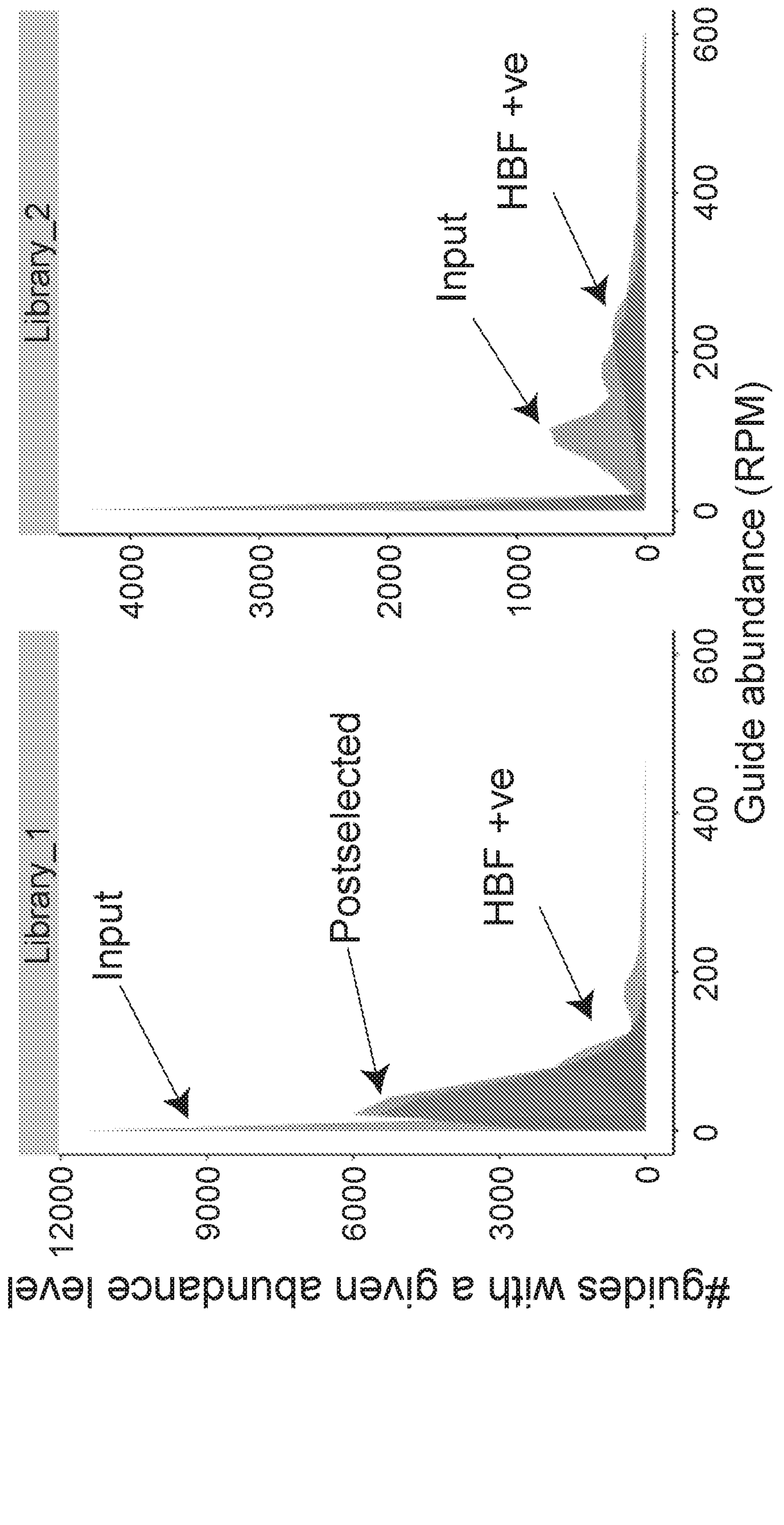
Library_1
Library_2
Input
Postselected
HBF +ve
Input
HBF +ve
12000
9000
6000
3000
0
4000
3000
2000
1000
0
0
200
400
600
0
200
400
600
Guide abundance (RPM)
#guides with a given abundance level

FIG. 4B

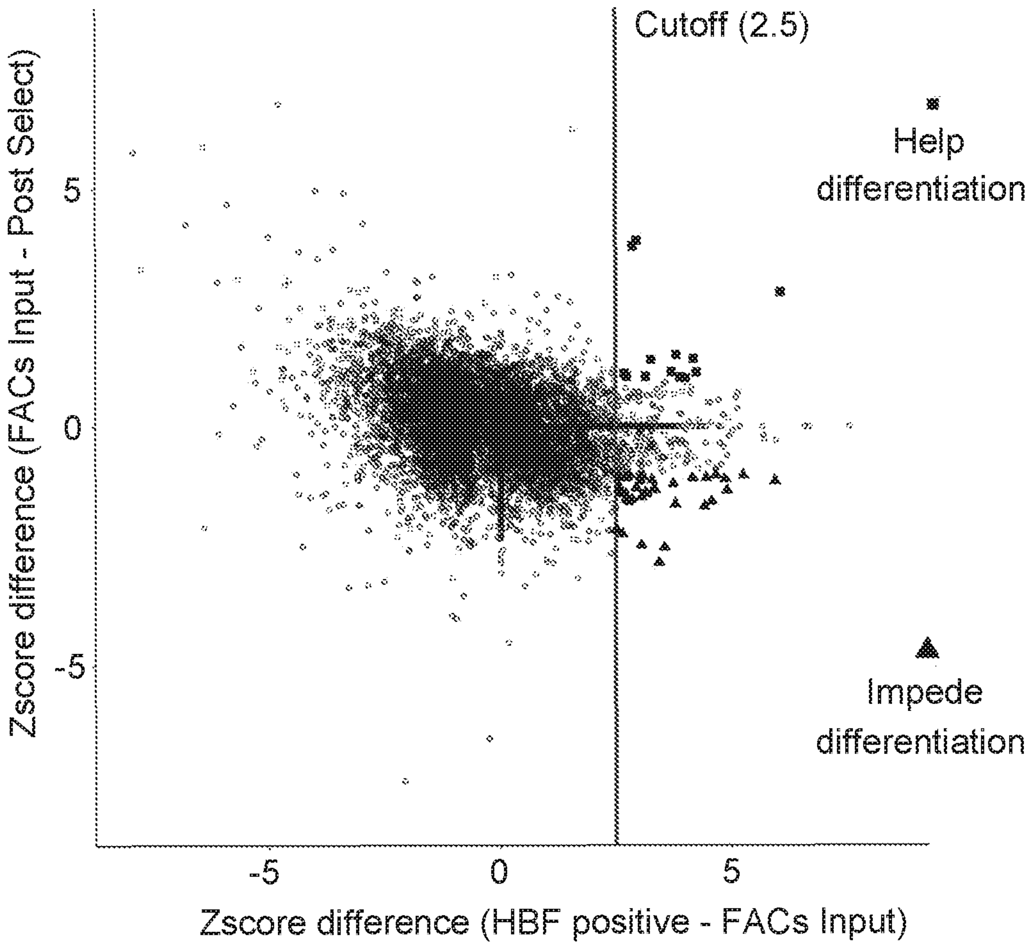

Cutoff (2.5)
Help
differentiation
Impede
differentiation
Zscore difference (FACs Input - Post Select)
5
0
-5
-5
0
5
Zscore difference (HBF positive - FACs Input)


FIG. 4C

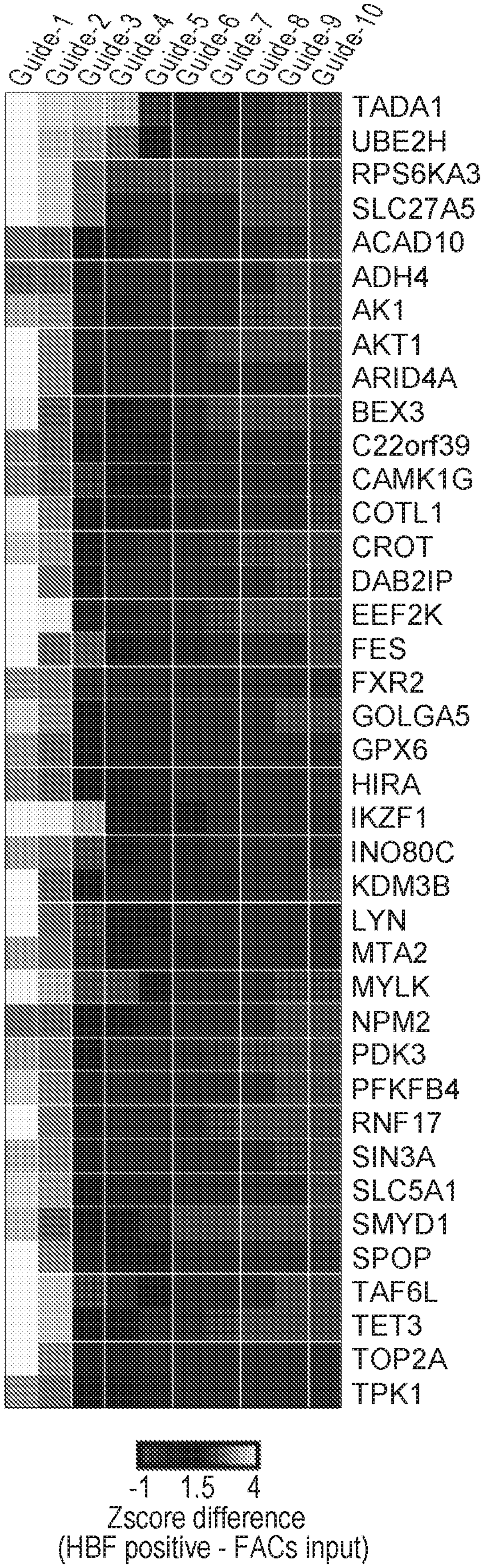
Guide-1
Guide-2
Guide-3
Guide-4
Guide-5
Guide-6
Guide-7
Guide-8
Guide-9
Guide-10
TADA1
UBE2H
RPS6KA3
SLC27A5
ACAD10
ADH4
AK1
AKT1
ARID4A
BEX3
C22orf39
CAMK1G
COTL1
CROT
DAB2IP
EEF2K
FES
FXR2
GOLGA5
GPX6
HIRA
IKZF1
INO80C
KDM3B
LYN
MTA2
MYLK
NPM2
PDK3
PFKFB4
RNF17
SIN3A
SLC5A1
SMYD1
SPOP
TAF6L
TET3
TOP2A
TPK1
-1 1.5 4
Zscore difference
(HBF positive - FACs input)

FIG. 5A

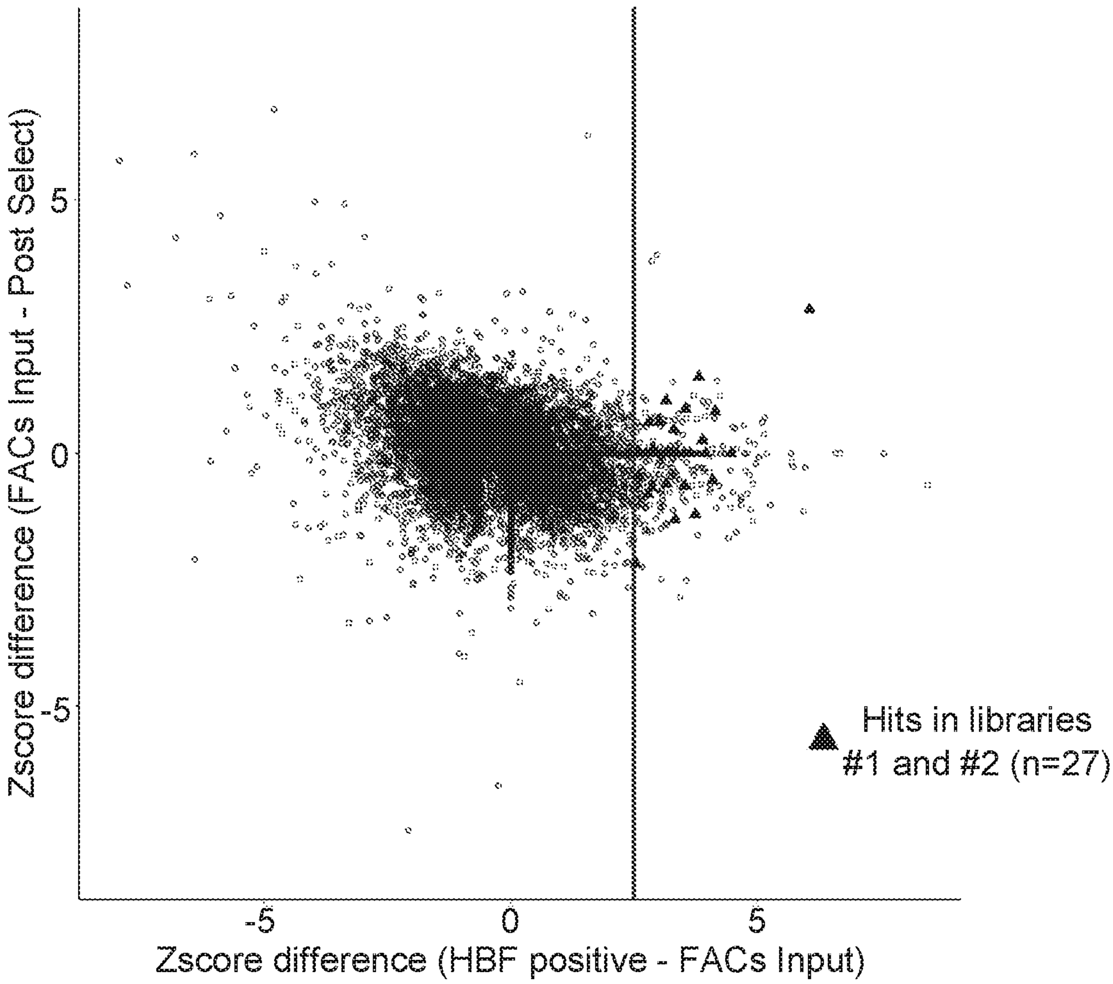
Zscore difference (FACs Input - Post Select)
5
0
-5
-5
0
5
Zscore difference (HBF positive - FACs Input)
Hits in libraries
#1 and #2 (n=27)

FIG. 5B

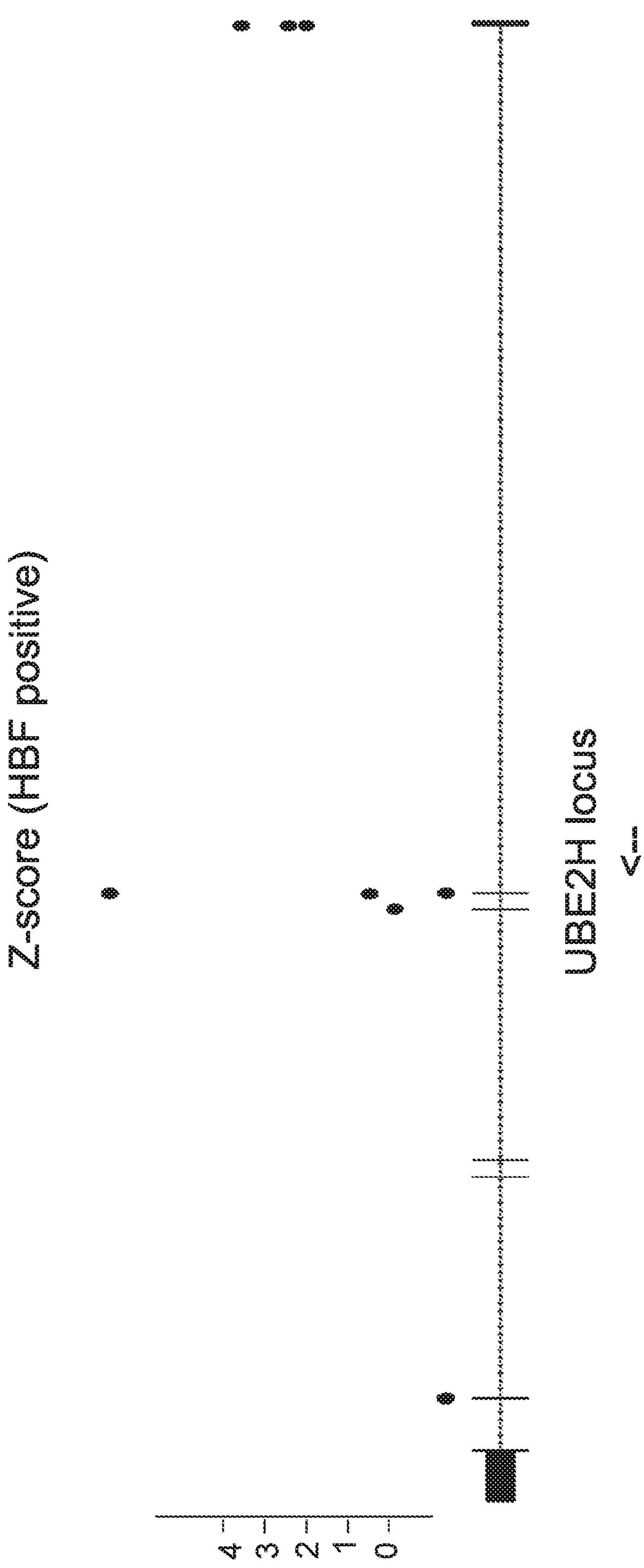


FIG. 5C

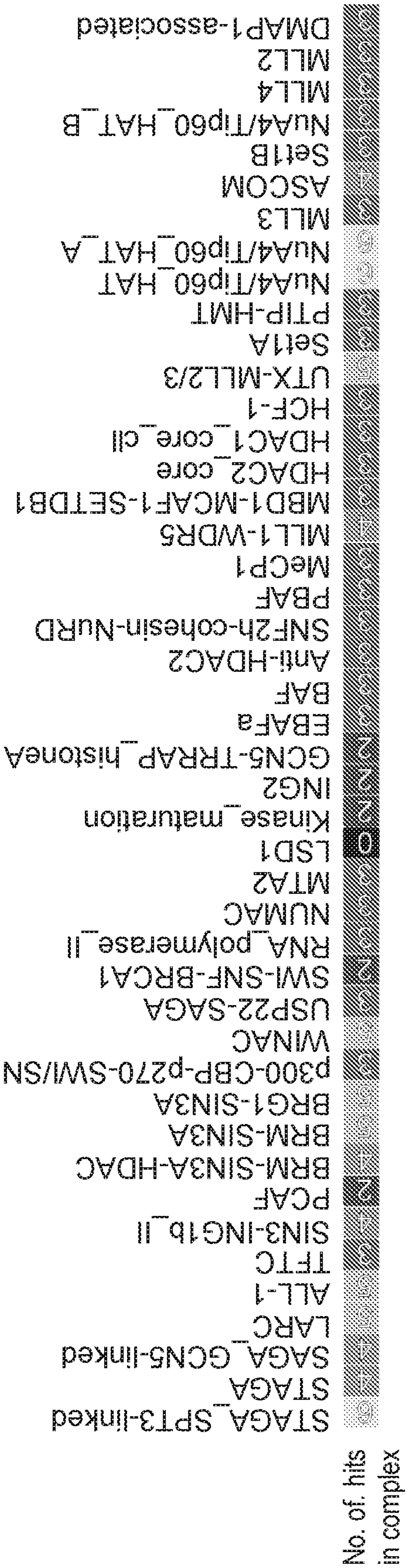
No. of hits in complex
STAGA_SPT3-linked
STAGA_
SAGA_GCN5-linked
LARC_
ALL-1
TFTC
SIN3-ING1b_II
PCAF
BRM-SIN3A-HDAC
BRM-SIN3A
BRG1-SIN3A
p300-CBP-p270-SWI/SN
WINAC
USP22-SAGA
SWI-SNF-BRCA1
RNA_polymerase_II
NUMAC
MTA2
LSD1
Kinase_maturation
ING2
GCN5-TRRAP_histoneA
EBAFa
BAF
Anti-HDAC2
SNF2h-cohesin-NuRD
PBAF
MeCP1
MLL1-WDR5
MBD1-MCAF1-SETDB1
HDAC2_core
HDAC1_core_cII
HCF-1
UTX-MLL2/3
Set1A
PTIP-HMT
NuA4/Tip60_HAT
NuA4/Tip60_HAT_A
MLL3
ASCOM
Set1B
NuA4/Tip60_HAT_B
MLL4
MLL2
DMAP1-associated

FIG. 6

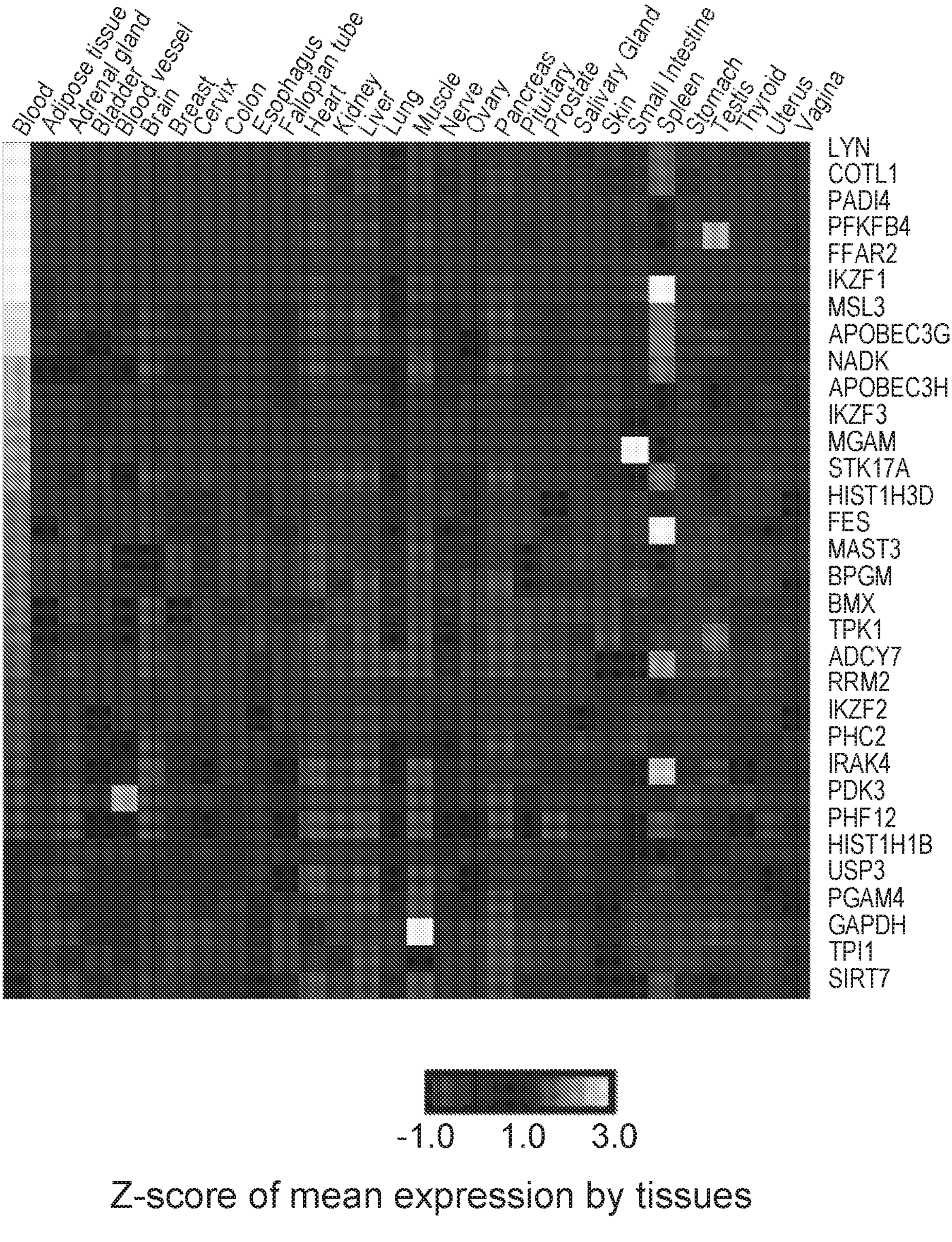
Blood
Adipose tissue
Adrenal gland
Bladder
Blood vessel
Brain
Breast
Cervix
Colon
Esophagus
Fallopian tube
Heart
Kidney
Liver
Lung
Muscle
Nerve
Ovary
Pancreas
Pituitary
Prostate
Salivary Gland
Skin
Small Intestine
Spleen
Stomach
Testis
Thyroid
Uterus
Vagina
LYN
COTL1
PADI4
PFKFB4
FFAR2
IKZF1
MSL3
APOBEC3G
NADK
APOBEC3H
IKZF3
MGAM
STK17A
HIST1H3D
FES
MAST3
BPGM
BMX
TPK1
ADCY7
RRM2
IKZF2
PHC2
IRAK4
PDK3
PHF12
HIST1H1B
USP3
PGAM4
GAPDH
TPI1
SIRT7
-1.0
1.0
3.0
Z-score of mean expression by tissues

FIG. 7A

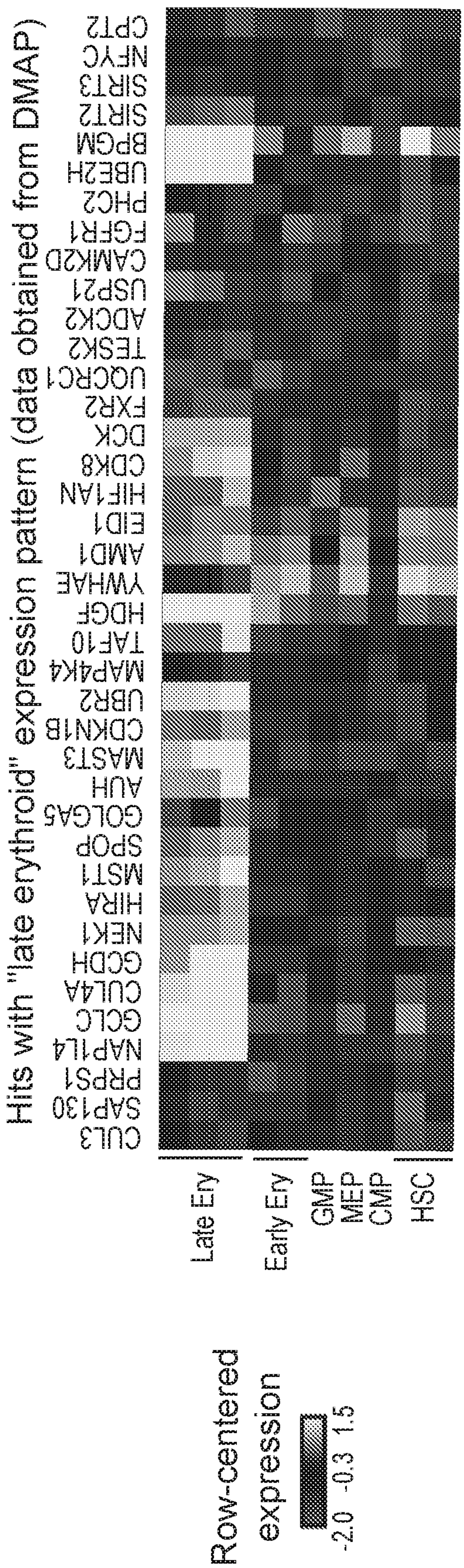
Hits with "late erythroid" expression pattern (data obtained from DMAP)
CUL3
SAP130
PRPS1
NAP1L4
GCLC
CUL4A
GCDH
NEK1
HIRA
MST1
SPOP
GOLGA5
AUH
MAST3
CDKN1B
UBR2
MAP4K4
TAF10
HDGF
YWHAE
AMD1
EID1
HIF1AN
CDK8
DCK
FXR2
UQCRC1
TESK2
ADCK2
USP21
CAMK2D
FGFR1
PHC2
UBE2H
BPGM
SIRT2
SIRT3
NFYC
CPT2
Late Ery
Early Ery
GMP
MEP
CMP
HSC
Row-centered expression
-2.0
-0.3
1.5

FIG. 7B

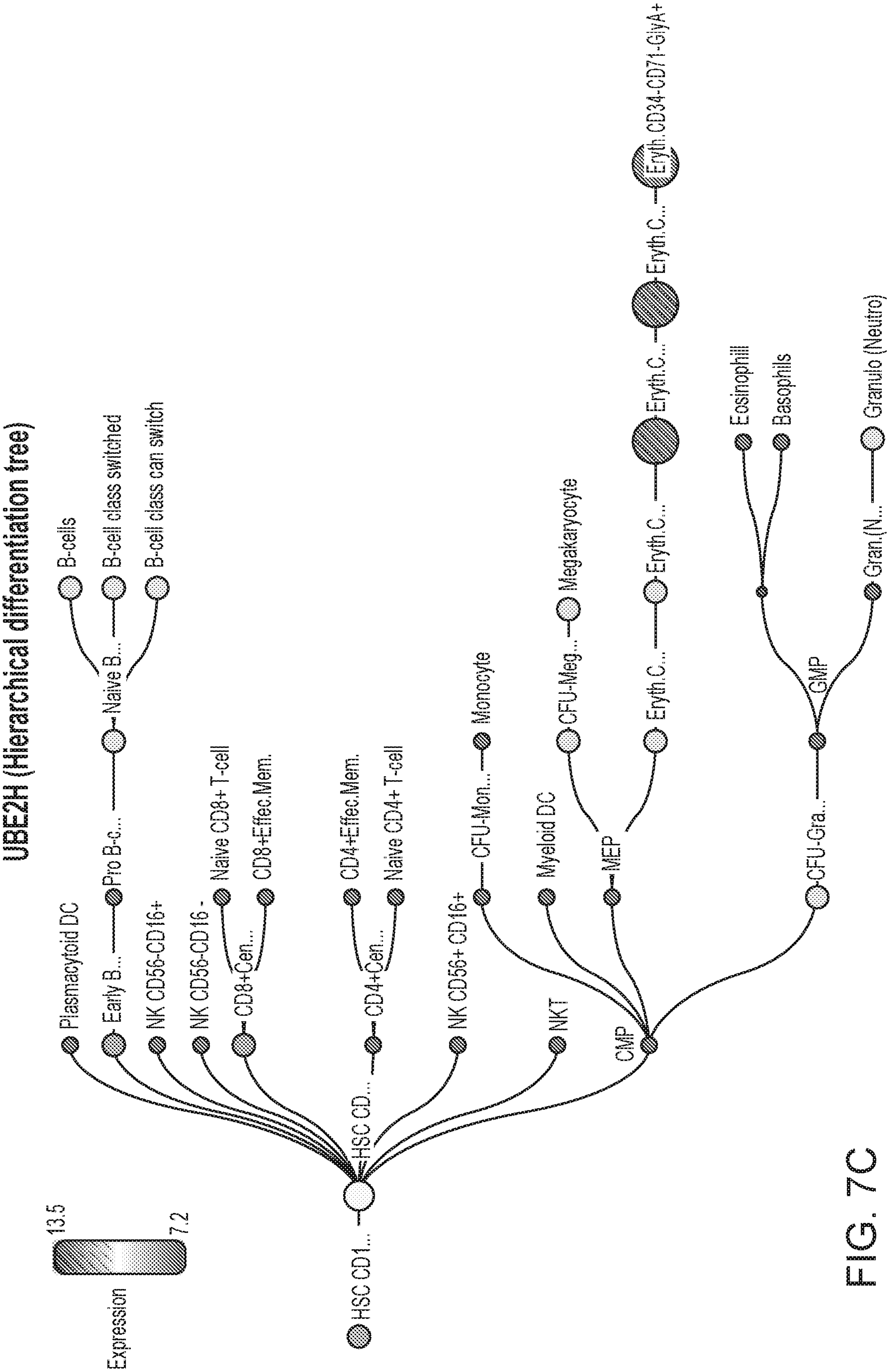
UBE2H (Hierarchical differentiation tree)
Expression
13.5
7.2
HSC CD1...
HSC CD...
Plasmacytoid DC
Early B...
Pro B-c...
Naive B...
B-cells
B-cell class switched
B-cell class can switch
NK CD56-CD16+
NK CD56-CD16 -
CD8+Cen...
Naive CD8+ T-cell
CD8+Effec.Mem.
CD4+Cen...
CD4+Effec.Mem.
Naive CD4+ T-cell
NK CD56+ CD16+
NKT
CMP
CFU-Mon...
Monocyte
Myeloid DC
MEP
CFU-Meg...
Megakaryocyte
Eryth.C...
Eryth.C...
Eryth.C...
Eryth.C...
Eryth.C...
Eryth.CD34-CD71-GlyA+
CFU-Gra...
GMP
Eosinophill
Basophils
Gran.(N...
Granulo (Neutro)

FIG. 7C

FIG. 8A

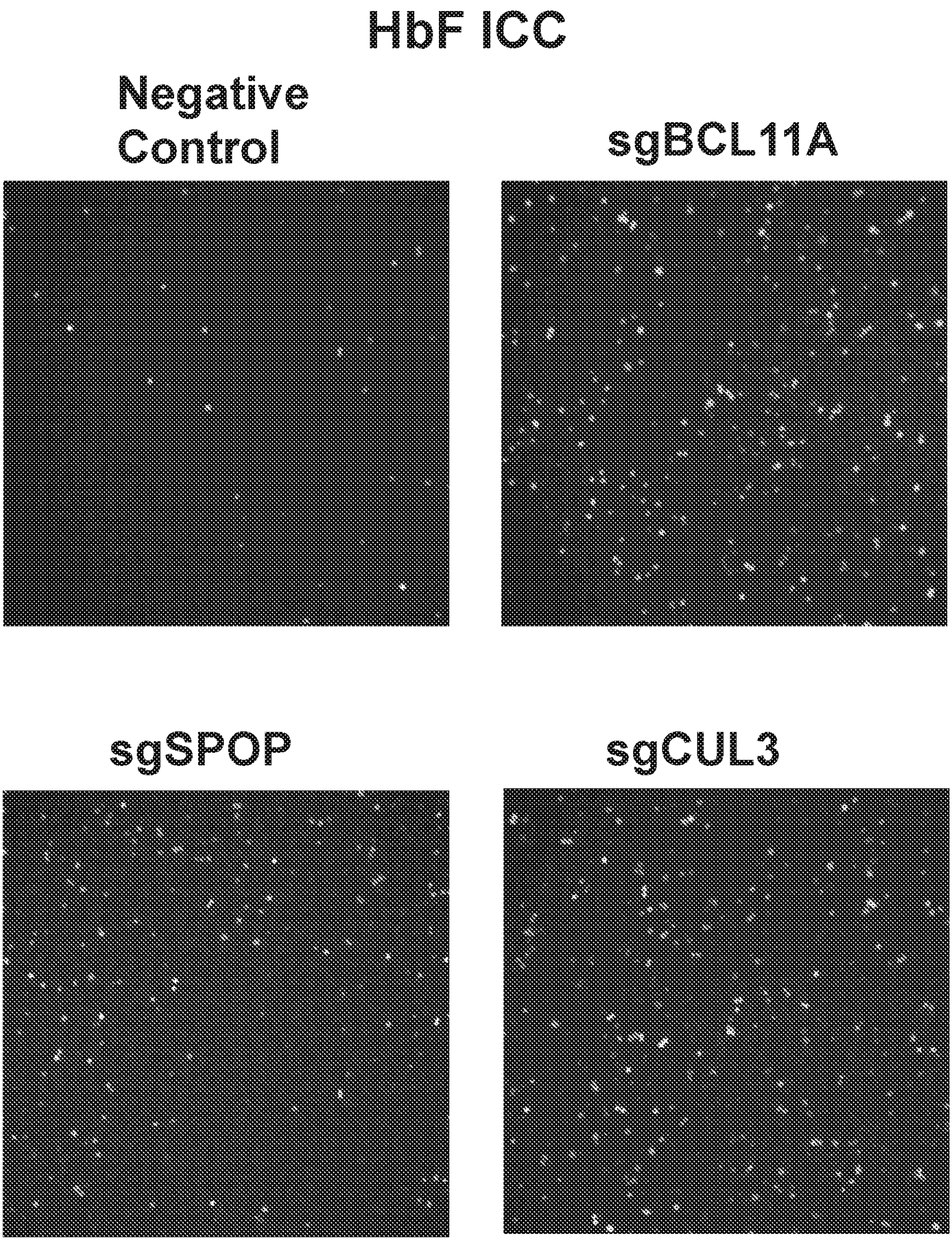

FIG. 8B

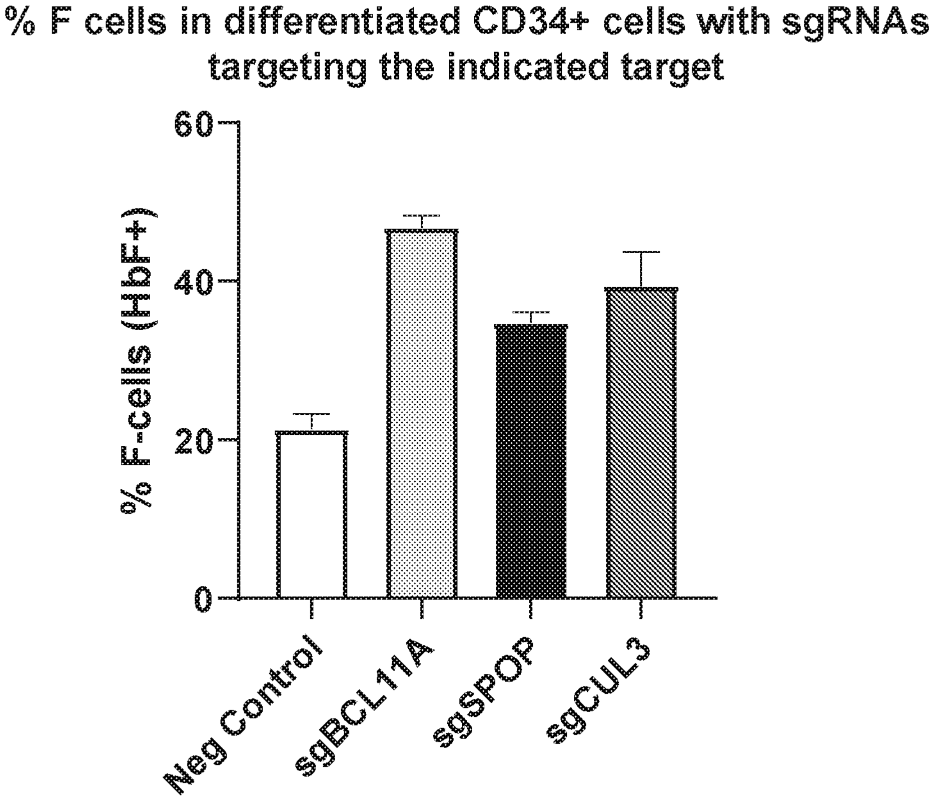
% F cells in differentiated CD34+ cells with sgRNAs
targeting the indicated target
60
40
20
0
% F-cells (HbF+)
Neg Control
sgBCL11A
sgSPOP
sgCUL3

FIG. 8C

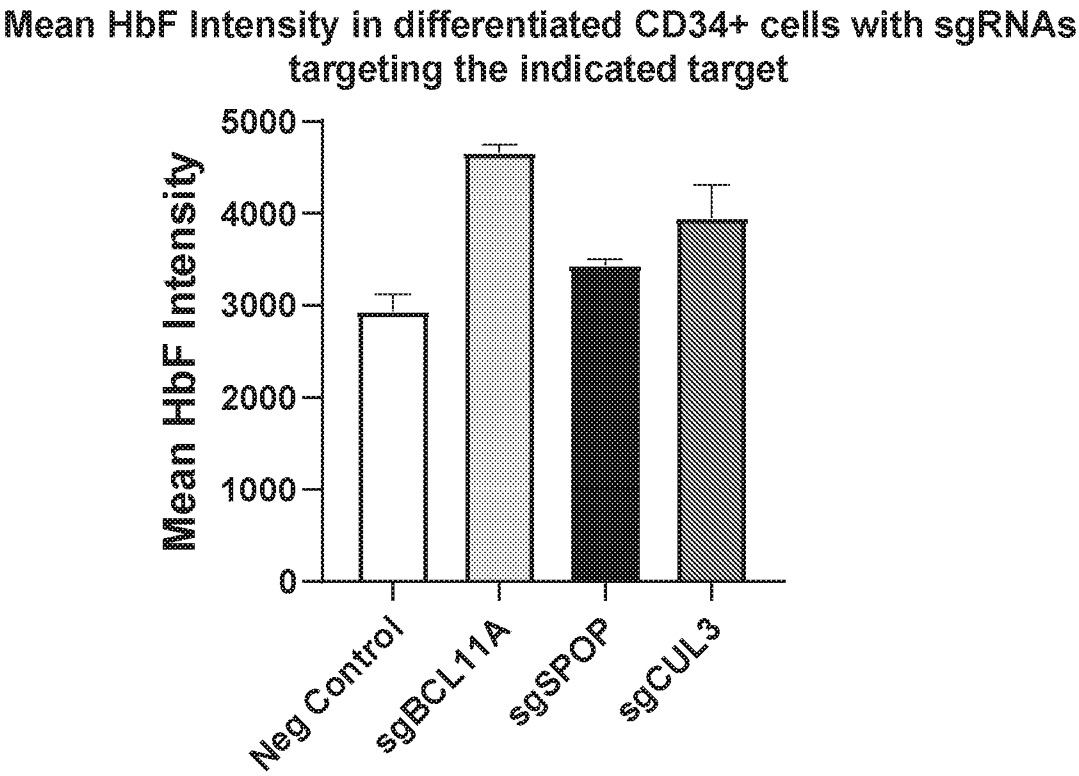
Mean HbF Intensity in differentiated CD34+ cells with sgRNAs
targeting the indicated target
5000
4000
3000
2000
1000
0
Mean HbF Intensity
Neg Control
sgBCL11A
sgSPOP
sgCUL3

FIG. 8D

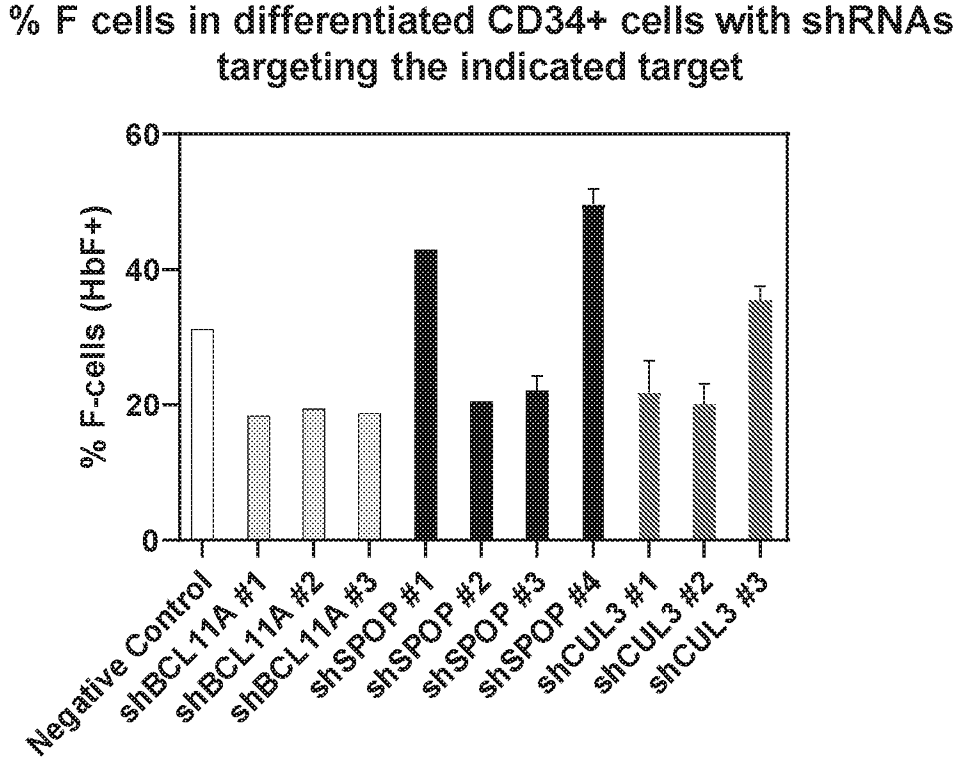
% F cells in differentiated CD34+ cells with shRNAs targeting the indicated target
60
40
20
0
% F-cells (HbF+)
Negative Control
shBCL11A #1
shBCL11A #2
shBCL11A #3
shSPOP #1
shSPOP #2
shSPOP #3
shSPOP #4
shCUL3 #1
shCUL3 #2
shCUL3 #3

FIG. 8E

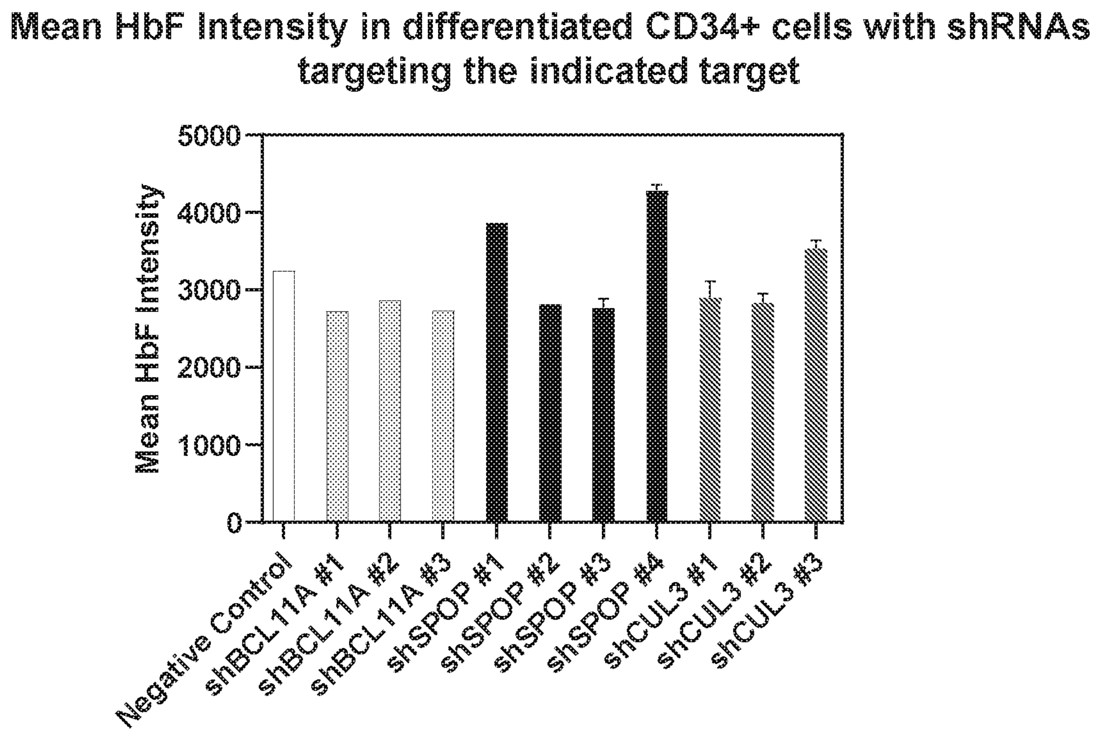
Mean HbF Intensity in differentiated CD34+ cells with shRNAs targeting the indicated target
5000
4000
3000
2000
1000
0
Mean HbF Intensity
Negative Control
shBCL11A #1
shBCL11A #2
shBCL11A #3
shSPOP #1
shSPOP #2
shSPOP #3
shSPOP #4
shCUL3 #1
shCUL3 #2
shCUL3 #3

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 62769796 A
- US 4522811 A


### Non-patent literature cited in the description


- **BLOBEL et al.** *Exp Hematol*, 2015
- **STAMATOYANNOPOULOS G.** *Exp Hematol*, 2005
- **LI et al.** *Blood*, 2002
- **PAIKARI** ; **SHEEHAN**. *Br J Haematol*, 2018
- **LETTNE** ; **BAUER**. *Lancet*, 2016
- **MOUTOUH-DE PARSEVAL et al.** *J Clin Invest*, 2008
- **LETVIN et al.** *NEJM*, 1984
- **RENNEVILLE et al.** *Blood*, 2015
- **SHI et al.** *Nature Med*, 2015
- **WAHL. G. M.** ; **S. L. BERGER**. *Methods Enzymol.*, 1987, vol. 152, 399
- **KIMMEL, A. R.** *Methods Enzymol.*, 1987, vol. 152, 507
- *CHEMICAL ABSTRACTS*, 905579-51-3
- *CHEMICAL ABSTRACTS*, 145-63-1
- *CHEMICAL ABSTRACTS*, 2245887-38-9
- *Nat Methods*, November 2013, vol. 10 (11), 1116-1121
- *Sci Rep.*, 2014, vol. 4, 5405
- **GIURGIU M et al.** *Nucleic Acids Research*
- **NOVERSHTEM et al.** *Cell*
- **BLOBEL GA, BODINE D, BRAND M, CRISPINO J, DE BRUIJN MF, NATHAN D, PAPAYANNOPOULOU T, PORCHER C, STROUBOULIS J. ZON L. HIGGS DR. STAMATOYANNOPOULOS G, ENGEL JD**. An international effort to cure a global health problem: A report on the 19th Hemoglobin Switching Conference. *Exp Hematol.*, October 2015, vol. 43 (10), 821-37
- **STAMATOYANNOPOULOS G**. Control of globin gene expression during development and erythroid differentiation. *Exp Hematol.*, March 2005, vol. 33 (3), 259-71
- **PAIKARI A, SHEEHAN VA**. Fetal haemoglobin induction in sickle cell disease. *Br J Haematol.*, 16 November 2017, vol. 180 (2), 189-200
- **LETTRE G, BAUER DE**. Fetal haemoglobin in sickle-cell disease: from genetic epidemiology to new therapeutic. *Lancet*, 18 June 2016, vol. 387 (10037), 2554-64
- **LI Q. PETERSON KR. FANG X, STAMATOYANNOPOULOS G**. Locus control regions. *Blood*, 01 November 2002, vol. 100 (9), 3077-86
- **MOUTOUH-DE PARSEVAL LA, VERHELLE D, GLEZER E, JENSEN-PERGAKES K, FERGUSON GD, CORRAL LG. MORRIS CL. MULLER G. BRADY H, CHAN K**. Pomalidomide and lenalidomide regulate erythropoiesis and fetal hemoglobin production in human CD34+ cells. *J Clin Invest.*, January 2008, vol. 118 (1), 248-58
- **LETVIN NL, LINCH DC, BEARDSLEY GP, MCINTYRE KW, NATHAN DG**. Augmentation of fetal-hemoglobin production in anemic monkeys by hydroxyurea. *N Engl J Med.*, 05 April 1984, vol. 310 (14), 869-73
- **RENNEVILLC A, VAN GALEN P, CANVER MC, MCCONKEY M. KRILL-BURGER JM. DORFMAN DM, HOLSON EB, BERNSTEIN BE, ORKIN SH, BAUER DE, EBERT BL**. EHMT1 and EHMT2 inhibition induces fetal hemoglobin expression. *Blood*, 28 August 2015, vol. 126 (16), 1930-9
- **SHI L. CUI S, ENGEL JD, TANABE O**. Lvsine-specific demethylase 1 is a therapeutic target for fetal hemoglobin induction. *Nat Med.*, 17 February 2013, vol. 19 (3), 291-4
- **GIURGIU M. REINHARD J, BRAUNER B. DUNGER-KALTENBACH 1, FOBO G, FRISHMAN G, MONTRONE C. RUEPP A**. CORUM: the comprehensive resource of mammalian protein complexes-2009. *Nucleic Acids Res.*, 01 November 2009, vol. 38, D497-501
- **NOVERSHTERN N, SUBRAMANIAN A. LAWTON L.N. RAYMOND H. M, HAINING N, MCCONKEY M. E, HABIB N, YOSEF N, CHANG C. Y, SHAY T, FRAMPTON C. M, DRAKE A. C. B, LESKOV I, NILSSON B. PROFFER F. DOMBKOWSKI D, EVANS J. W. LIEFELD R, SMUTKO J. S, CHEN J, FRIEDMAN N, YOUNG R. A. GOLUB T. R. REGEV A. EBERT B. L.** Densely interconnected transcriptional circuits control cell states in human hematopoiesis. *Cell*, 21 January 2011, vol. 144 (2), 296-309

- The Genotype-Tissue Expression (GTEx) project The GTEx Consortium. *Nat Genet.*, June 2013, vol. 45 (6), 580-5